(19) Europäisches Patentamt
European Patent Office
Office européen des brevets

(11) EP 4 092 111 A1

(12) **EUROPEAN PATENT APPLICATION**
published in accordance with Art. 153(4) EPC

(43) Date of publication:
**23.11.2022 Bulletin 2022/47**

(21) Application number: **21741267.5**

(22) Date of filing: **14.01.2021**

(51) International Patent Classification (IPC):
***C12N 5/0775*** *(2010.01)* ***C12N 9/22*** *(2006.01)*
***C12N 15/10*** *(2006.01)* ***C12N 15/11*** *(2006.01)*
***C07K 14/47*** *(2006.01)*

(52) Cooperative Patent Classification (CPC):
**C07K 14/47; C12N 5/06; C12N 9/22; C12N 15/10; C12N 15/11**

(86) International application number:
**PCT/KR2021/000546**

(87) International publication number:
**WO 2021/145700 (22.07.2021 Gazette 2021/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.01.2020 KR 20200004614**

(71) Applicant: **Toolgen Incorporated**
**Seoul 08501 (KR)**

(72) Inventors:
- **SHIN, Eun Ji**
  **Suwon-si Gyeonggi-do 16538 (KR)**
- **LEE, Kang In**
  **Seoul 02830 (KR)**
- **LEE, Jae Young**
  **Seoul 06721 (KR)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

Remarks:
The complete document including Reference Table(s) and the Sequence Listing(s) can be downloaded from the EPO website

# (54) CELLS HAVING HIGH ADAPTABILITY UNDER HYPOXIC CONDITIONS, AND USE THEREOF

(57) The present application relates to cells having high adaptability under hypoxic conditions and to a preparation method therefor. Particularly, the present disclosure provides cells having high adaptability under hypoxic conditions, the cells including at least one engineered gene having an indel within a wild-type gene selected from the group consisting of HIF1AN, HIF3A, PHD2, TLR4 and PAI1. In addition, the present disclosure provides, as a method for preparing cells having high adaptability under hypoxic conditions, a gene editing method including introducing a CRISPR/Cas9 system into cells.

FIG. 16

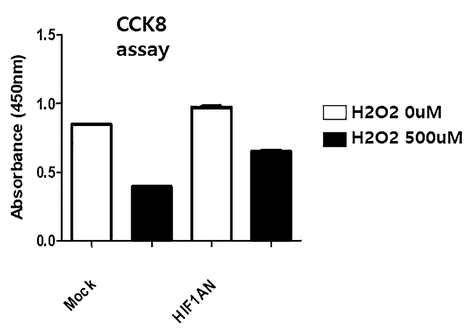


EP 4 092 111 A1

## Description

### Technical Field

The present application relates to a cell having high adaptability under hypoxic conditions, a preparation method therefor, and a cell therapeutic agent containing the cell as an active ingredient. These days, various studies are being conducted on a method of differentiating stem cells, such as embryonic stem cells and adult stem cells, into various cells and using the produced cells in cell therapy. Embryonic stem cells with pluripotency have been attracting attention as a cell therapeutic agent because they can differentiate into various cells. However, it is difficult to practically use the embryonic stem cells for cell therapy due to ethical problems in using the embryonic stem cells. In order to avoid these ethical problems, research using adult stem cells is being actively conducted.

### Background Art

It is known that cells under hypoxic conditions highly express hypoxia-inducible factor 1 (HIF1) proteins to survive. HIF1 protein is a gene transcriptional regulator involved in angiogenesis (vascularization) and energy metabolism in a hypoxic environment, thereby playing a role in creating an environment in which cells can survive. HIF1 has HIF1$\alpha$ and HIF1 $\beta$ as subunits thereof. HIF1$\alpha$ and HIF1 $\beta$ are encoded by HIF1A and HIF1B genes, respectively.

[Documents of Related Art]

Mahon PC, Hirota K, Semenza GL. FIH-1: a novel protein that interacts with HIF-lalpha and VHL to mediate repression of HIF-1 transcriptional activity, Genes Dev. 2001;15(20):2675?2686. doi:10.1101/gad.924501.

### Disclosure

Technical Problem

An objective of the present disclosure is to provide a cell having high adaptability to a hypoxic environment.

Another objective of the present disclosure is to provide a gene-engineering composition for producing cells having high adaptability to a hypoxic environment.

A further objective of the present disclosure is to provide a genetic engineering method for producing cells having high adaptability to a hypoxic environment.

A yet further objective of the present disclosure is to provide a use of a cell having high adaptability to a hypoxic environment.

Technical Solution

The present disclosure discloses an engineered cell including: at least one engineered gene having an indel in a wild-type gene selected from the group consisting of HIF1AN, HIF3A, PHD2, TLR4, and PAI1, in which the engineered gene and the wild-type gene differ in gene sequence, mRNA transcribed from the wild-type gene exhibits a lower expression level in the engineered cell than the wild-type cell, and HIF$\alpha$ exhibits a higher expression level in the engineered cell than the wild-type cell.

In one embodiment, there is provided an engineered cell configured such that: when the engineered cell includes an engineered gene having an indel in an HIF1AN gene, the indel in the HIF1AN gene is located in exon 1 of the HIF1AN gene; when the engineered cell includes an engineered gene having an indel in an HIF3A gene, the indel in the HIF3A gene is located in one or more regions selected from the group consisting of exon 2, exon 3, exon 4, exon 5, and exon 6 of the HIF3A gene; when the engineered cell includes an engineered gene having an indel in an PHD2 gene, the indel in the PHD2 gene is located in exon 1 of the PHD2 gene; when the engineered cell includes an engineered gene having an indel in an TLR4 gene, the indel in the TLR4 gene is located in one or more regions selected from the group consisting of exon 1, exon 2 and exon 3 of the TLR4 gene; and when the engineered cell includes an engineered gene having an indel in an PAI1 gene, the indel in the PAI1 gene is located in exon 2 of the PAI1 gene.

In one embodiment, there is provided an engineered cell configured such that: when the engineered cell include an engineered gene having an indel in an HIF1AN gene, the engineered gene does not include one or more sequences selected from the group consisting of SEQ ID NOs:1 to 9; when the engineered cell includes an engineered gene having an indel in an HIF3A gene, the engineered gene does not include one or more sequences selected from the group consisting of SEQ ID NOs:10 to 34; when the engineered cell includes an engineered gene having an indel in an PHD2 gene, the engineered gene does not include one or more sequences selected from the group consisting of SEQ ID NOs:35 to 38; when the engineered cell includes an engineered gene having an indel in an TLR4 gene, the engineered gene does not include one or more sequences selected from the group consisting of SEQ ID NOs:39 to 59; and when the engineered cell includes an engineered gene having an indel in a PAI1 gene, the engineered gene does not include one or more sequences selected from the group consisting of SEQ ID NOs:60 to 71.

In one embodiment, there is provided an engineered cell including an engineered gene having an indel in an HIF1AN gene.

In one embodiment, there is provided an engineered cell in which the indel in the HIF1AN gene is located in exon 1 of the HIF1AN gene.

In one embodiment, there is provided an engineered cell in which a sequence of the engineered gene does not include one or more sequences selected from the group consisting of SEQ ID NOs:1 to 9.

In one embodiment, there is provided an engineered cell in which an expression level of FIH-1 in the engineered cell is 70% or lower than an expression level of FIH-1 in the wild-type cell.

In one embodiment, there is provided an engineered cell in which expression levels of VEGF and IL-8 in the engineered cell are higher than expression levels of VEGF and IL-8 in the wild-type cell, respectively.

In one embodiment, there is provided an engineered cell including an engineered gene having an indel in an HIF3A gene.

In one embodiment, there is provided an engineered cell in which the indel in the HIF3A gene is located in one or more regions selected from the group consisting of exon 2, exon 3, exon 4, exon 5, and exon 6.

In one embodiment, there is provided an engineered cell in which a sequence of the engineered gene does not include one or more sequences selected from the group consisting of SEQ ID NOs:10 to 34.

In one embodiment, there is provided an engineered cell including an engineered gene having an indel in an PHD2 gene.

In one embodiment, there is provided an engineered cell in which the indel in the PHD2 gene is located in exon 1 of the PHD2 gene.

In one embodiment, there is provided an engineered cell in which a sequence of the engineered gene does not include one or more sequences selected from the group consisting of SEQ ID NOs:35 to 38.

In one embodiment, there is provided an engineered cell including an engineered gene having an indel in an TLR4 gene.

In one embodiment, there is provided an engineered cell in which the indel in the TLR4 gene is located in one or more regions selected from the group consisting of exon 1, exon 2, and exon 3.

In one embodiment, there is provided an engineered cell in which a sequence of the engineered gene does not include one or more sequences selected from the group consisting of SEQ ID NOs:39 to 59.

In one embodiment, there is provided an engineered cell including an engineered gene having an indel in a PAI1 gene.

In one embodiment, there is provided an engineered cell in which the indel in the PAI1 gene is located in exon 2 of the PAI1 gene.

In one embodiment, there is provided an engineered cell in which a sequence of the engineered gene does not include one or more sequences selected from the group consisting of SEQ ID NOs:60 to 71.

In one embodiment, the engineered cell is a mesenchymal stem cell derived from a tissue selected from the group consisting of bone marrow, adipose tissue, umbilical cord, placenta, amniotic fluid, and umbilical cord blood.

In one embodiment, the engineered cell is a mesenchymal stem cell derived from bone marrow.

The present disclosure discloses an engineered cell including: at least one engineered gene selected from the group consisting of an engineered HIF1AN gene, an engineered HIF3A gene, an engineered PHD2 gene, an engineered TLR4 gene, and an engineered PAI1 gene, in which: the engineered HIF1AN gene does not include one or more sequences selected from SEQ ID NOs:1 to 9; the engineered HIF3A gene does not include one or more sequences selected from SEQ ID NOs:10 to 34; the engineered PHD2 gene does not include one or more sequences selected from SEQ ID NOs:35 to 38; the engineered TLR4 gene does not include one or more sequences selected from SEQ ID NOs:39 to 59; and the engineered PAI1 gene does not include one or more sequences selected from SEQ ID NOs:60 to 71.

The present disclosure discloses a gene-editing composition including: Cas9 protein derived from *streptococcus pyogenes* or DNA encoding the Cas9 protein; and guide RNA or DNA encoding the guide RNA, in which the guide RNA has a sequence selected from the group consisting of SEQ ID NOs:146 to 216.

In one embodiment, there is provided a gene-editing composition including the Cas9 protein and the guide RNA in the form of ribonucleoprotein (RNP).

In one embodiment, there is provided a gene-editing composition including the DNA encoding the Cas9 protein and the DNA encoding the guide RNA in the form of a single vector.

In one embodiment, there is provided a gene-editing composition in which the single vector is selected from the group consisting of plasmid, AAV, and the like.

The present disclosure discloses a method of editing a gene in a cell, the method including introducing a gene-editing composition into a cell, the composition including: *Streptococcus* pyogenes-derived Cas9 protein or DNA encoding the Cas9 protein; and guide RNA or DNA encoding the guide RNA, in which the guide RNA has a sequence selected from the group consisting of SEQ ID NOs:146 to 216.

In one embodiment, there is provided a gene editing method in which the composition includes the DNA encoding the Cas9 protein and the DNA encoding the guide RNA in the form of a vector.

The present disclosure discloses a method of editing a gene including a target DNA in a cell, the method including bringing a CRISPR/Cas9 complex into contact with the target DNA, the CRISPR/Cas9 complex including: *a Streptococcus* pyogenes-derived Cas9 protein; and a guide RNA targeting the target DNA, in which the guide RNA has a sequence selected from the group consisting of SEQ ID NOs:146 to 216.

## Advantageous Effect of Invention

In the present disclosure, a cell having high adaptability in a hypoxic environment and a method of producing the same are disclosed. Since the cells having high adaptability to a hypoxic environment show high adaptability and survival rate in the hypoxic environment, the cells are suitable for use in cell therapy.

## Description of Drawings

FIG. 1 is experimental data for checking whether gene modification is occurred after treating with a gene-engineering composition targeting a portion of the nucleotide sequence of the hHIF3A gene. Specifically, the indel generation rate was checked with change in guide nucleic acid.

FIG. 2 is experimental data for checking whether gene modification is occurred after treating with a gene-engineering composition targeting a portion of the nucleotide sequence of the hTLR4 gene. Specifically, the indel generation rate was checked with change in guide nucleic acid.

FIG. 3 is experimental data for checking whether gene modification is occurred after treating with a gene engineering composition targeting a portion of the nucleotide sequence of the hPAII gene. Specifically, the indel generation rate was checked with change in guide nucleic acid.

FIG. 4 is experimental data for checking whether gene modification is occurred after treating with a gene-engineering composition targeting a portion of the nucleotide sequence of the hHIFIAN gene. Specifically, the indel generation rate was checked with change in guide nucleic acid.

FIG. 5 is experimental data for checking whether gene modification is occurred after treating with a gene-engineering composition targeting a portion of the nucleotide sequence of the hPHD2 gene. Specifically, the indel generation rate was checked with change in guide nucleic acid.

FIGS. 6 and 10 show cells suitable for experiments obtained by treating MSC with a gene-engineering composition and screening the treated MSC with Miseq.

FIGS. 7 to 9 and 11 to 13 are comparison data showing mRNA expression levels in the cells selected through Miseq in FIGS. 6 and 10, respectively, in which the mRNA expression levels were compared through qRT-PCR.

FIG. 14 shows cells suitable for experiments obtained by treating MSC with a hHIFIAN gene-engineering composition and screening the treated MSC through deep sequencing.

FIG. 15 is comparison data showing mRNA expression levels in the cells selected through deep sequencing in FIG. 10, in which the mRNA expression levels were compared through qRT-PCR.

FIG. 16 is data obtained by measuring changes in the proliferative capacity of MSCs under reactive-oxygen stress frequently occurring under hypoxic conditions, and is data obtained by counting the number of viable cells through CCK8 assay.

FIG. 17 is data obtained by measuring changes in the proliferative capacity of MSCs under reactive-oxygen stress frequently occurring under hypoxic conditions, and is data obtained by counting the number of viable cells of MSCs in which hHIFIAN gene is knocked out and the number of viable cells of control MSCs for each of the cases where the molar concentration of $H_2O_2$ is 0 uM and 500 uM.

FIG. 18 is a graph showing the result of comparison in indel efficiency and out-of-frame ratio obtained by deep sequencing after engineering the HIF1AN gene of mesenchymal stem cells according to an experimental example. Mock denotes a non-genetically engineered cell, HIF1AN KO denotes a cell in which an HIF1AN gene is engineered, SHS231 KO denotes a cell in which the SHS231 locus is engineered, AAVS1 denotes a cell in which the AAVS1 locus is engineered, and the SHS231 KO and AAVS1 are used as control.

FIG. 19 is a graph showing the result of comparison between HIF1AN mRNA expression levels measured by qRT-PCR after engineering the HIF1AN gene of mesenchymal stem cells according to an experimental example. Mock denotes a non-genetically engineered cell, HIF1AN KO denotes a cell in which an HIF1AN gene is engineered, SHS231 KO denotes a cell in which the SHS231 locus is engineered, AAVS1 denotes a cell in which the AAVS1 locus is engineered, and the SHS231 KO and AAVS1 are used as control.

FIG. 20 shows the results of evaluation of the viability of the mesenchymal stem cells engineered with the HIF1AN gene prepared according to an experimental example for SNAP (reactive nitrogen species) through CCK-8 assay. Mock denotes a non-genetically engineered cell, and HIF1AN KO denotes a HIF1AN gene-engineered cell.

FIGS. 21 to 22 show results of BrdU cell proliferation analysis to determine the characteristics of the mesenchymal stem cells engineered with the HIF1AN gene prepared according to an experimental example. Mock denotes a non-genetically engineered cell, HIF1AN KO denotes a cell in which an HIF1AN gene is engineered, SHS231 KO denotes a cell in which the SHS231 locus is engineered, and the SHS231 KO is used as control.

FIGS. 23 to 25 show results of FACS analysis to determine MSC markers of the mesenchymal stem cells engineered with the HIF1AN gene prepared according to an experimental example. Mock denotes a non-genetically engineered cell, HIF1AN KO denotes a cell in which an HIF1AN gene is engineered, SHS231 KO denotes a cell in which the SHS231 locus is engineered, and the SHS231 KO is used as control.

FIGS. 26 to 27 are measurement results of measurement of the population doubling levels (PDLs) and the population doubling times (PDTs) of the mesenchymal stem cells engineered with the HIF1AN gene prepared according to an experimental example. Mock denotes a non-genetically engineered cell, HIF1AN KO denotes a cell in which an HIF1AN gene is engineered, SHS231 KO denotes a cell in which the SHS231 locus is engineered, AAVS1 denotes a cell in which the AAVS1 locus is engineered, and the SHS231 KO and AAVS1 are used as control.

FIG. 28 is a result of comparison in the secretion amount of Cytokine in the culture medium of the mesenchymal stem cells engineered with the HIF1AN gene prepared according to an experimental example. Mock denotes a non-genetically engineered cell, HIF1AN KO denotes a cell in which an HIF1AN gene is engineered, AAVS1 denotes a cell in which the AAVS1 locus is engineered, and the AAVS1 is used as control.

FIGS. 29 to 30 show the results of Experimental Example 3 in a new MSC Stock in order to determine the reproducibility of the results of the experimental example. Mock denotes a non-genetically engineered cell, Mock $H_2O_2$ 300 $\mu$M+ denotes a cell in which an oxidative stress situation is created by adding 300 $\mu$M of $H_2O_2$ to a non-genetically engineered cell, HIF1AN KO denotes a cell in which the HIF1AN gene is engineered, HIF1AN KO $H_2O_2$ 300 $\mu$M+ denotes a cell in which an oxidative stress situation is created adding 300 $\mu$M of $H_2O_2$ to the HIF1AN gene-engineered cells. A: graph showing comparison between HIF1AN mRNA expression levels. B: graph showing comparison between the expression levels of cytokine (VEGF and IL-8) mRNA.

FIG. 31 shows the Western blot results of Experimental Example 4-1. Mock denotes a non-genetically engineered cell, sgHIFIAN denotes a cell in which an HIF1AN gene is engineered, sgAAVSI denotes a cell in which the AAVS1 locus is engineered, and the sgAAVSI is used as control.

## Best Mode

### Definition of Terms

#### About

As used herein, the term "about" refers to a degree close to a certain quantity, and it refers to an amount, level, value, number, frequency, percent, dimension, size, amount, weight, or length that varies by to the extent of 30%, 25%, 20%, 25%, 10%, 9%, 8%, 7%, 6%, 5%, 4%, 3%, 2%, or 1% with respect to a reference amount, level, value, number, frequency, percentage, dimension, size, amount, weight, or length.

#### Meaning of A, T, C, G, and U

As used herein, the symbols A, T, C, G and U are interpreted as meanings understood by those of ordinary skill in the art. Each of these symbols may be properly interpreted as a base, a nucleoside, or a nucleotide on DNA or RNA according to context and technology. For example, when each of the symbols means a base, the symbols A, T, C, G and U can be interpreted as adenine (A), thymine (T), cytosine (C), guanine (G), or uracil (U), respectively. When each of the symbols means a nucleoside, the symbols A, T, C, G and U can be interpreted as adenosine (A), thymidine (T), cytidine (C), guanosine (G) or uridine (U), respectively. When meaning a nucleotide in the sequence, the symbols A, T, C, G and U denote nucleotides including the nucleosides, respectively.

#### "Engineered"

As used herein, the term "engineered" is a term used to distinguish a certain substance, molecule, etc. from a substance, molecule, etc. having a composition already existing in nature, and the term "engineered" means that artificial modifications are applied to substances, molecules, etc. For example, in the case of "engineered gene", it refers to a gene in which an artificial change has been made to the composition of a gene existing in nature. In addition, the term includes all meanings recognizable by those skilled in the art and may be appropriately interpreted according to the context.

Wild-type

The term "Wild-type" means that a gene comprising a naturally occurring nucleotide sequence and a protein expressed from the gene have normal functional properties. Wild-type genes are most frequently observed in the population. When the term "wild-type" is used herein in contrast to an artificially engineered (engineered) gene and/or cell, it means a gene that is the same kind as a corresponding engineered gene and includes a "non-artificially engineered" and naturally occurring nucleotide sequence, and a cell including the gene. In addition, the term includes all meanings recognizable by those skilled in the art and may be appropriately interpreted according to the context.

Knock-out

As used herein, the expression "knock-out" or "knockout gene" means that a protein expressed by a wild-type gene cannot be produced through transcription and/or translation. For example, a cell containing a knocked-out gene A may not express mRNA and/or protein expressed by a wild-type gene A. A cell including a knocked-out gene A may be cell in which only one of the genes A present in the cell is knocked out, or two or more genes of the genes A are knocked out. In addition, the term includes all meanings recognizable by those skilled in the art and may be appropriately interpreted according to the context.

Knock-down

As used herein, the term "knock-down" or "knock-down gene" means expressing a substance in a lower amount than a wild-type gene. For example, a cell including a knocked-down gene A may express a smaller amount of mRNA than the mRNA expressed by a wild-type gene A. A cell with a knocked-down gene A may be a cell in which only one of the genes A present in the cell is knocked down, or two or more genes of the genes A are knocked down. In addition, the term include all meanings recognizable by those skilled in the art and may be appropriately interpreted according to the context.

Expression Level

As used herein, the term "expression level" refers to how well expression products, for example, mRNA and/or proteins are produced from a particular gene. In general, when the expression level of a specific gene in a cell is high, the amount of mRNA and/or a specific protein produced from the specific gene contained in the cell, or the concentration of the expression products in the cytoplasm is high. On the contrary, when the expression level of a specific gene in a cell is low, the amount of mRNA and/or a specific protein produced from the specific gene contained in the cell, or the concentration of the expression products in the cytoplasm is low. In the present disclosure, when encountering the expression "expression level is high" or "expression level is low", the comparison target may be interpreted as a homogeneous, wild-type cell, unless the comparison target is otherwise specified. In addition, quantitative indicators that serve as comparative references should be interpreted as most appropriate in context. For example, an absolute amount, a relative amount, and/or a concentration of each cell may be a comparison criterion but is not limited thereto. For example, when it is expressed that the expression level of the gene a in a first cell is higher than the expression level of the gene a in a second cell, the expression product (mRNA, protein, etc.) of the gene a in the first cell is larger in absolute amount, relative amount, and/or concentration than the expression product of the gene a in the second cell. In addition, the term includes all meanings recognizable by those skilled in the art and may be appropriately interpreted according to the context.

Background - Factors Affecting Survival of Cells in Hypoxic Environment

HIF1 Protein

It is known that cells placed in a hypoxic environment express a large amount of a hypoxia-inducible factor 1 (HIF1) protein in order to survive. HIF1 protein is a gene transcriptional regulator involved in angiogenesis (vascularization) and energy metabolism in a hypoxic environment, thereby playing a role in creating an environment in which cells can survive. HIF1 has HIF1a and HIF1 β as subunits thereof. HIF1α and HIF1 β are encoded by HIF1A and HIF1B genes, respectively. Therefore, in order for cells to survive in a hypoxic environment, the expression level of the HIF1 protein or the expression levels of the subunits of the HIF1 protein must be high, or the activity thereof must be maintained high.

Factor Inhibiting Function of HIF1 Protein - FIH1

FIH-1 is a protein encoded by the HIF1AN gene and is a protein involved in the post-translational control of HIF1a. More specifically, FIH-1 is an asparaginyl hydroxylase. FIH-1 plays a role in reducing HIF1 activity by interfering with the interaction between HIF1α and p300/CBP, which is a nuclear receptor activator. In addition, FIH-1 functions to induce HIF1α to be degraded in cells after the HIF1α is synthesized. Since FIH-1 even works under severe hypoxia, it corresponds to a protein directly related to cell survival in an hypoxic environment.

Factor Inhibiting Function of HIF1 Protein - PHD2

PHD2 is a protein involved in post-translational control of HIF1a in concurrence with FIH-1. More specifically, PHD2 is prolyl hydroxylase. PHD2 functions to induce HIF1α to be degraded in cells after the HIF1α is synthesized.

Factor Inhibiting Function of HIF1 Protein - HIF3A

HIF3A is a protein involved in the transcription control of HIF1a. HIF3A plays a role in interfering with the transcription of the HIF1α gene.

Others - Factors Affecting Survival of Cells in Hypoxic Environment

TLR4 is known as a factor involved in the innate immune response to wounds. In a hypoxic environment, activated TLR4 functions to induce pro-apoptotic signaling and, consequently, induces apoptosis. PAI1 is a factor that negatively regulates extracellular matrix attachment of mesenchymal stem cells (MSC). PAI1 is known that the expression thereof increases in a hypoxic environment and is also known to affect the autograft survival of stem cells.

Background - CRISPR/Cas System

Overview

A CRISPR/Cas system is an immune system found in prokaryotic organisms and includes a Cas protein and a guide RNA. The configuration of the Cas protein or guide RNA is described in detail in International Publication No. WO2018/231018, which is a published document. As used herein, the term "Cas protein" is a generic term for nucleases that can be interpreted as being used in the CRISPR/Cas system. However, for convenience of description, a DNA cleavage process in which the CRISPR/Cas9 system is most frequently used will be briefly described below as an example.

Cas9 Protein

In a CRISPR/Cas9 complex, a protein having a nuclease activity of cleaving a nucleic acid is called a Cas9 protein. The Cas9 protein corresponds to Class 2, Type II in the CRISPR/Cas system classification. Examples of the Cas9 protein include streptococcus pyogenes, streptococcus thermophilus, streptococcus sp., *Streptomyces pristinaespiralis,* streptomyces viridochromogenes, streptomyces viridochromogenes, streptosporangium roseum, and streptosporangium roseum-derived Cas9. Among them, the Cas9 protein which is most commonly used for research purposes is a Cas9 protein derived from Streptococcus pyogenes.

Guide RNA

In the CRISPR/Cas9 complex, RNA having a function of inducing the CRISPR/Cas9 complex to recognize a specific sequence included in a target nucleic acid is referred to as a guide RNA. The guide RNA is functionally divided into 1) a scaffold sequence portion and 2) a guide sequence portion. The scaffold sequence portion is a portion that interacts with the Cas9 protein and is a portion that binds to the Cas9 protein to form a complex. In general, the scaffold sequence portion includes a tracrRNA and a crRNA repeat sequence portion, and the scaffold sequence is determined depending on which Cas9 protein is used. The guide sequence portion is a portion capable of complementary binding to a nucleotide sequence portion having a predetermined length in a target nucleic acid. The guide sequence portion is a base portion that can be artificially modified and is determined by the target nucleotide sequence of interest.

Process of Cleaving Target Nucleic Acid by CRISPR/Cas9 Complex

When a CRISPR/Cas9 complex comes into contact with the target nucleic acid, the Cas9 protein recognizes a nucleotide sequence of a predetermined length. When a portion of the guide RNA (the guide sequence portion) complementarily binds to a portion adjacent to the PAM sequence, the target nucleic acid is cleaved by the CRISPR/Cas9 complex.

In this case, the nucleotide sequence of a certain length recognized by the Cas9 protein is referred to as a protospacer-adjacent motif (PAM) sequence, which is a sequence determined depending on the type or origin of the Cas9 protein. For example, a Cas9 protein originated in streptococcus pyogenes can recognize a 5'-NGG-3' sequence in a target nucleic acid. In this case, N is one of adenosine (A), thymidine (T), cytidine (C), and guanosine (G).

In order for the CRISPR/Cas9 complex to cleave a target nucleic acid, the guide sequence portion of the guide RNA must complementarily bind to the sequence portion adjacent to the PAM sequence. Therefore, the guide sequence portion is determined according to the sequence of the target nucleic acid, specifically, the portion of the sequence adjacent to the PAM sequence.

When the CRISPR/Cas9 complex cleaves the target nucleic acid, a certain position in the PAM sequence portion of the target nucleic acid and/or the sequence portion complementary to the guide sequence is cleaved.

Target Strand and Non-Target Strand

The CRISPR/Cas9 complex has cleavage activity for double-stranded DNA. In the double-stranded DNA, a strand having a protospacer binding to the guide sequence portion is referred to as a target strand (TS). A strand complementary to the target strand and having a protospacer that does not bind to the guide sequence portion is referred to as a non-target strand (NTS). The guide sequence portion may complementarily bind to a protospacer sequence portion included in the target strand (TS) of the double-stranded DNA. The guide sequence and the protospacer sequence included in the non-target strand (NTS) of the double-stranded DNA are equivalent sequences. Specifically, the only difference between them is that the guide sequence is an RNA sequence, and the protospacer sequence included in the non-target strand (NTS) is a corresponding DNA sequence.

Limitations of Existing Arts

In recent years, so-called "cell therapeutics" in which cells themselves are used as therapeutic agents for the purpose of treating, diagnosing, and/or preventing diseases, have been extensively studied. Cells used in cell therapeutics are cells capable of expressing therapeutic substances or cells with intact functions. Cells used in cell therapeutics are administered directly to a diseased site, a damaged tissue, or a damaged organ to treat the disease or to regenerate the damaged tissue or organ. In order to achieve this purpose, cells administered as a cell therapeutic agent must survive for a long period of time at the site to which the cells are administered to perform normal functions thereof. However, when cells for treatment or the like are selected and proliferated in vitro to prepare a cell therapeutic agent and then administered to the body, there is a problem in that the cells do not adapt to the body environment and die quickly. The reason for apoptosis is that i) the culture environment outside the body is different from the internal environment of the body, ii) the inside of the body is in a hypoxic environment (hypoxia), iii) nutrients in the body are insufficient, iv) the administered cells cannot be attached to the ECM in the body, or v) an immune response occurs. Among these, the typical apoptosis is due to hypoxia. For this reason, conventional cell therapeutic agents do not guarantee a sufficient survival time in the body, leading to a problem in which it is difficult to achieve the purposes of treatment of diseases and regeneration of tissues.

Cells with High Adaptability to Hypoxic Environment

Overview

Disclosed herein are cells with high adaptability to a hypoxic environment. The type of the cell is not particularly limited as long as the cell can be used as a cell therapeutic agent. The cell is characterized in that a wild-type gene expressing a substance that inhibits the expression of a HIF1 protein is artificially engineered. Accordingly, the expression level of a negative regulator of HIF1 expression is reduced in the cell compared to that of a wild-type cell. As a result, there is a characteristic in which the expression level and/or activity level of the HIF1 protein maintained in the cell is higher than that of the wild-type cell. The cells having high adaptability to a hypoxic environment disclosed herein have high adaptability or viability in a hypoxic environment, and thus are suitable for use in cell therapeutics.

In one embodiment, the cell having a high adaptability to a hypoxic environment provided herein may be an engineered cell. In one embodiment, the engineered cell may be an engineered mesenchymal stem cell.

In one embodiment, the engineered cell contains at least one engineered gene having an indel in a wild-type gene selected from the group consisting of HIF1AN, HIF3A, PHD2, TLR4, and PAI1 expressing a substance that inhibits the expression of HIF1 protein. In one embodiment, the expression level of mRNA transcribed from the wild-type gene, in the engineered cell, may be lower than that in the wild-type cell. In one embodiment, the expression level of HIFa in the engineered cell may be higher than that in the wild-type cells.

In one embodiment, the engineered cell may contain at least one engineered gene selected from the group consisting of an engineered HIF1AN gene, an engineered HIF3A gene, an engineered PHD2 gene, an engineered TLR4 gene, and an engineered PAI1 gene.

Type of Cell

In one embodiment, the cells having high adaptability to a hypoxic environment disclosed herein are somatic cells, for example, cumulus cells, epithelial cells, fibroblasts, nerve cells, keratinocytes, hematopoietic cells, melanocytes, chondrocytes, macrophages, Monocytes, myocytes, B lymphocytes, T lymphocytes, dendritic cells, NK cells, regulatory T cells, germ cells, embryonic stem cells, neural stem cells, mesenchymal stem cells, and adult stem cells including tissue-derived stem cells derived from the tissue of fat, uterus, bone marrow, liver, muscle, placenta, nerve, umbilical cord blood, or skin (epithelium), and/or induced pluripotent stem cells (iPSCs). In one embodiment, the cell may be a mesenchymal stem cell. In one embodiment, the cells may be mesenchymal stem cells derived from bone marrow, adipose tissue, umbilical cord, placenta, amniotic fluid, and/or umbilical cord blood. In one embodiment, the cells may be bone marrow-derived mesenchymal stem cells.

Cell Genotype 1 - Gene to be Engineered

The cell having high adaptability to a hypoxic environment is characterized in that it is obtained by artificially editing a wild-type gene expressing a substance that inhibits transcription, expression, and/or activity of a HIF1 protein. In other words, the cell contains an engineered gene expressing a substance that inhibits the transcription, expression, and/or activity of the HIF1 protein.

In one embodiment, the artificially edited wild-type gene may be a HIF1AN, HIF3A, PHD2, TLR4, and/or PAI1 gene. In this case, the wild-type gene may be a human gene but is not limited thereto. In one embodiment, the cell may contain an engineered HIF1AN gene, an engineered HIF3A gene, an engineered PHD2 gene, an engineered TLR4 gene, and/or an engineered PAI1 gene. In this case, the sequence of the engineered gene is different from the original sequence of a wild-type gene corresponding to the engineered gene. Specifically, the sequence of the engineered HIF1AN gene is different from the sequence of the wild-type HIF1AN gene. Specifically, the sequence of the engineered HIF3A gene is different from the sequence of the wild-type HIF3A gene. Specifically, the sequence of the engineered PHD2 gene is different from the sequence of the wild-type PHD2 gene. Specifically, the sequence of the engineered TLR4 gene is different from the sequence of the wild-type TLR4 gene. Specifically, the sequence of the engineered PAI1 gene is different from the sequence of the wild-type PAI1 gene.

Cell Genotype 2 - Gene to be Engineered

The engineered gene is characterized in that it has a sequence different from that of the corresponding wild-type gene. The engineered gene may not be able to express the substance that can be expressed by the non-engineered wild-type gene. In the engineered gene, the substance expressed in the wild-type gene may be less expressed.

In one embodiment, the engineered gene may be in the form of a mutation of a wild-type gene. In this case, the mutation may not be a mutation that exists in nature but be an artificially generated mutation.

In one embodiment, the engineered gene may be a corresponding wild-type gene that is knocked out.

In one embodiment, the engineered gene may be a corresponding wild-type gene that is knocked down.

In one embodiment, the engineered gene may be a form in which one or more nucleotides are inserted, deleted, substituted, and/or inverted compared to a corresponding wild-type gene.

In one embodiment, the engineered gene may be a gene having an indel in the corresponding wild-type gene. In this case, the indel is generated by a non-homologous end joining (NHEJ) mechanism in the cell, and one or more nucleotides in the wild-type gene may be inserted, deleted, and/or substituted by the NHEJ mechanism. In this case, the expression "including an indel in a gene" refers to an insertion, deletion, and/or substitution of one or more nucleotides in a wild-type gene, and/or a result thereof.

For example, an engineered A gene having an indel in a wild-type A gene may have one or more nucleotides inserted at any position in the wild-type A gene. Alternatively, for example, an engineered A gene having an indel in a wild-type A gene may have one or more nucleotides deleted at any position in the wild-type A gene. Further alternatively, for example, an engineered A gene having an indel in a wild-type A gene may be a form in which one or more nucleotides at any position in the wild-type A gene are substituted.

Cell Genotype 3 - Examples of Engineering Regions in Target Genes

As described above, the cells having high adaptability to a hypoxic environment provided herein are characterized in that a wild-type gene expressing a substance that inhibits the transcription, expression, and/or activity of HIF1 protein has been artificially edited. This means that one or more regions in the wild-type gene have been engineered. In this case, the engineered region in the wild-type gene is not particularly limited as long as it is a region capable of achieving the above objective.

In one embodiment, the cell having high adaptability to a hypoxic environment may be a form in which an exon region, an intron region, and/or a regulatory region in a wild-type gene is edited. In this case, the wild-type gene may be selected from the group consisting of HIF1AN, HIF3A, PHD2, TLR4, and PAI1.

In one embodiment, the cell having high adaptability to a hypoxic environment may include one or more engineered genes that are forms in which one or more regions selected from among exon 1, exon 2, exon 3, exon 4, exon 5, ..., exon N in one or more wild-type genes selected from the group consisting of HIF1AN, HIF3A, PHD2, TLR4, and PAI1 genes are artificially edited. In this case, N is an arbitrary integer. In one embodiment, the cell having high adaptability to a hypoxic environment may include one or more engineered genes that are forms in which one or more regions selected from among intron 1, intron 2, intron 3, intron 4, intron 5, ..., intron M in one or more wild-type genes selected from the group consisting of HIF1AN, HIF3A, PHD2, TLR4, and PAI1 genes are artificially edited. In this case, M is an arbitrary integer.

In one embodiment, the cell may include an engineered HIF1AN gene that is a form in which exon 1 in a wild-type HIF1AN gene is artificially engineered. In one embodiment, the cell may include an engineered HIF3A gene that is a form in which one or more regions selected from the group consisting of exon 2, exon 3, exon 4, exon 5, and exon 6 in a wild-type HIF3A gene are artificially edited. In one embodiment, the cell may include an engineered PHD2 gene that is a form in which exon 1 in a wild-type PHD2 gene is artificially engineered. In one embodiment, the cell may include an engineered TLR4 gene that is a form in which one or more regions selected from the group consisting of exon 1, exon 2, and exon 3 in a wild-type TLR4 gene are artificially edited. In one embodiment, the cell may include an engineered PAI1 gene that is a form in which exon 2 in a wild-type PAI1 gene is artificially engineered.

In one embodiment, when the engineered cell includes an engineered gene having an indel in an HIF1AN gene, the indel is located in exon 1 of the HIF1AN gene. When the engineered cell includes an engineered gene having an indel in an HIF3A gene, the indel is located in one or more regions selected from the group consisting of exon 2, exon 3, exon 4, exon 5, and exon 6 of the HIF3A gene, When the engineered cell includes an engineered gene having an indel in an PHD2 gene, the indel is located in exon 1 region of the PHD2 gene, When the engineered cell includes an engineered gene having an indel in an TLR4 gene, the indel is located in one or more regions selected from the group consisting of exon 1, exon 2, and exon 3 of the TLR4 gene. When the engineered cell includes an engineered gene having an indel in an PAI1 gene, the indel is located in exon 2 of the PAI1 gene.

Cell Genotype 4 - Examples of Sequence to be Engineered

In one embodiment, the cell having high adaptability to a hypoxic environment provided herein may be a cell having an HIF1AN gene in which one or more sequences selected from SEQ ID NOs: 1 to 9 are artificially edited. In one embodiment, the cell may include an engineered HIF1AN gene, and the sequence of the engineered HIF1AN gene may not include one or more sequences selected from SEQ ID NOs: 1 to 9. In other words, a sequence identical to one or more sequences selected from SEQ ID NOs: 1 to 9 among the sequences of the engineered HIF1AN gene may not exist.

In one embodiment, the cell having high adaptability to a hypoxic environment provided herein may be a cell having an HIF3A gene in which one or more sequences selected from SEQ ID NOs: 10 to 34 are artificially edited. In one embodiment, the cell may include an engineered HIF3A gene, and the sequence of the engineered HIF3A gene may not include one or more sequences selected from SEQ ID NOs: 10 to 34. In other words, a sequence identical to one or more sequences selected from SEQ ID NOs: 10 to 34 among the sequences of the engineered HIF3A gene may not exist.

In one embodiment, the cell having high adaptability to a hypoxic environment provided herein may be a cell having an PHD2 gene in which one or more sequences selected from SEQ ID NOs: 35 to 38 are artificially edited. In one embodiment, the cell may include an engineered PHD2 gene, and the sequence of the engineered PHD2 gene may not include one or more sequences selected from SEQ ID NOs: 35 to 38. In other words, a sequence identical to one or more sequences selected from SEQ ID NOs: 35 to 38 among the sequences of the engineered PHD2 gene may not exist.

In one embodiment, the cell having high adaptability to a hypoxic environment provided herein may be a cell having an TLR4 gene in which one or more sequences selected from SEQ ID NOs: 39 to 59 are artificially edited. In one embodiment, the cell may include an engineered TLR4 gene, and the sequence of the engineered TLR4 gene may not include one or more sequences selected from SEQ ID NOs: 39 to 59. In other words, a sequence identical to one or more sequences selected from SEQ ID NOs: 39 to 59 among the sequences of the engineered TLR4 gene may not exist.

In one embodiment, the cell having high adaptability to a hypoxic environment provided herein may be a cell having an PAI1 gene in which one or more sequences selected from SEQ ID NOs: 60 to 71 are artificially edited. In one embodiment, the cell may include an engineered PAI1 gene, and the sequence of the engineered PAI1 gene may not include one or more sequences selected from SEQ ID NOs: 60 to 71. In other words, a sequence identical to one or more sequences selected from SEQ ID NOs: 60 to 71 among the sequences of the engineered PAI1 gene may not exist.

Cell Phenotype 1 - Reduction in Expression Product of Engineered Gene

In the cells having high adaptability to a hypoxic environment disclosed herein, a wild-type gene expressing a substance that inhibits the expression of the HIF1 protein is artificially engineered, so that the wild-type gene cannot express the substance or expresses a smaller amount of the substance. Accordingly, the cell exhibits a lower expression level for a HIF1 expression negative regulator than a corresponding wild-type cell.

In one embodiment, the cell having high adaptability to a hypoxic environment includes at least one engineered gene having an indel in a gene selected from the group consisting of wild-type HIF1AN, HIF3A, PHD2, TLR4, and PAI1 genes. In this case, the mRNA sequence expressed in the engineered gene may be different from the mRNA sequence expressed in the wild-type gene. In one embodiment, the cell having high adaptability to a hypoxic environment may include an engineered HIF1AN gene having an indel in a wild-type HIF1AN gene, and the mRNA sequence in the engineered HIF1AN gene may be different from the mRNA sequence expressed in the wild-type gene. In one embodiment, the cell having high adaptability to a hypoxic environment includes an engineered HIF3A gene having an indel in a wild-type HIF3A gene, and the mRNA sequence expressed in the engineered HIF3A gene may be different from the mRNA sequence expressed in the wild-type HIF3A gene. In one embodiment, the cell having high adaptability to a hypoxic environment includes an engineered PHD2 gene having an indel in a wild-type PHD2 gene, and the mRNA sequence expressed in the engineered PHD2 gene may be different from the mRNA sequence expressed in the wild-type PHD2 gene. In one embodiment, the cell having high adaptability to a hypoxic environment includes an engineered TLR4 gene having an indel in a wild-type TLR4 gene, and the mRNA sequence expressed in the engineered TLR4 gene may be different from the mRNA sequence expressed in the wild-type TLR4 gene. In one embodiment, the cell having high adaptability to a hypoxic environment includes an engineered PAI1 gene having an indel in a wild-type PAI1 gene, and the mRNA sequence expressed in the engineered PAI1 gene may be different from the mRNA sequence expressed in the wild-type PAI1 gene.

In one embodiment, the cell having high adaptability to a hypoxic environment includes at least one engineered gene having an indel in a gene selected from the group consisting of wild-type HIF1AN, HIF3A, PHD2, TLR4, and PAI1 genes. In this case, the mRNA sequence expressed in the engineered gene may be the same as the mRNA sequence expressed in the wild-type gene. In this case, in the cell having high adaptability to a hypoxic environment, the expression level of mRNA expressed in the engineered gene may be lower than that in the wild-type cell. In one embodiment, the cell having high adaptability to a hypoxic environment includes an engineered HIF1AN gene having an indel in a wild-type HIF1AN gene, the mRNA sequence expressed in the engineered HIF1AN gene may be the same as the mRNA sequence expressed in the wild-type HIF1AN gene. In addition, in the cell having high adaptability to a hypoxic environment, the expression level of mRNA expressed in the engineered HIF1AN gene may be lower than that in the wild-type HIF1AN gene. In one embodiment, the cell having high adaptability to a hypoxic environment may include an engineered HIF3A gene having an indel in a wild-type HIF3A gene, and the mRNA sequence expressed in the engineered HIF3A gene may be the same as the mRNA sequence expressed in the wild-type HIF3A gene. In this case, in the cell having high adaptability to a hypoxic environment, the expression level of mRNA expressed in the engineered HIF3A gene may be lower than that in the wild-type HIF3A gene. In one embodiment, the cell having high adaptability to a hypoxic environment may include an engineered PHD2 gene having an indel in a wild-type PHD2 gene, and the mRNA sequence expressed in the engineered PHD2 gene may be the same as the mRNA sequence expressed in the wild-type PHD2 gene. In this case, in the cell having high adaptability to a hypoxic environment, the expression level of mRNA expressed in the engineered PHD2 gene may be lower than that in the wild-type PHD2 gene. In one embodiment, the cell having high adaptability to a hypoxic environment may include an engineered TLR4 gene having an indel in a wild-type TLR4 gene, and the mRNA sequence expressed in the engineered TLR4 gene may be the same as the mRNA sequence expressed in the wild-type TLR4 gene. In this case, in the cell having high adaptability to a hypoxic environment, the expression level of mRNA expressed in the engineered TLR4 gene may be lower than the expression level of mRNA expressed in the wild-type TLR4 gene in the wild-type cell. In one embodiment, the cell having high adaptability to a hypoxic environment may include an engineered PAI1 gene having an indel in a wild-type PAI1 gene, and the mRNA sequence expressed in the engineered PAI1 gene may be the same as the mRNA sequence expressed in the wild-type PAI1 gene. In this case, in the cell having high adaptability to a hypoxic environment, the expression level of mRNA expressed in the engineered PAI1 gene may be lower than the expression level of mRNA expressed in the wild-type PAI1 gene in wild-type cells.

In one embodiment, the cell having high adaptability to a hypoxic environment may be a cell in which the amount of mRNA expressed from HIF1AN, HIF3A, PHD2, TLR4 and/or PAI1 is reduced. In one embodiment, in the cell having high adaptability to a hypoxic environment, the expression level of mRNA expressed from HIF1AN, HIF3A, PHD2, TLR4 and/or PAI1 is lower than that in wild-type cells.

In one embodiment, the cell having high adaptability to a hypoxic environment may be a cell in which the amount of a protein expressed from HIF1AN, HIF3A, PHD2, TLR4 and/or PAI1 is reduced. In one embodiment, in the cell having high adaptability to a hypoxic environment, the expression level of a protein expressed from HIF1AN, HIF3A, PHD2, TLR4 and/or PAI1 is lower than that in wild-type cells.

In one embodiment, when the cell having high adaptability to a hypoxic environment includes an engineered HIF1AN gene having an indel in a HIF1AN gene, the concentration of mRNA and/or FIH-1 expressed from the HIF1AN gene included in the cell may be lower than the concentration in wild-type cells. In one embodiment, when the cell having high adaptability to a hypoxic environment includes an engineered HIF1AN gene having an indel in a HIF1AN gene, the expression level of mRNA and/or FIH-1 expressed from the HIF1AN gene included in the cell may be lower than that in wild-type cells.

Cell Phenotype 2 - Examples of Quantitative Indicators Related to Reduction of Expression Product of Gene to be Engineered

In one embodiment, in the cell having high adaptability to a hypoxic environment, the expression level of mRNA expressed from HIF1AN, HIF3A, PHD2, TLR4 and/or PAI1 genes is about 0.9 times, about 0.85 times, about 0.8 times, about 0.75 times, about 0.7 times, about 0.65 times, about 0.6 times, about 0.55 times, about 0.5 times, about 0.45 times, about 0.4 times, about 0.35 times, about 0.3 times, about 0.25 times, about 0.2 times, about 0.15 times, about 0.10 times, about 0.05 times, about 0.04 times, about 0.03 times, about 0.02 times, or about 0.01 or less times compared to the expression level in wild-type cells. In one embodiment, the cells having high adaptability to a hypoxic environment may exhibit mRNA expression levels within the range of two numerical values selected in the previous sentence compared to wild-type cells. For example, cells having high adaptability to a hypoxic environment may exhibit mRNA expression levels in the range of about 0.6 times to about 0.7 times that of wild-type cells.

In one embodiment, in the cell having high adaptability to a hypoxic environment, the expression level of a protein expressed from HIF1AN, HIF3A, PHD2, TLR4 and/or PAI1 genes is about 0.9 times, about 0.85 times, about 0.8 times, about 0.75 times, about 0.7 times, about 0.65 times, about 0.6 times, about 0.55 times, about 0.5 times, about 0.45 times, about 0.4 times, about 0.35 times, about 0.3 times, about 0.25 times, about 0.2 times, about 0.15 times, about 0.10 times, about 0.05 times, about 0.04 times, about 0.03 times, about 0.02 times, or about 0.01 or less times compared to the expression level in wild-type cells. In one embodiment, the cells having high adaptability to a hypoxic environment may exhibit protein expression levels within the range of two numerical values selected in the previous sentence compared to wild-type cells. For example, cells having high adaptability to a hypoxic environment may exhibit protein expression levels in the range of about 0.6 times to about 0.7 times that of wild-type cells.

In one embodiment, the cell having high adaptability to a hypoxic environment exhibits a FIH-1 expression level that is about 0.9 times, about 0.85 times, about 0.8 times, about 0.75 times, about 0.7 times, about 0.65 times, about 0.6 times, about 0.55 times, about 0.5 times, about 0.45 times, about 0.4 times, about 0.35 times, about 0.3 times, about 0.25 times, about 0.2 times, about 0.15 times, about 0.10 times, about 0.05 times, about 0.04 times, about 0.03 times, about 0.02 times, or about 0.01 or less times compared to the expression level in wild-type cells. In one embodiment, the cells having high adaptability to a hypoxic environment may exhibit FIH-1 expression levels within the range of two numerical values selected in the previous sentence compared to wild-type cells. For example, cells having high adaptability to a hypoxic environment may exhibit FIH-1 expression levels in the range of about 0.6 times to about 0.7 times that of wild-type cells.

Cell Phenotype 3 - Regarding HIF$\alpha$ Expression

The cells having high adaptability to a hypoxic environment disclosed herein have a high intracellular HIF1$\alpha$ expression level or exhibits a high HIF1$\alpha$ activity.

In one embodiment, the cells having a high adaptability to a hypoxic environment may have a higher HIF1$\alpha$ expression level than wild-type cells. In this case, the HIF1$\alpha$ expression level may be the amount and/or concentration of HIF1$\alpha$ in the cell but is not limited thereto.

In one embodiment, the cells having a high adaptability to a hypoxic environment may exhibit a higher HIF1$\alpha$ activity than wild-type cells.

Cell Phenotype 4 - Examples of Quantitative Indicators Related to HIF1a Expression

In one embodiment, the cell having high adaptability to a hypoxic environment exhibits a HIF1$\alpha$ expression level that is about 1.1 times, about 1.2 times, about 1.3 times, about 1.4 times, about 1.5 times, about 1.6 times, about 1.7 times, about 1.8 times, about 1.9 times, about 2.0 times, about 2.1 times, about 2.2 times, about 2.3 times, about 2.4 times, about 2.5 times, about 2.6 times, about 2.7 times, about 2.8 times, about 2.9 times, or about 3.0 or more times the HIF1$\alpha$ expression level in wild-type cells. In one embodiment, the cells having high adaptability to a hypoxic environment may exhibit HIF1$\alpha$ expression levels within the range of two numerical values selected in the previous sentence compared to wild-type cells. For example, cells having high adaptability to a hypoxic environment may exhibit HIF1$\alpha$ expression levels in the range of about 1.1 times to about 1.5 times that of wild-type cells.

In one embodiment, the cell having high adaptability to a hypoxic environment exhibits a HIF1$\alpha$ activity level that is about 1.1 times, about 1.2 times, about 1.3 times, about 1.4 times, about 1.5 times, about 1.6 times, about 1.7 times, about 1.8 times, about 1.9 times, about 2.0 times, about 2.1 times, about 2.2 times, about 2.3 times, about 2.4 times, about 2.5 times, about 2.6 times, about 2.7 times, about 2.8 times, about 2.9 times, or about 3.0 or more times the HIF1$\alpha$ activity level in wild-type cells. In one embodiment, the cells having high adaptability to a hypoxic environment may exhibit HIF1$\alpha$ activity levels within the range of two numerical values selected in the previous sentence compared to wild-type cells. For example, cells having high adaptability to a hypoxic environment may exhibit HIF1$\alpha$ activity levels in the range of about 1.1 times to about 1.5 times that of wild-type cells.

Cell Characteristic 1 - High Viability in Hypoxic Environment

The cells having high adaptability to a hypoxic environment disclosed herein are characterized in that they exhibit improved adaptability or viability in the hypoxic environment. In this case, the term "hypoxic environment" means a gaseous environment containing a low concentration of oxygen ($O_2$). As a result, when the cells having high adaptability to a hypoxic environment are injected into the body of a living organism (for example, a human), the cells show improved adaptability or viability.

In one embodiment, the cell having a high adaptability to a hypoxic environment disclosed herein is characterized in that it survives for a longer period of time than a wild-type cell in the hypoxic environment.

Cell Characteristic 2 - Examples of Quantitative Indicators of High Survival Rate in Hypoxic Environment

In one embodiment, the hypoxic environment may mean a gaseous environment in which the concentration of $O_2$ is about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, or about 21% or less. In one embodiment, the hypoxic environment may be an artificially created environment that mimics the hypoxic environment. In one embodiment, the hypoxic environment may be an environment in which reactive oxidative stress (ROS) commonly occurring in the hypoxic environment is artificially induced by using $H_2O_2$.

In one embodiment, when one or more cells of the cells with high adaptability to a hypoxic environment disclosed herein and wild-type cells are placed in the same hypoxic environment for a certain period of time, the number of viable cells among the cells with high adaptability to a hypoxic environment disclosed herein is about 1.01 times, about 1.02 times, about 1.03 times, about 1.04 times, about 1.05 times, about 1.06 times, about 1.07 times, about 1.08 times, about 1.09 times, about 1.1 times, about 1.15 times, about 1.2 times, about 1.25 times, about 1.3 times, about 1.35 times, about 1.4 times, about 1.45 times, about 1.5 times, about 1.55 times, about 1.6 times, about 1.65 times, about 1.7 times, about 1.75 times, about 1.8 times, about 1.85 times, About 1.9 times, about 1.95 times, about 2.0 times, about 2.1 times, about 2.2 times, about 2.3 times, about 2.4 times, about 2.5 times, about 2.6 times, about 2.7 times, about 2.8 times, about 2.9 times, about 3.0 times, about 4 times, about 5 times, or about 10 or more times larger than the number of viable cells among the wild-type cells. In one embodiment, the number of viable cells having high adaptability to a hypoxic environment may be larger than the number of viable wild-type cells to the extent that is in the range of two numerical values selected in the previous sentence. In one embodiment, the number of viable cells having high adaptability to a hypoxic environment may be 1.5 to 2.5 times larger than the number of viable wild-type cells.

In one embodiment, the certain period of time may be about 10 minutes, about 30 minutes, about 1 hour, about 2 hours, about 3 hours, about 4 hours, about 6 hours, about 8 hours, about 12 hours, about 24 hours, about 48 hours, about 72 hours, about 98 hours, about 120 hours, about 1 week, about 2 weeks, about 3 weeks, about 4 weeks, or about a month or more. In one embodiment, the predetermined time may be a value in the range of two numerical values selected in the immediately preceding sentence. For example, the predetermined time may be in the range of about 3 hours to about 12 hours.

Cell Characteristic 3 - Cytokine Secretion

The cells having a high adaptability to a hypoxic environment disclosed herein may be cells that secrete a lot of specific cytokines or specific growth factors.

In one embodiment, the cells having a high adaptability to a hypoxic environment may have a higher cytokine secretion level than wild-type cells. In one embodiment, the cells having a high adaptability to a hypoxic environment may have a higher secretion level of VEGF and/or IL-8 compared to wild-type cells. In one embodiment, the cells having a high adaptability to a hypoxic environment may include an engineered HIF1AN gene, and may have a higher secretion level of VEGF and/or IL-8 than wild-type cells.

In one embodiment, the cells having a high adaptability to hypoxic environment may exhibit a higher cytokine secretion level under the hypoxic environment than wild-type cells. In one embodiment, the cells having a high adaptability to a hypoxic environment may have a higher VEGF and/or IL-8secretion level under the hypoxic environment than wild-type cells. In one embodiment, the cells having a high adaptability to a hypoxic environment may include an engineered HIF1AN gene, and may exhibit a higher secretion level of VEGF and/or IL-8 under the hypoxic environment than wild-type cells.

Cell Characteristic 4 - Examples of Quantitative Indicators for Cytokine Secretion

In one embodiment, the cells having a high adaptability to a hypoxic environment may secrete a specific cytokine in an amount that is about 1.1 times, about 1.2 times, about 1.3 times, about 1.4 times, about 1.5 times, about 1.6 times, about 1.7 times, about 1.8 times, about 1.9 times, about 2.0 times, about 2.1 times, about 2.2 times, about 2.3 times, about 2.4 times, about 2.5 times, about 2.6 times, about 2.7 times, about 2.8 times, about 2.9 times, or about 3.0 or more times larger than that of wild-type cells. In one embodiment, the cells having a high adaptability to hypoxic environment may secrete a specific cytokine in an amount within the range of two numerical values selected in the previous sentence, compared to wild-type cells. For example, the secreted amount may be 1.1 to 1.5 times larger. In this case, the specific cytokine may be VEGF and/or IL-8, but is not limited thereto.

Use of Cell

The cells having a high adaptability to a hypoxic environment disclosed herein can be used for cell therapeutics.

In one embodiment, the cells having a high adaptability to a hypoxic environment may be used to prevent the following diseases, relieve symptoms, regenerate cells, or treat the following diseases: cancer, Alzheimer's disease, arthritis including, for example, osteoarthritis, which is a degenerative joint disease caused by aging, rheumatoid arthritis and psoriatic arthritis, which are autoimmune diseases, and septic arthritis caused by infection. In another embodiment, the cells may be used for prophylaxis, symptom relief, regeneration, or treatment of diabetic retinopathy, damaged bones, cartilage, skin and organs such as myocardium, osteogenesis imperfecta (01), Crohn's fistula, diabetic foot ulcer, myocardial infarction, Huntington's disease, Parkinson's disease, Lou Gehrig's disease, active rickets, Paget's disease (osteitis deformans), progressive ossified fibrodysplasia, anterior arthropathy, and enteropathic arthritis.

Genetic Engineering Composition for Producing Cells having High Adaptability to Hypoxic Environment.

Overview

A genetic engineering composition for producing a cell having a high adaptability to a hypoxic environment disclosed herein is configured to be able to edit an engineering target gene in a cell, using a CRISPR/Cas system. The genetic engineering composition is designed to recognize and edit a suitable intracellular target sequence so that the genotype and/or phenotype of the aforementioned cell having a high adaptability to a hypoxic environment can be exhibited. In one embodiment, the genetic engineering composition may include a Cas9 protein or a DNA encoding the same, and a guide RNA or a DNA encoding the same. In one embodiment, the Cas9 protein may be a streptococcus pyogenes-derived Cas9 protein. In one embodiment, the sequence of the guide RNA may be a sequence selected from the group consisting of SEQ ID NOs: 146 to 216.

CRISPR/Cas System Component 1 - Cas9 Protein

The genetic engineering composition for producing the cells having a high adaptability to a hypoxic environment disclosed herein includes a Cas9 protein or a DNA encoding the same. In this case, the Cas9 protein may be a Cas9 protein existing in nature, or a Cas9 protein in which one or more domains of a naturally occurring Cas9 protein are artificially modified. In one embodiment, the Cas9 protein may have inactivated functions of some or all domains included in the Cas9 protein. In one embodiment, the Cas9 protein may be a streptococcus pyogenes-derived Cas9 protein.

CRISPR/Cas System Component 2 - Guide RNA

The genetic engineering composition for producing the cells having a high adaptability to a hypoxic environment disclosed herein includes a guide RNA or a DNA encoding the same. In this case, the guide RNA may bind to the Cas9 protein to form a CRISPR/Cas complex. The structure of the guide RNA can be largely divided into a scaffold portion and a guide sequence portion. The scaffold portion can interact with the Cas9 protein and bind to the Cas9 protein, and the constituent (sequence) thereof may vary depending on the type of Cas9 protein included in the genetic engineering composition. The guide sequence portion is a portion capable of forming a complementary bond with a specific sequence portion in an engineering target gene included in a cell, and allows the CRISPR/Cas complex to edit the specific sequence portion and/or a portion adjacent to the specific sequence portion. The guide sequence portion is designed to be able to complementary bind to a predetermined target sequence.

In one embodiment, the engineering target gene may be a HIF1AN, HIF3A, PHD2, TLR4, and/or PAI1 gene.

In one embodiment, the scaffold portion may include a tracrRNA and a crRNA repeat sequence portion. In one embodiment, the sequence of the tracrRNA may be the sequence of SEQ ID NO: 144. In one embodiment, the sequence of the tracrRNA may be a sequence that matches the sequence of SEQ ID NO: 144 by at least about 50%, at least about 60%, at least about 70%, at least about 80%, or at least about 90%. In one embodiment, the crRNA repeat sequence may be the sequence of SEQ ID NO: 145. In one embodiment, the crRNA repeat sequence may be a sequence that matches the sequence of SEQ ID NO: 145 by at least about 50%, at least about 60%, at least about 70%, at least about 80%, or at least about 90%. In one embodiment, the sequence of the scaffold portion may be the sequence of SEQ ID NO: 143. In one embodiment, the sequence of the scaffold portion may be a sequence that matches the sequence of SEQ ID NO: 143 by at least about 50%, at least about 60%, at least about 70%, at least about 80%, or at least about 90%.

In one embodiment, the guide sequence portion may be an RNA sequence corresponding to a DNA sequence adjacent to a proto-spacer adjacent motif (PAM) sequence included in the engineering target gene. In one embodiment, the sequence of the PAM may be 5'- NGG'-3'.

Example of Guide RNA Sequence

In one embodiment, the guide sequence portion of the guide RNA may be a sequence selected from the group consisting of SEQ ID NOs: 72 to 142. In one embodiment, the sequence of the guide RNA may be a sequence selected from the group consisting of SEQ ID NOs: 146 to 216.

Forms of Components of CRISPR/Cas System

The form of each component of the CRISPR/Cas system included in the genetic engineering composition is not particularly limited if the components can a form CRISPR/Cas complex and thus artificially manipulate a target gene when the genetic engineering composition is introduced into cells.

In one embodiment, the genetic engineering composition may include ribonucleoprotein (RNP) in which the Cas9 protein and the guide RNA are bound. In one embodiment, the genetic engineering composition may have a vector including DNA encoding the Cas9 protein and DNA encoding the guide RNA. In one embodiment, the genetic engineering composition may have a single vector including DNA encoding the Cas9 protein and DNA encoding the guide RNA. In one embodiment, the single vector may be a plasmid and/or a viral vector. In one embodiment, the viral vector may be one selected from the group consisting of retrovirus, lentivirus, adenovirus, adeno-associated virus, vaccinia virus, poxvirus, and herpes simplex virus.

Intracellular Genetic Engineering Method for Producing Cells having High Adaptability to Hypoxic Environment

Overview

The present disclosure discloses an intracellular genetic engineering method for producing cells having a high adaptability to a hypoxic environment. Specifically, it is a method of obtaining the desired cell by editing a gene that expresses a substance that inhibits the expression of a HIF1 protein in order to prepare the aforementioned cell having a high adaptability to a hypoxic environment. In one embodiment, the genetic engineering method may include introducing a genetic engineering composition into a cell including a target gene. In this case, the genetic engineering composition may be the one described in the section titled "genetic engineering Composition for Producing Cells having High adaptability to Hypoxic Environment". In one embodiment, the genetic engineering method may include bringing a CRISPR/Cas9 complex into contact with a target gene. In this case, in the CRISPR/Cas9 complex, a Streptococcus pyogenes-derived Cas9 protein and a guide RNA having a sequence selected from the group consisting of SEQ ID NOs: 146 to 216 may form a complex.

Environment in Which Genetic Engineering Method is Performed

In this case, the genetic engineering method may be performed in vitro. In addition, since the cells having a high adaptability to a hypoxic environment as described above can be used as a cell therapeutic agent, the genetic engineering method can be performed on cells isolated from an organism (ex vivo). In one embodiment, the genetic engineering method may be performed on cells isolated from an organism in vitro or ex vivo. In this case, the organism may be a human organism, but is not limited thereto.

Target Gene

In the genetic engineering method, a gene that is a target to be engineered is referred to as a target gene. The gene to be subjected to the genetic engineering is a wild-type gene expressing a substance that inhibits transcription, expression, and/or activity of a HIF1 protein. The target gene may be a gene described in the section "Cell Genotype 1 - Gene to be Engineered".

Composition for Genetic Engineering

The composition for genetic engineering used in the genetic engineering method disclosed herein may be the same one as described in the section titled "Genetic Engineering Composition for Producing Cells having High adaptability to Hypoxic Environment". In one embodiment, the composition for genetic engineering used in the genetic engineering method may include: a streptococcus pyogenes-derived Cas9 protein or DNA encoding the same; and one or more guide RNAs having a sequence selected from the group consisting of SEQ ID NOs: 146 to 216 or DNA encoding the one or more guide RNAs.

Examples of Means for Introducing Genetic Engineering Composition

In the genetic engineering method disclosed herein, the introduction of the genetic engineering composition into a cell can be performed in various ways. The way is not particularly limited as long as it is possible to achieve the purpose of editing a target gene with the CRISPR/Cas complex.

In one embodiment, the introduction of the genetic engineering composition into cells may be performed by one or more means selected from electroporation, lipofection, microinjection, gene extract, liposomes, positive liposomes, EnGeneIC delivery vehicles (EDV), plasmids, viral vectors, nanoparticles, protein translocation domain (PTD) fusion protein methods, immunoliposomes, polycations or lipids-nucleic acid conjugates, naked DNA, artificial virions, and methods for enhancing absorption of preparations of DNA. In one embodiment, when the genetic engineering composition includes ribonucleoprotein in which a Cas9 protein and a guide RNA are bound, the genetic engineering composition may be introduced into cells by electroshock or electroporation.

Introduction of Components of CRISPR/Cas System, Component by Component

When each component of the CRISPR/Cas system is independently included in the genetic engineering composition, the genetic engineering method may include a process of individually introducing each component of the CRISPR/Cas system into cells. In this case, each component of the CRISPR/Cas system may be introduced into the cell simultaneously or sequentially in any order. In one embodiment, when the genetic engineering composition includes DNA encoding the Cas protein and a guide RNA as separate components, the genetic engineering method may include a process of introducing the DNA encoding the Cas protein and the guide RNA into cells at the same time. In one embodiment, when the genetic engineering composition includes DNA encoding the Cas protein and a guide RNA as separate components, the genetic engineering method may include a process of sequentially introducing the DNA encoding the Cas protein and the guide RNA into cells in an arbitrary order. In one embodiment, when the genetic engineering composition includes DNA encoding the Cas protein and DNA encoding the guide RNA in two or more different vectors, the genetic engineering method may include a process of introducing each of the vectors into a cell at the same time. In one embodiment, when the genetic engineering composition includes the DNA encoding the Cas protein and the DNA encoding the guide RNA in two or more different vectors, the genetic engineering method may include a process of sequentially introducing each of the vectors in an arbitrary order.

Introduction of Genetic Engineering Composition

The genetic engineering method for producing a cell having a high adaptability to a hypoxic environment disclosed herein may include a process of introducing the genetic engineering composition into a cell.

Contact between CRISPR/CAS Complex and Target Gene

The genetic engineering method for producing a cell having a high adaptability to a hypoxic environment disclosed herein may include a process of bringing a CRISPR/Cas complex into contact with a target gene.

Result of Performing Genetic Engineering Method 1 - Gene Editing Site

When the genetic engineering method for producing a cell having a high adaptability to a hypoxic environment disclosed herein is performed, gene editing takes place in a specific portion of a target gene within a cell. In this case, the expression "gene editing takes place in a specific part" means that the specific portion and/or a part adjacent thereto is edited by a CRISPR/Cas complex.

In one embodiment, as a result of performing the genetic engineering method, gene editing may take place in a portion of a target sequence within a target gene in a cell, or a portion adjacent thereto. In one embodiment, as a result of performing the genetic engineering method, gene editing may take place in a portion of a target sequence included in the HIF1AN, HIF3A, PHD2, TLR4, and/or PAI1 gene in a cell.

In one embodiment, as a result of performing the genetic engineering method, gene editing may take place in an exon region, an intron region, and/or a regulatory region within the HIF1AN, HIF3A, PHD2, TLR4, and/or PAI1 gene in a cell. In one embodiment, as a result of performing the genetic engineering method, the gene editing may take place in one or more regions selected from exon 1, exon 2, exon 1, exon 2, exon 3, exon 4, exon 5, ..., and exon N in HIF1AN, HIF3A, PHD2, TLR4, and/or PAI1 genes in a cell. In this case, N is an arbitrary integer. In one embodiment, as a result of performing the genetic engineering method, the gene editing may take place in one or more regions selected from intron 1, intron 2, intron 3, intron 4, intron 5, ..., and intron M in HIF1AN, HIF3A, PHD2, TLR4, and/or PAI1 genes in a cell. In this case, M is an arbitrary integer. In one embodiment, as a result of performing the genetic engineering method, gene editing may take place in a sequence selected from the group consisting of SEQ ID NOs: 1 to 71 included in a target gene in a cell.

Result of Performing Genetic Engineering Method 2 - Aspects of Gene Editing

When the genetic engineering method for producing a cell having a high adaptability to a hypoxic environment disclosed herein is performed, gene editing takes place in a specific portion of a target gene within a cell. As a result of the gene editing, the expression level of the expression product of the target gene is lower than that of the wild-type cell. In this case, the expression product may be mRNA or a specific protein.

In one embodiment, as a result of performing the genetic engineering method, one or more nucleotides may be inserted, deleted, substituted with other nucleotides, and/or inverted in a specific portion and/or a portion adjacent thereto in the target gene. In one embodiment, as a result of performing the genetic engineering method, an indel may occur in a specific portion and/or a portion adjacent thereto within the target gene. In this case, the specific portion may be the portion exemplified in the section titled "Result 1 of Performing Genetic Engineering Method - Gene Editing Site". In one embodiment, as a result of performing the genetic engineering method, the target gene may be knocked out. In one embodiment, as a result of performing the genetic engineering method, the target gene may be knocked down.

Pharmaceutical Composition Including Cells with High Adaptability to Hypoxic Environment

Disclosed herein is a pharmaceutical composition including cells having a high adaptability to a hypoxic environment. The pharmaceutical composition includes cells having a high adaptability to a hypoxic environment as an active ingredient, and can be used for diagnosis, symptom relief, regeneration, and/or treatment of a target disease.

In one embodiment, the pharmaceutical composition may be administered to the body to replace damaged cells, or to treat or regenerate damaged tissues or organs, etc. and can be easily used by those skilled in the art.

In one embodiment, the pharmaceutical composition may further include a pharmaceutically acceptable carrier. In this case, the pharmaceutically acceptable carrier is a lubricant, wetting agent, sweetener, flavoring agent, emulsifying agent, suspending agent, preservative, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, saline, phosphate buffered saline (PBS), and/or medium, but is not limited thereto.

In one embodiment, the pharmaceutical composition or the cell, which is an active ingredient of the pharmaceutical composition, may be cultured in a hypoxic environment. Specifically, the pharmaceutical composition or the cell may be pre-conditioned in vitro or in vivo in a hypoxic environment.

In one embodiment, the pharmaceutical composition may be formulated in a parenteral dosage form. In this case, parenteral administration includes nasal administration, eye drop administration, intravenous injection, intramuscular injection, intraperitoneal injection, transdermal administration, subcutaneous injection, intravenous injection, intramuscular injection, brain tissue injection, hippocampus injection, caudate putamen (CPu) intra-injection, intra-articular injection, intra-chondral injection, or intra-thoracic injection, etc., but is not limited thereto.

In one embodiment, in order to formulate the pharmaceutical composition into a formulation for parenteral administration, the cells as an active ingredient and a stabilizer or buffer may be mixed together with water to prepare a solution or suspension. Furthermore, the pharmaceutical composition may be formulated as ampoules or a vial unit dosage form. In one embodiment, the pharmaceutical composition may contain adjuvants such as preservatives, stabilizers, wetting agents or emulsification accelerators, salts for regulating osmotic pressure and/or buffers, and other therapeutically useful substances. The pharmaceutical composition may be formulated by mixing, granulating, or coating, which are conventional methods.

Treatment Methods Using Cells with High Adaptability to Hypoxic Environment

Disclosed herein is a treatment method using cells having a high adaptability to a hypoxic environment or a pharmaceutical composition including the same cell. When cells having a high adaptability to a hypoxic environment are administered into the body of a living subject (for example, a human), they can provide a remarkable therapeutic effect while surviving for a long time in the hypoxic environment at the administration site. In this case, the therapeutic effect may be to reduce a dose per each administration or increase intervals between administrations.

The present disclosure provides a method for diagnosing a target disease, alleviating symptoms, regenerating damages cells, or treating a target disease by administering, to a target site, a pharmaceutical composition including cells having a high adaptability to hypoxic environment as an active ingredient.

In one embodiment, the pharmaceutical composition may be used alone or in combination with methods such as surgery, radiation therapy, hormone therapy, chemotherapy, and a method of using biological response modifiers.

In one embodiment, a suitable dosage of the pharmaceutical composition is determined by factors such as formulation method, administration method, patient's age, weight, sex, and medical condition, food, administration time, administration route, excretion rate, and response sensitivity. An ordinarily skilled practitioner can readily determine and prescribe a dosage effective for the desired treatment or prophylaxis.

**Mode for Disclosure**

**Experimental Example**

Hereinafter, the inventions provided by the present disclosure will be described in more detail with reference to experimental examples and examples. These examples are only for illustrating the present disclosure, and it will be apparent to those of ordinary skill in the art that the scope of the present disclosure is not to be construed as being limited by these examples.

Experimental Example 1: Preparation of Cells with High Adaptability to Hypoxic Environment

*Experimental Example 1-1: Culture of Mesenchymal Stem Cell*

MSCs derived from human bone marrow were purchased from Lonza (Walkersville, Maryland, USA). MSCs were cultured in MEM alpha 1× media (Gibco, Rockville, MD) containing 10% fetal bovine serum (FSB, Gibco) and Gentamicin 0.05 mg/mL (Thermo Fisher Scientific) at 37°C and 5% $CO_2$ conditions.

*Experimental example 1-2: Preparation of Guide RNA*

RNA was transcribed in vitro, using MEGA short script T7 kit (Ambion), according to the manufacturer's instructions. A template for sgRNA was prepared through annealing and extension of two complementary oligonucleotides.

The guide domain of the sgRNA for each target is shown in Tables 1 to 5 below.

[Table 1]

| Label | Guide Domain (5'to 3') | SEQ ID NO. | PAM | Indel (%) | Target Sequence Location (Exon) |
|---|---|---|---|---|---|
| Sp-hHIF1AN sgRNA1 | GAAGCUAUAACUGCGCAACU | 72 | GGG | 51.8 | 1 |
| Sp-hHIF1AN sgRNA2 | GGAAGCUAUAACUGCGCAAC | 73 | UGG | 9.5 | 1 |
| Sp-hHIF1AN sgRNA3 | GCAGUUAUAGCUUCCCGACU | 74 | AGG | 14.3 | 1 |
| Sp-hHIF1AN sgRNA4 | GACGCGGAAUGGGCCUAGUC | 75 | GGG | 2 | 1 |
| Sp-hHIF1AN sgRNA5 | AGACGCGGAAUGGGCCUAGU | 76 | CGG | 7.3 | 1 |
| Sp-hHIF1AN sgRNA6 | CUCUGACUCAGACGCGGAAU | 77 | GGG | 7.2 | 1 |
| Sp-hHIF1AN sgRNA7 | GGGUCGCUCUGACUCAGACG | 78 | CGG | 20.6 | 1 |
| Sp-hHIF1AN sgRNA8 | GUCUGAGUCAGAGCGACCCC | 79 | CGG | 26.5 | 1 |
| Sp-hHIF1AN sgRNA9 | CAAUAAGCUCCUCUGCCCGG | 80 | GGG | 59.8 | 1 |

[Table 2]

| Label | Guide Domain (5'to 3') | SEQ ID NO. | PAM | Indel (%) | Target Sequence Location (Exon) |
|---|---|---|---|---|---|
| Sp-hHIF3A sgRNA1 | GGUACAGCACCUCGGUCUCC | 81 | UGG | 4.1 | 2 |
| Sp-hHIF3A sgRNA2 | GACCGAGGUGCUGUACCAGC | 82 | UGG | 13 | 2 |
| Sp-hHIF3A sgRNA3 | GCAUGAUAGAGGCCUUGUCC | 83 | AGG | 10.8 | 2 |
| Sp-hHIF3A sgRNA4 | GAUGGUGAGGCGCAUGAUAG | 84 | AGG | 3.7 | 2 |
| Sp-hHIF3A sgRNA5 | GUGCAUGCGCAGGUAGCUGA | 85 | UGG | 6.4 | 2 |
| Sp-hHIF3A sgRNA6 | CAUCCACCCCUGUGACCAAG | 86 | AGG | 13.1 | 4 |
| Sp-hHIF3A sgRNA7 | GCUCCUCUUGGUCACAGGGG | 87 | UGG | 6.4 | 4 |
| Sp-hHIF3A sgRNA8 | GGGCGUCCUGAAGCUCCUCU | 88 | UGG | 3.3 | 4 |
| Sp-hHIF3A sgRNA9 | AGGAGAAGCACCGCUCCGUG | 89 | GGG | 1 | 5 |

[Table 3]

| Label | Guide Domain (5'to 3') | SEQ ID NO. | PAM | Indel (%) | Target Sequence Location (Exon) |
|---|---|---|---|---|---|
| Sp-hPHD2 sgRNA1 | AAUGACAGCGGCGGGCCCGG | 106 | CGG | 65.4 | 1 |
| Sp-hPHD2 sgRNA2 | AUGACAGCGGCGGGCCCGGC | 107 | GGG | 37.1 | 1 |
| Sp-hPHD2 sgRNA3 | ACUGCCGGUCUCGCUCGCUC | 108 | GGG | 90.6 | 1 |
| Sp-hPHD2 sgRNA4 | UACUGCCGGUCUCGCUCGCU | 109 | CGG | 50.1 | 1 |

[Table 4]

| Label | Guide Domain (5'to 3') | SEQ ID NO. | PAM | Indel (%) | Target Sequence Location (Exon) |
|---|---|---|---|---|---|
| Sp-TLR4 sgRNA1 | UGGUCUCACGCAGGAGAGGA | 110 | AGG | 1.1 | 1 |
| Sp-hTLR4 sgRNA2 | CCUGCGUGAGACCAGAAAGC | 111 | UGG | 1.3 | 1 |
| Sp-hTLR4 sgRNA3 | CCAGCUUUCUGGUCUCACGC | 112 | AGG | 3.2 | 1 |
| Sp-hTLR4 sgRNA4 | CUGCGUGAGACCAGAAAGCU | 113 | GGG | 2.9 | 1 |
| Sp-hTLR4 sgRNA5 | GGUUGAGAAGGGGAGGUUGU | 114 | AGG | 1.2 | 2 |
| Sp-hTLR4 sgRNA6 | GUCCAGGUUCUUGGUUGAGA | 115 | AGG | 1.1 | 2 |
| Sp-hTLR4 sgRNA7 | GGGGAUUAAAGCUCAGGUCC | 116 | GGG | 8.8 | 2 |
| Sp-hTLR4 sgRNA8 | ACCUGAGCUUUAAUCCCCUG | 117 | AGG | 0 | 3 |
| Sp-hTLR4 sgRNA9 | UAGCUGCCUAAAUGCCUCAG | 118 | AGG | 5.1 | 3 |
| Sp-hTLR4 sgRNA10 | AUGCCCCAUCUUCAAUUGUC | 119 | AGG | 2.9 | 3 |
| Sp-hTLR4 sgRNA11 | CUGUCAAUAUUAAGGUAGAG | 120 | AGG | 0.1 | 2 |
| Sp-hTLR4 sgRNA12 | CUCUCUACCUUAAUAUUGAC | 121 | AGG | 15.1 | 2 |
| Sp-hTLR4 sgRNA13 | GGGGUUUCCUGUCAAUAUUA | 122 | AGG | 0.1 | 3 |
| Sp-hTLR4 sgRNA14 | CCCCAUCCAGAGUUUAGCCC | 123 | UGC | 18.5 | 3 |
| Sp-hTLR4 sgRNA15 | CCAGGGCUAAACUCUGGAUG | 124 | GGG | 4.7 | 3 |

(continued)

| Label | Guide Domain (5'to 3') | SEQ ID NO. | PAM | Indel (%) | Target Sequence Location (Exon) |
|---|---|---|---|---|---|
| Sp-hTLR4 sgRNA16 | AGGCUCCCAGGGCUAAACUC | 125 | UGG | 26.6 | 3 |
| Sp-hTLR4 sgRNA17 | UAGUCCAGAAAAGGCUCCCA | 126 | GGG | 23 | 3 |
| Sp-hTLR4 sgRNA18 | UAAACUUGAUAGUCCAGAAA | 127 | AGG | 3 | 3 |
| Sp-hTLR4 sgRNA19 | AUCAAGUUUACAGAAGCUGG | 128 | UGG | 26.3 | 3 |
| Sp-hTLR4 sgRNA20 | UUUACAGAAGCUGGUGGCUG | 129 | UGG | 22.4 | 3 |
| Sp-hTLR4 sgRNA21 | UCUCUAGAGAACUUCCCCAU | 130 | UGG | 23.3 | 3 |

[Table 5]

| Label | Guide Domain (5'to 3') | SEQ ID NO. | PAM | Indel (%) | Target Sequence Location (Exon) |
|---|---|---|---|---|---|
| Sp-hPAI1 sgRNA1 | AGCCCUCACCUGCCUAGUCC | 131 | UGG | 5.6 | 2 |
| Sp-hPAI1 sgRNA2 | GCCCUCACCUGCCUAGUCCU | 132 | GGG | 22.1 | 2 |
| Sp-hPAI1 sgRNA3 | UGGCCCUUGUCUUUGGUGAA | 133 | GGG | 21.2 | 2 |
| Sp-hPAI1 sgRNA4 | GCACCAUCCCCCAUCCUACG | 134 | UGG | 50 | 2 |
| Sp-hPAI1 sgRNA5 | AGGUGGGCCACGUAGGAUGG | 135 | GGG | 11.6 | 2 |
| Sp-hPAI1 sgRNA6 | CCAGGUGGGCCACGUAGGAU | 136 | GGG | 8.6 | 2 |
| Sp-hPAI1 sgRNA7 | GCCCACCUGGCCUCAGACUU | 137 | CGG | 1.2 | 2 |
| Sp-hPAI1 sgRNA8 | UCACCCCGAAGUCUGAGGCC | 138 | AGG | 12 | 2 |
| Sp-hPAI1 sgRNA9 | CACCCUCACCCCGAAGUCUG | 139 | AGG | 5.4 | 2 |
| Sp-hPAI1 sgRNA10 | AACCACGUUGCGGUCCUUGG | 140 | AGG | 73.1 | 2 |
| Sp-hPAI1 sgRNA11 | GAAAACCACGUUGCGGUCCU | 141 | UGC | 7.6 | 2 |
| Sp-hPAI1 sgRNA12 | AGGGUGAGAAAACCACGUUG | 142 | CGG | 56.2 | 2 |

The nucleotide sequence (crRNA repeat sequence, linker (GAAA), and tracrRNA) which is linked to each guide domain is

5'-GTTTTAGAGCTAGAAATAGCAAGTTAAAATAAGGCTAGTCGTTATCAACTTGAAAAAGTGGCACCGAGTCGGTGCTTTTTTT-3' (SEQ ID NO: 143).

The full-length sequence of the sgRNA are shown in Tables 6 to 12 below.

[Table 6]

| Label | Guide RNA (5'to 3') | SEQ ID NO. |
|---|---|---|
| Sp-hHIF1AN sgRNA1 | GAAGCUAUAACUGCGCAACUGUUUUAGAGCUAGAAAUAGCAAGUUAAAAUAAGGCUAGUCCGUUAUCAACUUGAAAAAGUGGCACCGAGUCGGUGCUUUUUUU | 146 |
| Sp-hHIF1AN sgRNA2 | GGAAGCUAUAACUGCGCAACGUUUUAGAGCUAGAAAUAGCAAGUUAAAAUAAGGCUAGUCCGUUAUCAACUUGAAAAAGUGGCACCGAGUCGGUGCUUUUUUU | 147 |
| Sp-hHIF1AN sgRNA3 | GCAGUUAUAGCUUCCCGACUGUUUUAGAGCUAGAAAUAGCAAGUUAAAAUAAGGCUAGUCCGUUAUCAACUUGAAAAAGUGGCACCGAGUCGGUGCUUUUUUU | 148 |
| Sp-hHIF1AN sgRNA4 | GACGCGGAAUGGGCCUAGUCGUUUUAGAGCUAGAAAUAGCAAGUUAAAAUAAGGCUAGUCCGUUAUCAACUUGAAAAAGUGGCACCGAGUCGGUGCUUUUUUU | 149 |
| Sp-hHIF1AN sgRNA5 | AGACGCGGAAUGGGCCUAGUGUUUUAGAGCUAGAAAUAGCAAGUUAAAAUAAGGCUAGUCCGUUAUCAACUUGAAAAAGUGGCACCGAGUCGGUGCUUUUUUU | 150 |
| Sp-hHIF1AN sgRNA6 | CUCUGACUCAGACGCGGAAUGUUUUAGAGCUAGAAAUAGCAAGUUAAAAUAAGGCUAGUCCGUUAUCAACUUGAAAAAGUGGCACCGAGUCGGUGCUUUUUUU | 151 |
| Sp-hHIF1AN sgRNA7 | GGGUCGCUCUGACUCAGACGGUUUUAGAGCUAGAAAUAGCAAGUUAAAAUAAGGCUAGUCCGUUAUCAACUUGAAAAAGUGGCACCGAGUCGGUGCUUUUUUU | 152 |
| Sp-hHIF1AN sgRNA8 | GUCUGAGUCAGAGCGACCCCGUUUUAGAGCUAGAAAUAGCAAGUUAAAAUAAGGCUAGUCCGUUAUCAACUUGAAAAAGUGGCACCGAGUCGGUGCUUUUUUU | 153 |
| Sp-hHIF1AN sgRNA9 | CAAUAAGCUCCUCUGCCCGGGUUUUAGAGCUAGAAAUAGCAAGUUAAAAUAAGGCUAGUCCGUUAUCAACUUGAAAAAGUGGCACCGAGUCGGUGCUUUUUUU | 154 |

[Table 7]

| Label | Guide RNA (5'to 3') | SEQ ID NO. |
|---|---|---|
| Sp-hHIF3A sgRNA1 | GGUACAGCACCUCGGUCUCCGUUUUAGAGCUAGAAAUAGCAAGUUAAAAUAAGGCUAGUCCGUUAUCAACUUGAAAAAGUGGCACCGAGUCGGUGCUUUUUUU | 155 |
| Sp-hHIF3A sgRNA2 | GACCGAGGUGCUGUACCAGCGUUUUAGAGCUAGAAAUAGCAAGUUAAAAUAAGGCUAGUCCGUUAUCAACUUGAAAAAGUGGCACCGAGUCGGUGCUUUUUUU | 156 |

(continued)

| Label | Guide RNA (5'to 3') | SEQ ID NO. |
|---|---|---|
| Sp-hHIF3A sgRNA3 | GCAUGAUAGAGGCCUUGUCCGUUUUAGAGCUAGAAAUAGCAAGUUAAAAUAAGGCUAGUCCGUUAUCAACUUGAAAAAGUGGCACCGAGUCGGUGCUUUUUUU | 157 |
| Sp-hHIF3A sgRNA4 | GAUGGUGAGGCGCAUGAUAGGUUUUAGAGCUAGAAAUAGCAAGUUAAAAUAAGGCUAGUCCGUUAUCAACUUGAAAAAGUGGCACCGAGUCGGUGCUUUUUUU | 158 |
| Sp-hHIF3A sgRNA5 | GUGCAUGCGCAGGUAGCUGAGUUUUAGAGCUAGAAAUAGCAAGUUAAAAUAAGGCUAGUCCGUUAUCAACUUGAAAAAGUGGCACCGAGUCGGUGCUUUUUUU | 159 |
| Sp-hHIF3A sgRNA6 | CAUCCACCCCUGUGACCAAGGUUUUAGAGCUAGAAAUAGCAAGUUAAAAUAAGGCUAGUCCGUUAUCAACUUGAAAAAGUGGCACCGAGUCGGUGCUUUUUUU | 160 |
| Sp-hHIF3A sgRNA7 | GCUCCUCUUGGUCACAGGGGGUUUUAGAGCUAGAAAUAGCAAGUUAAAAUAAGGCUAGUCCGUUAUCAACUUGAAAAAGUGGCACCGAGUCGGUGCUUUUUUU | 161 |
| Sp-hHIF3A sgRNA8 | GGGCGUCCUGAAGCUCCUCUGUUUUAGAGCUAGAAAUAGCAAGUUAAAAUAAGGCUAGUCCGUUAUCAACUUGAAAAAGUGGCACCGAGUCGGUGCUUUUUUU | 162 |
| Sp-hHIF3A sgRNA9 | AGGAGAAGCACCGCUCCGUGGUUUUAGAGCUAGAAAUAGCAAGUUAAAAUAAGGCUAGUCCGUUAUCAACUUGAAAAAGUGGCACCGAGUCGGUGCUUUUUUU | 163 |

[Table 8]

| Label | Guide RNA (5'to 3') | SEQ ID NO |
|---|---|---|
| Sp-hHIF3A sgRNA14 | UUCAGGUAGCAGGCAUCCAGGUUUUAGAGCUAGAAAUAGCAAGUUAAAAUAAGGCUAGUCCGUUAUCAACUUGAAAAAGUGGCACCGAGUCGGUGCUUUUUUU | 168 |
| Sp-hHIF3A sgRNA15 | ACUGGAUGCCUGCUACCUGAGUUUUAGAGCUAGAAAUAGCAAGUUAAAAUAAGGCUAGUCCGUUAUCAACUUGAAAAAGUGGCACCGAGUCGGUGCUUUUUUU | 169 |
| Sp-hHIF3A sgRNA16 | CACCAUGACGAAGCCCUCCAGUUUUAGAGCUAGAAAUAGCAAGUUAAAAUAAGGCUAGUCCGUUAUCAACUUGAAAAAGUGGCACCGAGUCGGUGCUUUUUUU | 170 |
| Sp-hHIF3A sgRNA17 | CGUCAUGGUGCUCACCGCCGGUUUUAGAGCUAGAAAUAGCAAGUUAAAAUAAGGCUAGUCCGUUAUCAACUUGAAAAAGUGGCACCGAGUCGGUGCUUUUUUU | 171 |
| Sp-hHIF3A sgRNA18 | GCUCACCGCCGAGGGAGACAGUUUUAGAGCUAGAAAUAGCAAGUUAAAAUAAGGCUAGUCCGUUAUCAACUUGAAAAAGUGGCACCGAGUCGGUGCUUUUUUU | 172 |
| Sp-hHIF3A sgRNA19 | GGGAGACAUGGCUUACCUGUGUUUUAGAGCUAGAAAUAGCAAGUUAAAAUAAGGCUAGUCCGUUAUCAACUUGAAAAAGUGGCACCGAGUCGGUGCUUUUUUU | 173 |

(continued)

| Label | Guide RNA (5'to 3') | SEQ ID NO |
|---|---|---|
| Sp-hHIF3A sgRNA20 | GUUUGCUGACAUUCUCCGACGUUUUAGAGCUAGAAAUAGCAAGUUAAAAUAAGGCUAGUCCGUUAUCAACUUGAAAAAGUGGCACCGAGUCGGUGCUUUUUUU | 174 |
| Sp-hHIF3A sgRNA21 | AGUCUGCGCAGGUGGCUUGUGUUUUAGAGCUAGAAAUAGCAAGUUAAAAUAAGGCUAGUCCGUUAUCAACUUGAAAAAGUGGCACCGAGUCGGUGCUUUUUUU | 175 |
| Sp-hHIF3A sgRNA22 | GCUGGAGAAGUCUGCGCAGGGUUUUAGAGCUAGAAAUAGCAAGUUAAAAUAAGGCUAGUCCGUUAUCAACUUGAAAAAGUGGCACCGAGUCGGUGCUUUUUUU | 176 |
| Sp-hHIF3A sgRNA23 | CCUGCGCAGACUUCUCCAGCGUUUUAGAGCUAGAAAUAGCAAGUUAAAAUAAGGCUAGUCCGUUAUCAACUUGAAAAAGUGGCACCGAGUCGGUGCUUUUUUU | 177 |
| Sp-hHIF3A sgRNA24 | CCAGCUGGAGAAGUCUGCGCGUUUUAGAGCUAGAAAUAGCAAGUUAAAAUAAGGCUAGUCCGUUAUCAACUUGAAAAAGUGGCACCGAGUCGGUGCUUUUUUU | 178 |
| Sp-hHIF3A sgRNA25 | UGGCUUCGCAGAUGAGCACCGUUUUAGAGCUAGAAAUAGCAAGUUAAAAUAAGGCUAGUCCGUUAUCAACUUGAAAAAGUGGCACCGAGUCGGUGCUUUUUUU | 179 |

[Table 9]

| Label | Guide RNA (5'to 3') | SEQ ID NO. |
|---|---|---|
| Sp-hPHD2 sgRNA1 | AAUGACAGCGGCGGGCCCGGGUUUUAGAGCUAGAAAUAGCAAGUUAAAAUAAGGCUAGUCCGUUAUCAACUUGAAAAAGUGGCACCGAGUCGGUGCUUUUUUU | 180 |
| Sp-hPHD2 sgRNA2 | AUGACAGCGGCGGGCCCGGCGUUUUAGAGCUAGAAAUAGCAAGUUAAAAUAAGGCUAGUCCGUUAUCAACUUGAAAAAGUGGCACCGAGUCGGUGCUUUUUUU | 181 |
| Sp-hPHD2 sgRNA3 | ACUGCCGGUCUCGCUCGCUCGUUUUAGAGCUAGAAAUAGCAAGUUAAAAUAAGGCUAGUCCGUUAUCAACUUGAAAAAGUGGCACCGAGUCGGUGCUUUUUUU | 182 |
| Sp-hPHD2 sgRNA4 | UACUGCCGGUCUCGCUCGCUGUUUUAGAGCUAGAAAUAGCAAGUUAAAAUAAGGCUAGUCCGUUAUCAACUUGAAAAAGUGGCACCGAGUCGGUGCUUUUUUU | 183 |

[Table 10]

| Label | Guide RNA (5'to 3') | SEQ ID NO. |
|---|---|---|
| Sp-hTLR4 sgRNA1 | UGGUCUCACGCAGGAGAGGAGUUUUAGAGCUAGAAAUAGCAAGUUAAAAUAAGGCUAGUCCGUUAUCAACUUGAAAAAGUGGCACCGAGUCGGUGCUUUUUUU | 184 |

(continued)

| Label | Guide RNA (5'to 3') | SEQ ID NO. |
|---|---|---|
| Sp-hTLR4 sgRNA2 | CCUGCGUGAGACCAGAAAGCGUUUUAGAGCUAGAAAUAGCAAGUUAAAAUAAGGCUAGUCCGUUAUCAACUUGAAAAAGUGGCACCGAGUCGGUGCUUUUUUU | 185 |
| Sp-hTLR4 sgRNA3 | CCAGCUUUCUGGUCUCACGCGUUUUAGAGCUAGAAAUAGCAAGUUAAAAUAAGGCUAGUCCGUUAUCAACUUGAAAAAGUGGCACCGAGUCGGUGCUUUUUUU | 186 |
| Sp-hTLR4 sgRNA4 | CUGCGUGAGACCAGAAAGCUGUUUUAGAGCUAGAAAUAGCAAGUUAAAAUAAGGCUAGUCCGUUAUCAACUUGAAAAAGUGGCACCGAGUCGGUGCUUUUUUU | 187 |
| Sp-hTLR4 sgRNA5 | GGUUGAGAAGGGGAGGUUGUGUUUUAGAGCUAGAAAUAGCAAGUUAAAAUAAGGCUAGUCCGUUAUCAACUUGAAAAAGUGGCACCGAGUCGGUGCUUUUUUU | 188 |
| Sp-hTLR4 sgRNA6 | GUCCAGGUUCUUGGUUGAGAGUUUUAGAGCUAGAAAUAGCAAGUUAAAAUAAGGCUAGUCCGUUAUCAACUUGAAAAAGUGGCACCGAGUCGGUGCUUUUUUU | 189 |
| Sp-hTLR4 sgRNA7 | GGGGAUUAAAGCUCAGGUCCGUUUUAGAGCUAGAAAUAGCAAGUUAAAAUAAGGCUAGUCCGUUAUCAACUUGAAAAAGUGGCACCGAGUCGGUGCUUUUUUU | 190 |
| Sp-hTLR4 sgRNA8 | ACCUGAGCUUUAAUCCCCUGGUUUUAGAGCUAGAAAUAGCAAGUUAAAAUAAGGCUAGUCCGUUAUCAACUUGAAAAAGUGGCACCGAGUCGGUGCUUUUUUU | 191 |
| Sp-hTLR4 sgRNA9 | UAGCUGCCUAAAUGCCUCAGGUUUUAGAGCUAGAAAUAGCAAGUUAAAAUAAGGCUAGUCCGUUAUCAACUUGAAAAAGUGGCACCGAGUCGGUGCUUUUUUU | 192 |
| Sp-hTLR4 sgRNA10 | AUGCCCCAUCUUCAAUUGUCGUUUUAGAGCUAGAAAUAGCAAGUUAAAAUAAGGCUAGUCCGUUAUCAACUUGAAAAAGUGGCACCGAGUCGGUGCUUUUUUU | 193 |

Table 11

| Label | Guide RNA (5'to 3') | SEQ ID NO. |
|---|---|---|
| Sp-hTLR4 sgRNA11 | CUGUCAAUAUUAAGGUAGAGGUUUUAGAGCUAGAAAUAGCAAGUUAAAAUAAGGCUAGUCCGUUAUCAACUUGAAAAAGUGGCACCGAGUCGGUGCUUUUUUU | 194 |
| Sp-hTLR4 sgRNA12 | CUCUCUACCUUAAUAUUGACGUUUUAGAGCUAGAAAUAGCAAGUUAAAAUAAGGCUAGUCCGUUAUCAACUUGAAAAAGUGGCACCGAGUCGGUGCUUUUUUU | 195 |
| Sp-hTLR4 sgRNA13 | GGGGUUUCCUGUCAAUAUUAGUUUUAGAGCUAGAAAUAGCAAGUUAAAAUAAGGCUAGUCCGUUAUCAACUUGAAAAAGUGGCACCGAGUCGGUGCUUUUUUU | 196 |
| Sp-hTLR4 sgRNA14 | CCCCAUCCAGAGUUUAGCCCGUUUUAGAGCUAGAAAUAGCAAGUUAAAAUAAGGCUAGUCCGUUAUCAACUUGAAAAAGUGGCACCGAGUCGGUGCUUUUUUU | 197 |

(continued)

| Label | Guide RNA (5'to 3') | SEQ ID NO. |
|---|---|---|
| Sp-hTLR4 sgRNA15 | CCAGGGCUAAACUCUGGAUGGUUUUAGAGCUAGAAAUAGCAAGUUAA AAUAAGGCUAGUCCGUUAUCAACUUGAAAAAGUGGCACCGAGUCGGUG CUUUUUUU | 198 |
| Sp-hTLR4 sgRNA16 | AGGCUCCCAGGGCUAAACUCGUUUUAGAGCUAGAAAUAGCAAGUUAAA AUAAGGCUAGUCCGUUAUCAACUUGAAAAAGUGGCACCGAGUCGGUGC UUUUUUU | 199 |
| Sp-hTLR4 sgRNA17 | UAGUCCAGAAAAGGCUCCCAGUUUUAGAGCUAGAAAUAGCAAGUUAA AAUAAGGCUAGUCCGUUAUCAACUUGAAAAAGUGGCACCGAGUCGGUG CUUUUUUU | 200 |
| Sp-hTLR4 sgRNA18 | UAAACUUGAUAGUCCAGAAAGUUUUAGAGCUAGAAAUAGCAAGUUAA AAUAAGGCUAGUCCGUUAUCAACUUGAAAAAGUGGCACCGAGUCGGUG CUUUUUUU | 201 |
| Sp-hTLR4 sgRNA19 | AUCAAGUUUACAGAAGCUGGGUUUUAGAGCUAGAAAUAGCAAGUUAA AAUAAGGCUAGUCCGUUAUCAACUUGAAAAAGUGGCACCGAGUCGGUG CUUUUUUU | 202 |
| Sp-hTLR4 sgRNA20 | UUUACAGAAGCUGGUGGCUGGUUUUAGAGCUAGAAAUAGCAAGUUAA AAUAAGGCUAGUCCGUUAUCAACUUGAAAAAGUGGCACCGAGUCGGUG CUUUUUUU | 203 |
| Sp-hTLR4 sgRNA21 | UCUCUAGAGAACUUCCCCAUGUUUUAGAGCUAGAAAUAGCAAGUUAAA AUAAGGCUAGUCCGUUAUCAACUUGAAAAAGUGGCACCGAGUCGGUGC UUUUUUU | 204 |

[Table 12]

| Label | Guide RNA (5'to 3') | SEQ ID NO. |
|---|---|---|
| Sp-hPAI1 sgRNA1 | AGCCCUCACCUGCCUAGUCCGUUUUAGAGCUAGAAAUAGCAAGUUAAA AUAAGGCUAGUCCGUUAUCAACUUGAAAAAGUGGCACCGAGUCGGUGC UUUUUUU | 205 |
| Sp-hPAI1 sgRNA2 | GCCCUCACCUGCCUAGUCCUGUUUUAGAGCUAGAAAUAGCAAGUUAAA AUAAGGCUAGUCCGUUAUCAACUUGAAAAAGUGGCACCGAGUCGGUGC UUUUUUU | 206 |
| Sp-hPAI1 sgRNA3 | UGGCCCUUGUCUUUGGUGAAGUUUUAGAGCUAGAAAUAGCAAGUUAA AAUAAGGCUAGUCCGUUAUCAACUUGAAAAAGUGGCACCGAGUCGGUG CUUUUUUU | 207 |
| Sp-hPAI1 sgRNA4 | GCACCAUCCCCCAUCCUACGGUUUUAGAGCUAGAAAUAGCAAGUUAAA AUAAGGCUAGUCCGUUAUCAACUUGAAAAAGUGGCACCGAGUCGGUGC UUUUUUU | 208 |
| Sp-hPAI1 sgRNA5 | AGGUGGGCCACGUAGGAUGGGUUUUAGAGCUAGAAAUAGCAAGUUAA AAUAAGGCUAGUCCGUUAUCAACUUGAAAAAGUGGCACCGAGUCGGUG CUUUUUUU | 209 |
| Sp-hPAI1 sgRNA6 | CCAGGUGGGCCACGUAGGAUGUUUUAGAGCUAGAAAUAGCAAGUUAA AAUAAGGCUAGUCCGUUAUCAACUUGAAAAAGUGGCACCGAGUCGGUG CUUUUUUU | 210 |

(continued)

| Label | Guide RNA (5'to 3') | SEQ ID NO. |
|---|---|---|
| Sp-hPAI1 sgRNA7 | GCCCACCUGGCCUCAGACUUGUUUUAGAGCUAGAAAUAGCAAGUUAAA AUAAGGCUAGUCCGUUAUCAACUUGAAAAAGUGGCACCGAGUCGGUGC UUUUUUU | 211 |
| Sp-hPAI1 sgRNA8 | UCACCCCGAAGUCUGAGGCCGUUUUAGAGCUAGAAAUAGCAAGUUAAA AUAAGGCUAGUCCGUUAUCAACUUGAAAAAGUGGCACCGAGUCGGUGC UUUUUUU | 212 |
| Sp-hPAI1 sgRNA9 | CACCCUCACCCCGAAGUCUGGUUUUAGAGCUAGAAAUAGCAAGUUAAA AUAAGGCUAGUCCGUUAUCAACUUGAAAAAGUGGCACCGAGUCGGUGC UUUUUUU | 213 |
| Sp-hPAI1 sgRNA10 | AACCACGUUGCGGUCCUUGGGUUUUAGAGCUAGAAAUAGCAAGUUAA AAUAAGGCUAGUCCGUUAUCAACUUGAAAAAGUGGCACCGAGUCGGUG CUUUUUUU | 214 |
| Sp-hPAI1 sgRNA11 | GAAAACCACGUUGCGGUCCUGUUUUAGAGCUAGAAAUAGCAAGUUAA AAUAAGGCUAGUCCGUUAUCAACUUGAAAAAGUGGCACCGAGUCGGUG CUUUUUUU | 215 |
| Sp-hPAI1 sgRNA12 | AGGGUGAGAAAACCACGUUGGUUUUAGAGCUAGAAAUAGCAAGUUAA AAUAAGGCUAGUCCGUUAUCAACUUGAAAAAGUGGCACCGAGUCGGUG CUUUUUUU | 216 |

*Experimental Example 1-3: Transfection*

MSCs were transduced using the Amaxa P1 Primary Cell 4D Nucleofector kit with Program EW-104 according to the manufacturer's instructions. Specifically, Cas9 protein (4$\mu$g) premixed with ex vivo transcribed sgRNA (4$\mu$g) was incubated for 10 minutes at room temperature, and then transduced into $4 \times 10^5$ cells.

*Experimental example 1-4: Targeted Deep Sequencing*

Genomic DNAs covering target segment and potential non-target segment were amplified using Phusion polymerase (New England BioLabs). The PCR amplicon products were subjected to paired-end sequencing using Illumina MiSeq.

*Experimental Example 1-5: qRT-PCR*

Total RNA samples were obtained using RNeasy Mini Kit (Qiagen), and cDNA was synthesized from 1 ug total RNA, using ReverTra Ace qPCR RT Kit (Toyobo). Quantitative RT-PCR was performed on the synthesized cDNA using Power SYBR Green PCR Master Mix (Applied Biosystems) and StepOnePlus Real Time PCR system (Applied biosystems). The PCR temperature, time, and number of cycles are as follows. DNA denaturation was carried out at 95°C for 10 minutes, primer annealing was performed at 55°C to 60°C for 30 seconds, and polymerization was performed at 72°C for 30 seconds. Amplification was achieved by repeating 40 cycles. As a normalization control, beta-actin (ACTB) was used.

Experimental Example 2: Measurement of Viability of Cells with High Adaptability to Hypoxic Environment

*Experimental example 2-1: Measurement of Viability under Reactive-Oxygen Stress*

Changes in the proliferative capacity of MSCs were measured for reactive oxidative stress (ROS), which is common in hypoxic conditions. $1\times10^4$ cells were cultured in each well of a 96-well plate 24 hours before peroxide ($H_2O_2$) treatment inducing ROS. After 24 hours, 500 $\mu$M of peroxide ($H_2O_2$) was added to a serum-free medium and incubated. In order to check the cell viability 24 hours after the $H_2O_2$ treatment, the cell viability was measured with a CCK-8 cell proliferation kit (Dojindo®, USA).

The results of the experiment are shown in FIG. 16.

*Experimental example 2-2: Measurement of Cell Viability against Reactive Nitrogen Species (SNAP)*

To check changes in cell viability according to the concentration of S-Nitroso-N-acetyl-DL-penicillamine (SNAP, N3398)(Sigma), the number of viable cells for each group were counted using a Cell Counting Kit-8 (CCK-8). Cells grown in the 96-well plates were treated with SNAP at a concentration in the range of 0 to 500 μM and then cultured for 24 hours. Next, 10 μL of the CCK-8 solution was added to each well, followed by reaction for 2 hours. Absorbance was measured at 450 nm with an ELISA reader.
The results of the experiment are shown in FIG. 20.

Experimental Example 3: Characterization of Cells with High Adaptability to Hypoxic Environment

*Experimental Example 3-1: Experiment subject Cell*

The experiment subject cells were prepared as follows. The human bone marrow-derived mesenchymal stem cells cultured in Experimental Example 1-1 were treated with the hHIFIAN gene engineering composition prepared in Experimental Example 1-2, and then the cells suitable for this Experimental Example were selected through deep sequencing. As a control in this experiment, SHS231 KO and/or AAVS1 was used, in which SHS231 KO is cells in which the SHS231 genetic sequence location is engineered, and AAVS1 is cells in which the AAVS1 genetic sequence location is engineered.

*Experimental example 3-2: BrdU Cell Proliferation Assay*

Before harvesting the cells selected in Experimental Example 3-1, the cells were treated with 10uM BrdU for 1 hour. After the treatment with BrdU, the cells were fixed with 3% formaldehyde diluted in PBS at 4°C for 1 hour, then harvested, and then treated with 1% Triton X-100 at room temperature for 5 minutes. The cells were centrifuged, washed with PBS, and treated with 4N HCl at room temperature for 10 minutes to unwind DNA double strands, and then washed with PBS. After treatment with a blocking solution (30% FBS, 1% BSA, 0.01% Tween 20 in PBS) at room temperature for 30 minutes, the cells were harvested. Anti-BrdU mouse IgG diluted 100 times in PBS was reacted at 4°C for 30 minutes. The reaction product was washed with 0.2% tween 20 diluted in PBS, centrifuged, and collected. The collected cells were finally diluted with PBS and analyzed by flow cytometry.
The results of the BrdU cell proliferation assay are shown in FIGS. 21 to 22.

*Experimental Example 3-3: FACS Analysis*

After washing the MSCs selected in Experimental Example 3-1 twice with phosphate buffer saline (PBS), cells were isolated using 0.05% trypsin-EDTA and then centrifuged at 1,000 rpm for 5 minutes. The cells were suspended in 100ul of FACS staining buffer, mixed with the antibody, reacted at 4°C for 1 hour, washed twice with PBS, and suspended in 500ul of PBS, followed by FACS analysis.
The results of the FACS analysis are shown in FIGS. 23 to 25.

*Experimental example 3-4: Proteomics Data Validation via* Cytokine array

Cytokine array was performed with a Human XL Cytokine Array Kit (Cat. no.ARY022, R&D systems, USA). The method was performed according to the manufacturer's instructions. The array membrane was firstly soaked in a 4-well multi-dish in array buffer 6 and blocked for 1 hour in a rocking platform shaker. After the blocking, the array membrane of the desired sample volume was incubated overnight in the rocking platform shaker at 2°C to 8°C. Then, the array membrane was washed 3 times for 10 minutes with 1X wash buffer. Then, 1.5 mL of diluted detection antibody cocktail was added per well and incubated for 1 hour in a shaker. Then, the array membrane was washed 3 times again with 1X wash buffer. After the washing, the array membrane was incubated with 2 mL of 1X Streptavidin-HRP for 30 minutes. Finally, 1 mL of Chemi Reagent mix was evenly applied to each membrane, and was subjected to autoradiography. The pixel density of the X-ray film was scanned and analyzed with image analysis software (Image J).
The analysis results are shown in FIG. 28.

*Experimental Example 3-5: Measurement of Population Doubling Level (PDL) and Population Doubling Time (PDT)*

To check the growth of cells, the number of cells was measured before and after passage. The value obtained by dividing a harvest cell count ($C_t$) by a seeding cell count ($C_i$) was regarded as the growth rate, and the PDL (N) was calculated using the equation shown below.

$$2^n = \frac{C_t}{C_i}$$

The value obtained by dividing the incubation time (hr) by the PDL was expressed as population doubling time (PDT).

The PDL and PDT measurement results are shown in FIGS. 26 to 27.

Experimental Example 4: Result Reproduction Experiment

*Experimental Example 4-1: Result Reproduction Experiment*

In order to check the reproducibility of the result of Experimental Example 3, the experiments of Experimental Examples 1 to 3 were repeated with a new MSC stock. The results of the repeated experiment are shown in FIGS. 29 to 30.

*Experimental Example 4-2: Western Blot Analysis*

To create a hypoxic environment, stem cells (MSCs) were cultured in a hypoxic chamber in which the concentration of $O_2$ was maintained at 1%. To perform the Western blot, the MSCs were collected and then treated with a lysis buffer containing PBS, 1% Triton X-100, and phosphatase Inhibitor Cocktail II, III, and a complete protease inhibitor mixture. After the treatment, the lysate was centrifuged at 100,000 g at 4°C for 20 minutes. The supernatant (nonionic detergent-insoluble) of the centrifugation result was collected. For total lysates, cells were harvested, washed twice with PBS, and lysed with Pierce RIPA buffer (150 mM NaCl, 50 mM Tris, pH 8.0, 1% NP40, 1% SDS, and 0.5% sodium deoxycholate, and a protease inhibitor mixture; Thermo Scientific) on ice for 30 minutes. Protein concentration was measured using a BCA protein assay kit (Thermo Scientific). The equal amount of protein (10 to 20 μg) was dissolved in 8% to 16% SDS-PAGE and transferred to a nitrocellulose (NC) membrane. After washing with TBST (tris-buffer solution-Tween20; 10 mM Tris-HCl [pH 7.6], 150 mM NaCl, and 0.05% Tween-20), the membrane was blocked with 5% skim milk for 1 hour and then incubated along with an appropriate primary antibody in a diluent recommended by the supplier. The membrane was washed with TBST, and the primary antibody was detected with an HRP-conjugated secondary antibody. Immunoblot signals were visualized by Chemiluminescence (Pierce, USA).

The Western blot analysis results are shown in FIG. 31.

**Industrial Applicability**

The present disclosure provides cells having high adaptability under hypoxic conditions and a preparation method therefor. The cells having high adaptability under hypoxic conditions can be used as a cell therapeutic agent. Through the method for producing cells having high adaptability under hypoxic conditions, it is possible to produce cells having high adaptability under hypoxic conditions that can be used industrially.

**Claims**

**1.** An engineered cell comprising:

at least one engineered gene having an indel in a **wild-type gene** selected from the group consisting of HIF1AN, HIF3A, PHD2, TLR4 and PAI1,
wherein a sequence of the engineered gene is different from the sequence of the wild-type gene,
wherein an mRNA transcribed from the engineered gene in the engineered cell exhibits a lower expression level or has a different sequence than an mRNA transcribed from the wild-type gene in a wild-type cell, and
wherein the expression level of HIFα in the engineered cell is higher than the expression level of HIFα in the wild-type cell.

**2.** The engineered cell of claim 1,

wherein when the engineered cell comprises an engineered gene having an indel in an HIF1AN gene, the indel in the HIF1AN gene is located in exon 1 of the HIF1AN gene,
wherein when the engineered cell comprises an engineered gene having an indel in an HIF3A gene, the indel in the HIF3A gene is located in one or more regions selected from the group consisting of exon 2, exon 3, exon 4, exon 5, and exon 6 of the HIF3A gene,
wherein when the engineered cell comprises an engineered gene having an indel in an PHD2 gene, the indel in the PHD2 gene is located in exon 1 of the PHD2 gene,
wherein when the engineered cell comprises an engineered gene having an indel in an TLR4 gene, the indel in the TLR4 gene is located in one or more regions selected from the group consisting of exon 1, exon 2, and exon 3 of the TLR4 gene, and
wherein when the engineered cell comprises an engineered gene having an indel in a PAI1 gene, the indel in the PAI1 gene is located in exon 2 of the PAI1 gene.

**3.** The engineered cell of claim 1, wherein when the engineered cell comprises an engineered gene having an indel in an HIF1AN gene, the engineered gene does not comprise one or more sequences selected from the group consisting of SEQ ID NOs:1 to 9,

wherein when the engineered cell comprises an engineered gene having an indel in an HIF3A gene, the engineered gene does not comprise one or more sequences selected from the group consisting of SEQ ID NOs:10 to 34,
wherein when the engineered cell comprises an engineered gene having an indel in an PHD2 gene, the engineered gene does not comprise one or more sequences selected from the group consisting of SEQ ID NOs:35 to 38,
wherein when the engineered cell comprises an engineered gene having an indel in an TLR4 gene, the engineered gene does not comprise one or more sequences selected from the group consisting of SEQ ID NOs:39 to 59, and
wherein when the engineered cell comprises an engineered gene having an indel in an PAI1 gene, the engineered gene does not comprise one or more sequences selected from the group consisting of SEQ ID NOs:60 to 71.

**4.** The engineered cell of claim 1, wherein the engineered cell comprises an engineered gene having an indel in an HIF1AN gene.

**5.** The engineered cell of claim 4, wherein the indel in the HIF1AN gene is located in exon 1 of the HIF1AN gene.

**6.** The engineered cell of claim 4, wherein the sequence of the engineered gene does not comprise one or more sequences selected from the group consisting of SEQ ID NOs:1 to 9.

**7.** The engineered cell of claim 4, wherein the expression level of FIH-1 in the engineered cell is 70% or lower than the expression level of FIH-1 in the wild-type cell.

**8.** The engineered cell of claim 4, wherein the expression level of VEGF and IL-8 in the engineered cell is higher than the expression level of VEGF and IL-8 in the wild-type cell.

**9.** The engineered cell of claim 1, wherein the engineered cell comprises an engineered gene having an indel in an HIF3A gene.

**10.** The engineered cell of claim 9, wherein the indel in the HIF3A gene is located in one or more regions selected from the group consisting of exon 2, exon 3, exon 4, exon 5, and exon 6.

**11.** The engineered cell of claim 9, wherein the sequence of the engineered gene does not comprise one or more sequences selected from the group consisting of SEQ ID NOs:10 to 34.

**12.** The engineered cell of claim 1, wherein the engineered cell comprises an engineered gene having an indel in an PHD2 gene.

**13.** The engineered cell of claim 12, wherein the indel in the PHD2 gene is located in exon 1 of the PHD2 gene.

**14.** The engineered cell of claim 12, wherein the sequence of the engineered gene does not comprise one or more sequences selected from the group consisting of SEQ ID NOs:35 to 38.

**15.** The engineered cell of claim 1, wherein the engineered cell comprises an engineered gene having an indel in a TLR4 gene.

**16.** The engineered cell of claim 15, wherein the indel in the TLR4 gene is located in one or more regions selected from the group consisting of exon 1, exon 2, and exon 3.

**17.** The engineered cell of claim 15, wherein the sequence of the engineered gene does not comprise one or more sequences selected from the group consisting of SEQ ID NOs:39 to 59.

**18.** The engineered cell of claim 1, wherein the engineered cell comprises an engineered gene having an indel in a PAI1 gene.

**19.** The engineered cell of claim 18, wherein the indel in the PAI1 gene is located in exon 2 of the PAI1 gene.

**20.** The engineered cell of claim 18, wherein the sequence of the engineered gene does not comprise one or more sequences selected from the group consisting of SEQ ID NOs:60 to 71.

**21.** The engineered cell of claim 1, wherein the engineered cell is a mesenchymal stem cell derived from a tissue selected from the group consisting of bone marrow, adipose tissue, umbilical cord, placenta, amniotic fluid, and umbilical cord blood.

**22.** The engineered cell of claim 21, wherein the engineered cell is a mesenchymal stem cell derived from bone marrow.

**23.** An engineered cell comprising:

at least one engineered gene selected from the group consisting of an engineered HIF1AN gene, an engineered HIF3A gene, an engineered PHD2 gene, an engineered TLR4 gene, and an engineered PAI1 gene,
wherein the engineered HIF1AN gene does not comprise one or more sequences selected from SEQ ID NOs:1 to 9,
wherein the engineered HIF3A gene does not comprise one or more sequences selected from SEQ ID NOs:10 to 34,
wherein the engineered PHD2 gene does not comprise one or more sequences selected from SEQ ID NOs:35 to 38,
wherein the engineered TLR4 gene does not comprise one or more sequences selected from SEQ ID NOs:39 to 59, and
wherein the engineered PAI1 gene does not comprise one or more sequences selected from SEQ ID NOs:60 to 71.

**24.** A composition for gene editing, the composition comprising: a Cas9 protein derived from *streptococcus pyogenes* or a DNA encoding the Cas9 protein; and a guide RNA, or a DNA encoding the guide RNA, wherein the guide RNA has a sequence selected from the group consisting of SEQ ID NOs:146 to 216, or a 80% or more identical sequence to the selected sequence.

**25.** The composition of claim 24, the composition comprising the Cas9 protein and the guide RNA in the form of ribonucleoprotein (RNP).

**26.** The composition of claim 24, the composition comprising the DNA encoding the Cas9 protein and the DNA encoding the guide RNA in the form of a single vector.

**27.** The composition of claim 26, wherein the single vector is selected from the group consisting of plasmid, retrovirus, lentivirus, adenovirus, adeno-associated virus, vaccinia virus, poxvirus, and herpes simplex virus.

**28.** A method for editing a gene in a cell, the method comprising: introducing a gene-editing composition into the cell, wherein the gene-editing composition comprises: a *streptococcus* pyogenes-derived Cas9 protein, or a DNA encoding the Cas9 protein; and

a guide RNA or a DNA encoding the guide RNA, and
wherein the guide RNA has a sequence selected from the group consisting of SEQ ID NOs:146 to 216, or a 80% or more identical sequence to the selected sequence.

**29.** The method of claim 29, wherein the composition comprises the DNA encoding the Cas9 protein and the DNA encoding the guide RNA in the form of a single vector.

**30.** A method for editing a gene including a target DNA in a cell, the method comprising bringing a CRISPR/Cas9 complex into contact with a gene including a target DNA,
wherein the CRISPR/Cas9 complex comprises: a Cas9 protein derived from *streptococcus pyogenes;* and a guide RNA targeting the target DNA, wherein the guide RNA has a sequence selected from the group consisting of SEQ ID NOs:146 to 216, or a 80% or more identical sequence to the selected sequence.

FIG. 1

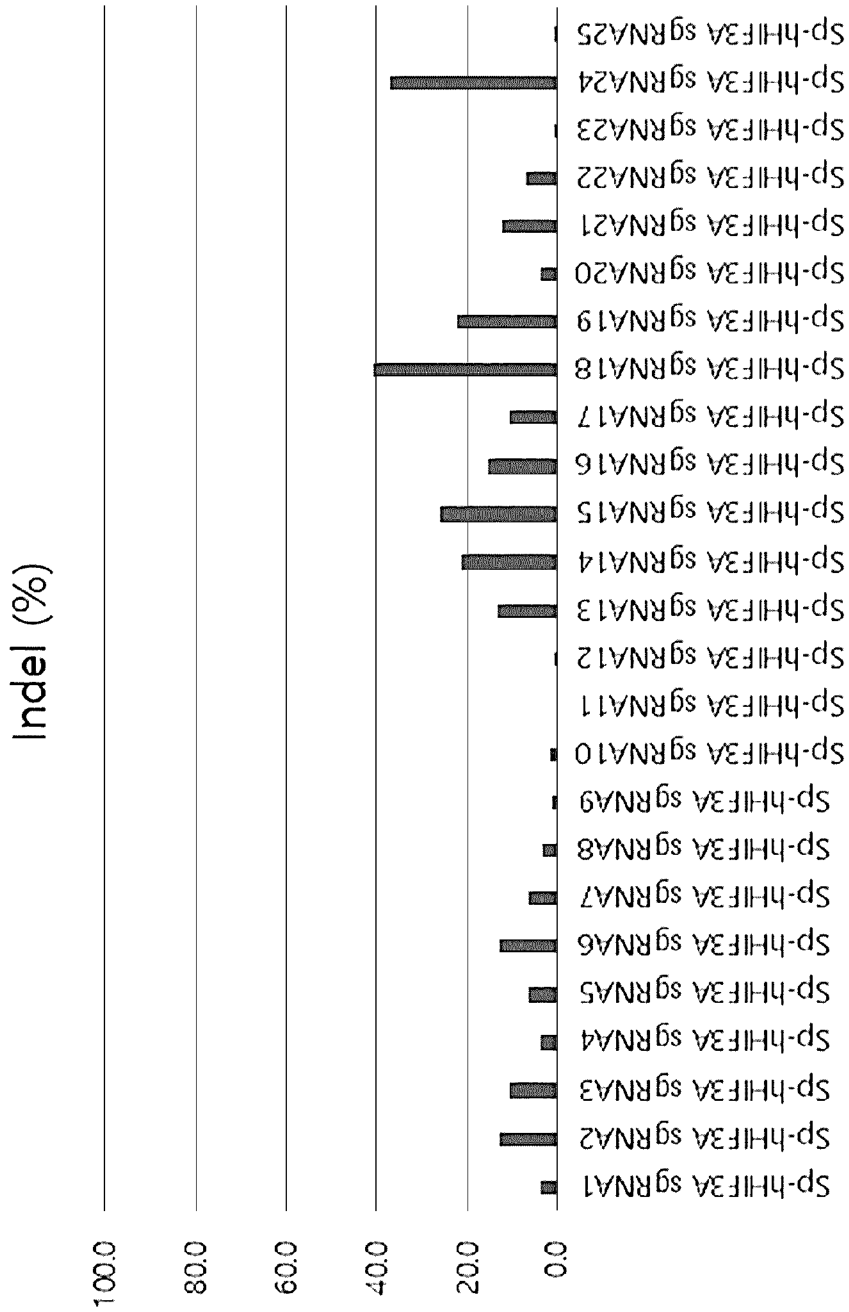
Indel (%)
100.0
80.0
60.0
40.0
20.0
0.0
Sp-hHIF3A sgRNA1
Sp-hHIF3A sgRNA2
Sp-hHIF3A sgRNA3
Sp-hHIF3A sgRNA4
Sp-hHIF3A sgRNA5
Sp-hHIF3A sgRNA6
Sp-hHIF3A sgRNA7
Sp-hHIF3A sgRNA8
Sp-hHIF3A sgRNA9
Sp-hHIF3A sgRNA10
Sp-hHIF3A sgRNA11
Sp-hHIF3A sgRNA12
Sp-hHIF3A sgRNA13
Sp-hHIF3A sgRNA14
Sp-hHIF3A sgRNA15
Sp-hHIF3A sgRNA16
Sp-hHIF3A sgRNA17
Sp-hHIF3A sgRNA18
Sp-hHIF3A sgRNA19
Sp-hHIF3A sgRNA20
Sp-hHIF3A sgRNA21
Sp-hHIF3A sgRNA22
Sp-hHIF3A sgRNA23
Sp-hHIF3A sgRNA24
Sp-hHIF3A sgRNA25

FIG. 2

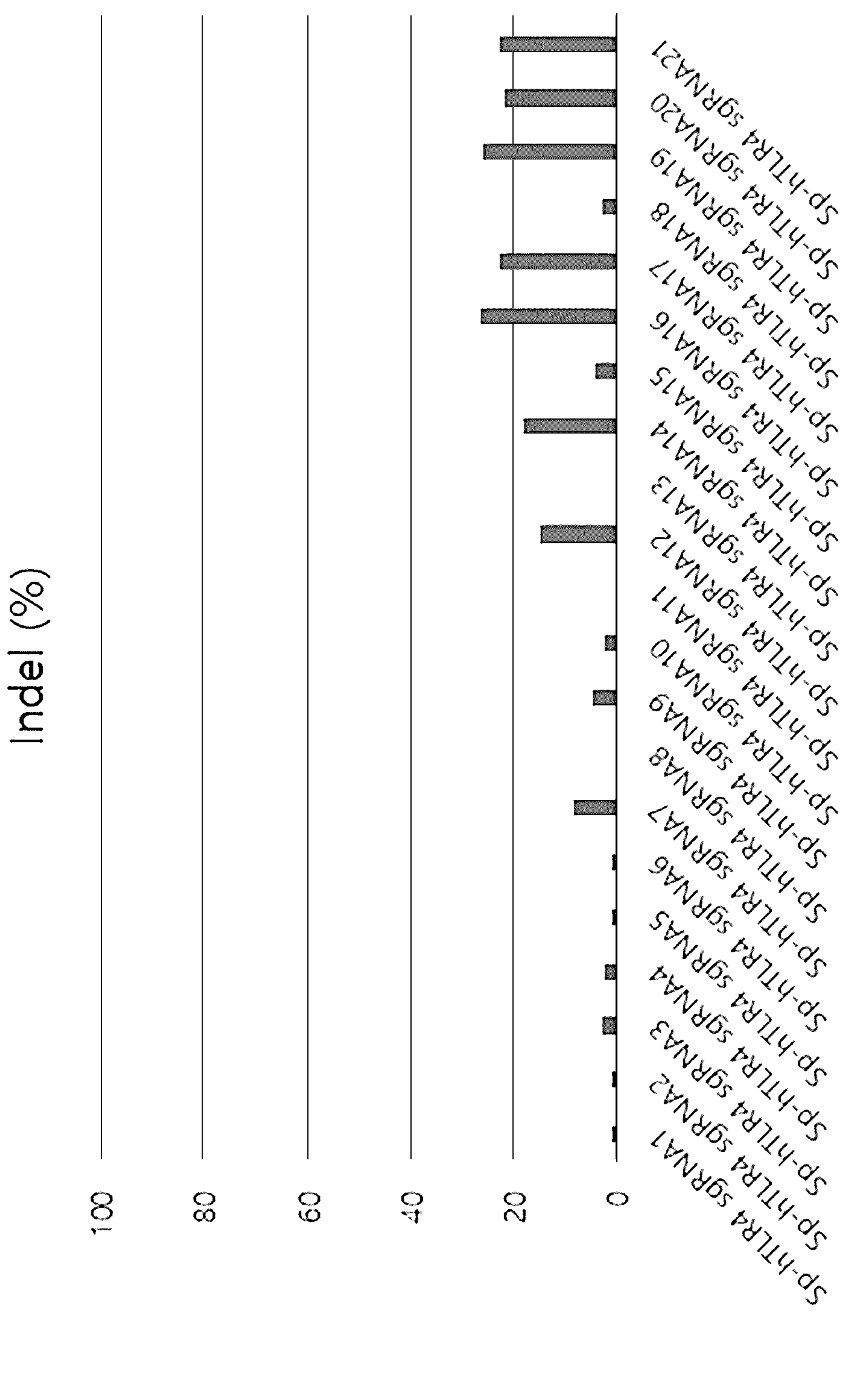
Indel (%)
100
80
60
40
20
0
Sp-hTLR4 sgRNA1
Sp-hTLR4 sgRNA2
Sp-hTLR4 sgRNA3
Sp-hTLR4 sgRNA4
Sp-hTLR4 sgRNA5
Sp-hTLR4 sgRNA6
Sp-hTLR4 sgRNA7
Sp-hTLR4 sgRNA8
Sp-hTLR4 sgRNA9
Sp-hTLR4 sgRNA10
Sp-hTLR4 sgRNA11
Sp-hTLR4 sgRNA12
Sp-hTLR4 sgRNA13
Sp-hTLR4 sgRNA14
Sp-hTLR4 sgRNA15
Sp-hTLR4 sgRNA16
Sp-hTLR4 sgRNA17
Sp-hTLR4 sgRNA18
Sp-hTLR4 sgRNA19
Sp-hTLR4 sgRNA20
Sp-hTLR4 sgRNA21

FIG. 3

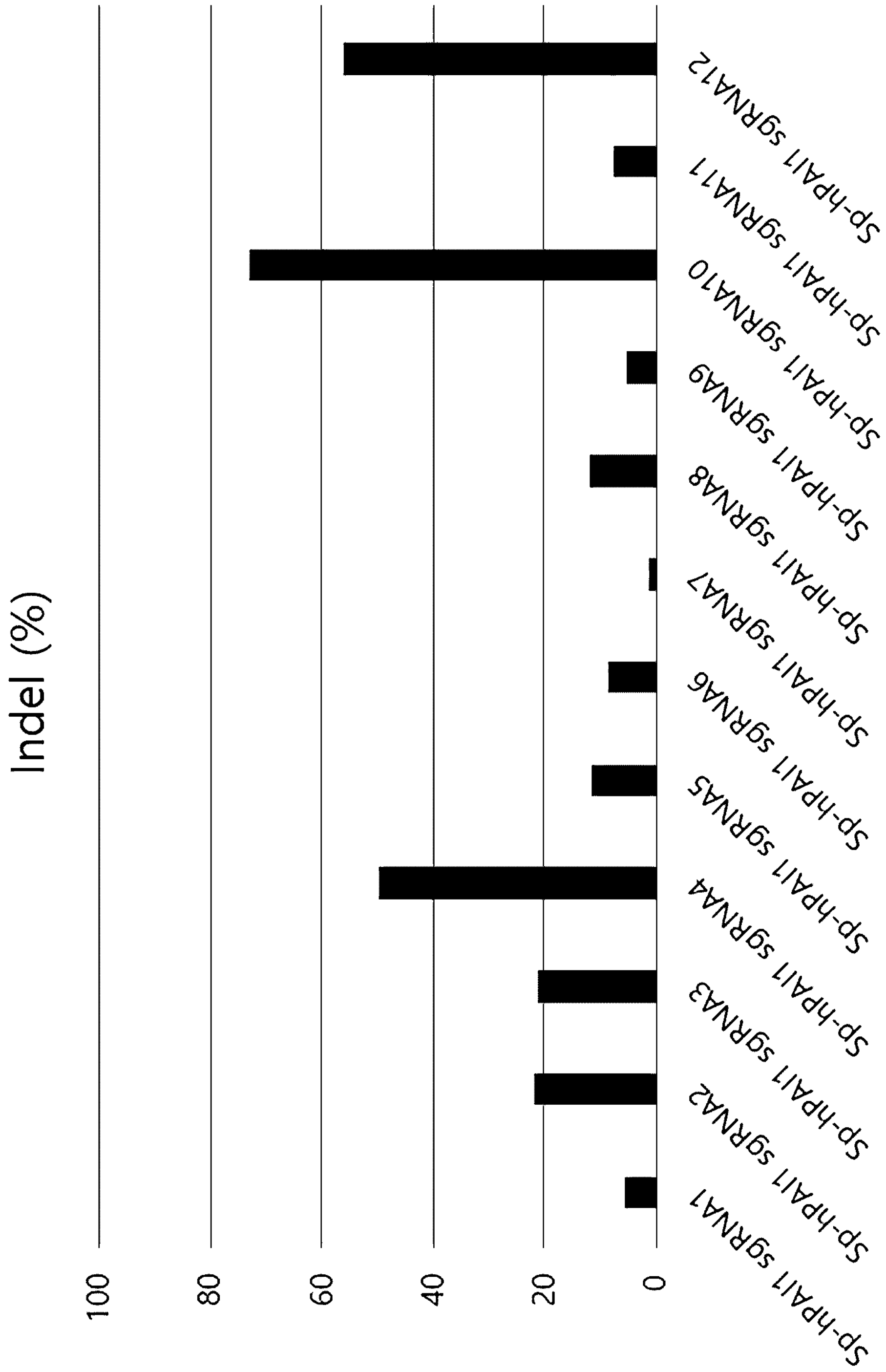
Indel (%)
0
20
40
60
80
100
Sp-hPAI1 sgRNA1
Sp-hPAI1 sgRNA2
Sp-hPAI1 sgRNA3
Sp-hPAI1 sgRNA4
Sp-hPAI1 sgRNA5
Sp-hPAI1 sgRNA6
Sp-hPAI1 sgRNA7
Sp-hPAI1 sgRNA8
Sp-hPAI1 sgRNA9
Sp-hPAI1 sgRNA10
Sp-hPAI1 sgRNA11
Sp-hPAI1 sgRNA12

FIG. 4

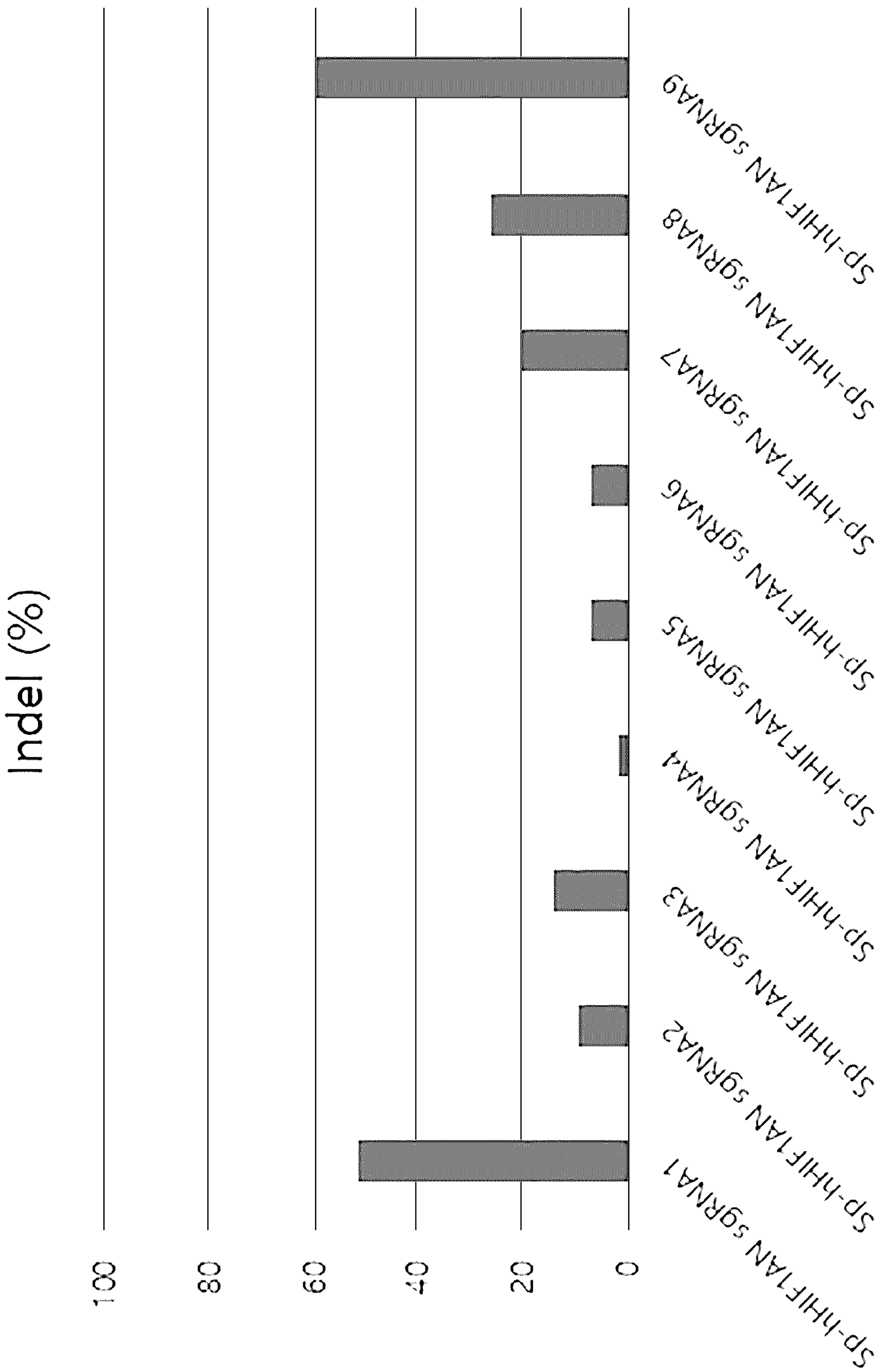
Indel (%)
100
80
60
40
20
0
Sp-hHIF1AN sgRNA1
Sp-hHIF1AN sgRNA2
Sp-hHIF1AN sgRNA3
Sp-hHIF1AN sgRNA4
Sp-hHIF1AN sgRNA5
Sp-hHIF1AN sgRNA6
Sp-hHIF1AN sgRNA7
Sp-hHIF1AN sgRNA8
Sp-hHIF1AN sgRNA9

FIG. 5

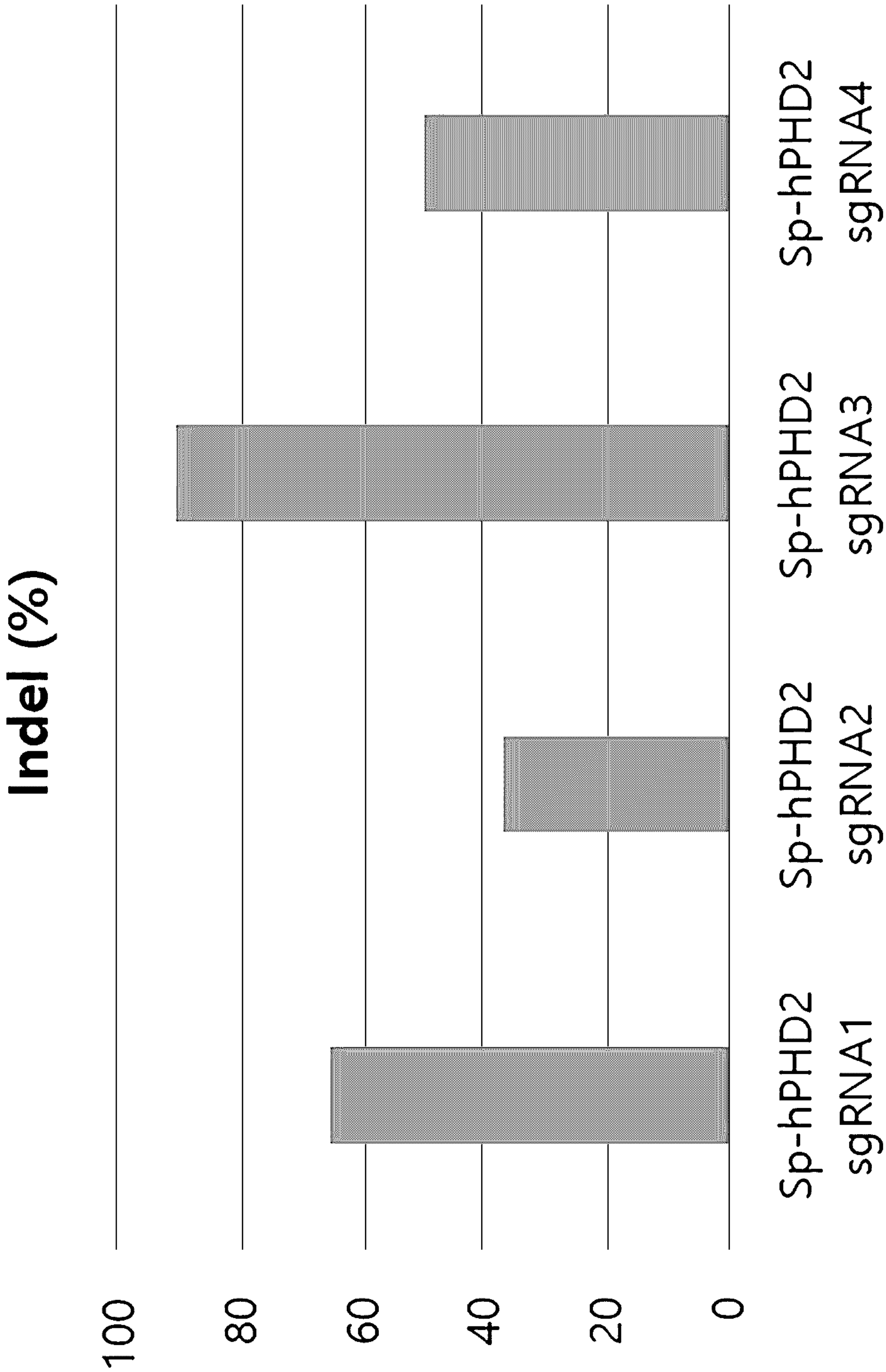
Indel (%)
100
80
60
40
20
0
Sp-hPHD2 sgRNA1
Sp-hPHD2 sgRNA2
Sp-hPHD2 sgRNA3
Sp-hPHD2 sgRNA4

FIG. 6

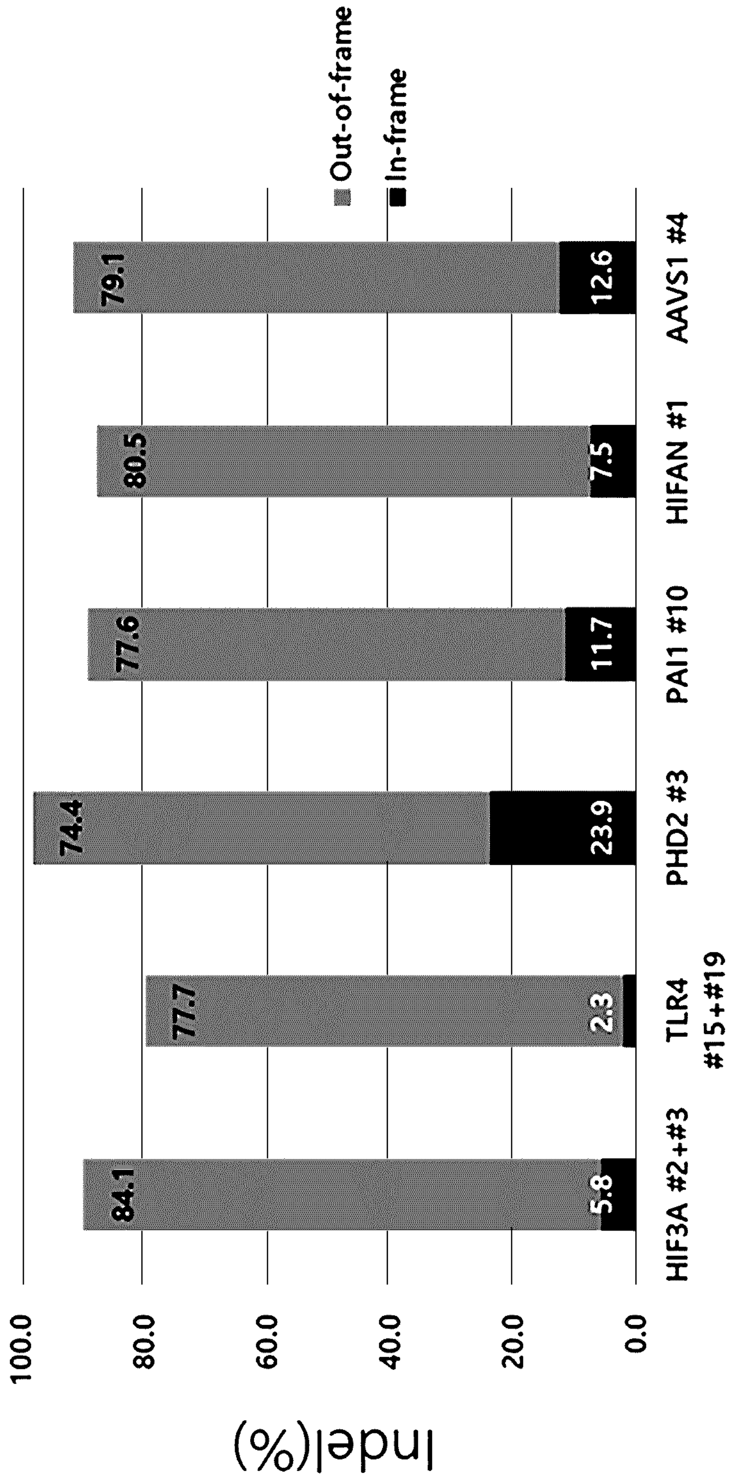
Indel(%)
100.0
80.0
60.0
40.0
20.0
0.0
Out-of-frame
In-frame
84.1
5.8
HIF3A #2+#3
77.7
2.3
TLR4 #15+#19
74.4
23.9
PHD2 #3
77.6
11.7
PAI1 #10
80.5
7.5
HIFAN #1
79.1
12.6
AAVS1 #4

FIG. 7

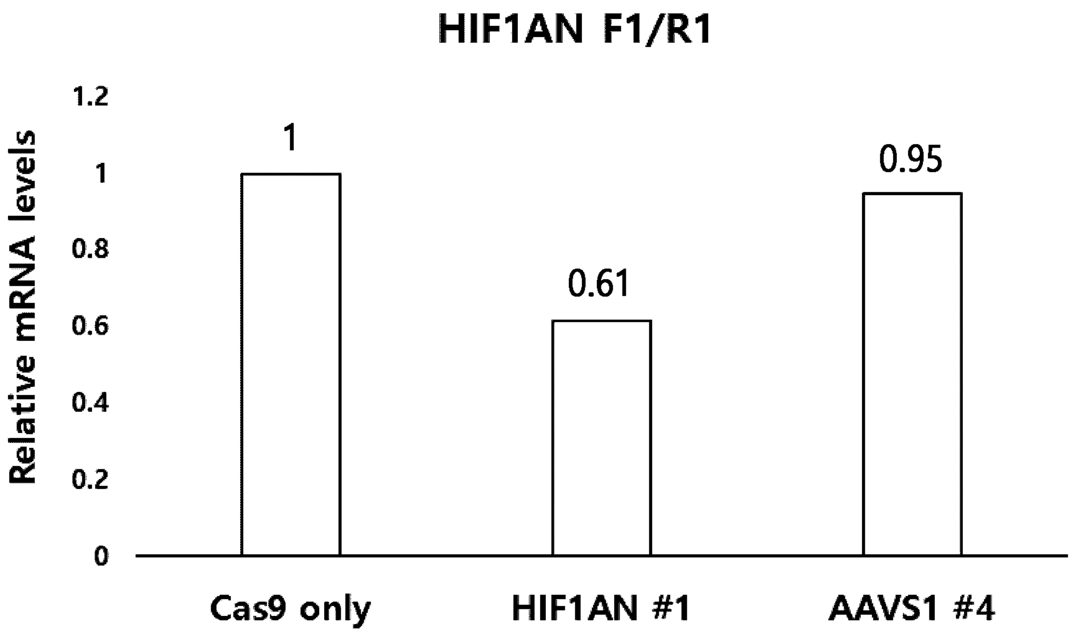
HIF1AN F1/R1
Relative mRNA levels
1.2
1
0.8
0.6
0.4
0.2
0
1
0.61
0.95
Cas9 only
HIF1AN #1
AAVS1 #4

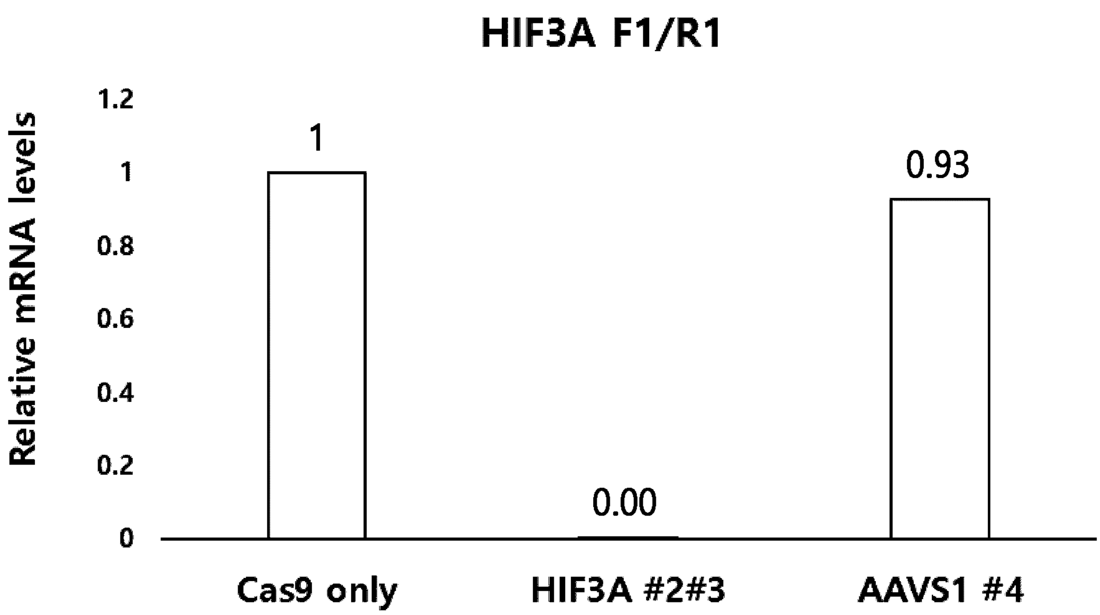
HIF3A F1/R1
Relative mRNA levels
1.2
1
0.8
0.6
0.4
0.2
0
1
0.00
0.93
Cas9 only
HIF3A #2#3
AAVS1 #4

FIG. 8

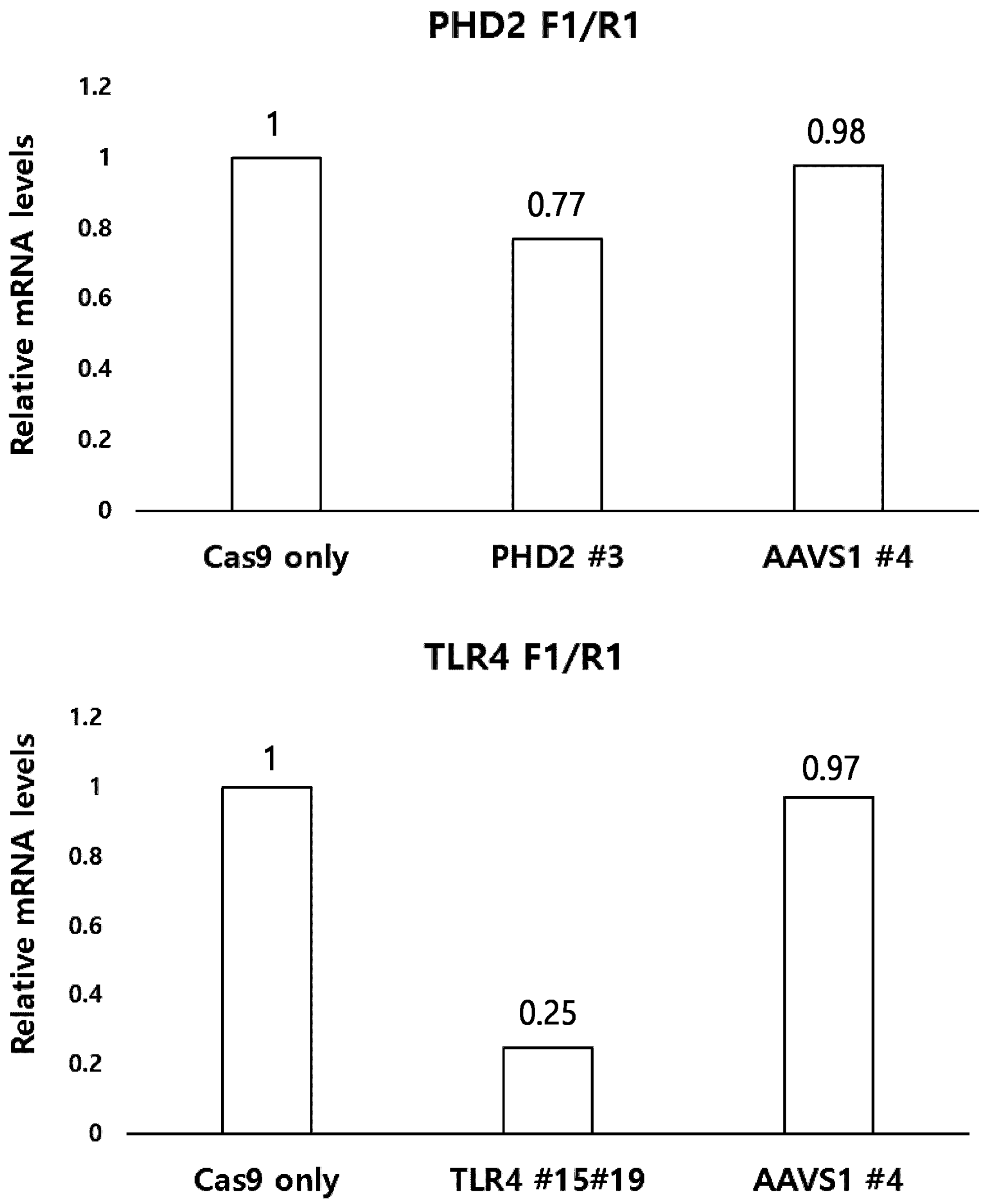
PHD2 F1/R1
Relative mRNA levels
1.2
1
0.8
0.6
0.4
0.2
0
1
0.77
0.98
Cas9 only
PHD2 #3
AAVS1 #4
TLR4 F1/R1
Relative mRNA levels
1.2
1
0.8
0.6
0.4
0.2
0
1
0.25
0.97
Cas9 only
TLR4 #15#19
AAVS1 #4

FIG. 9

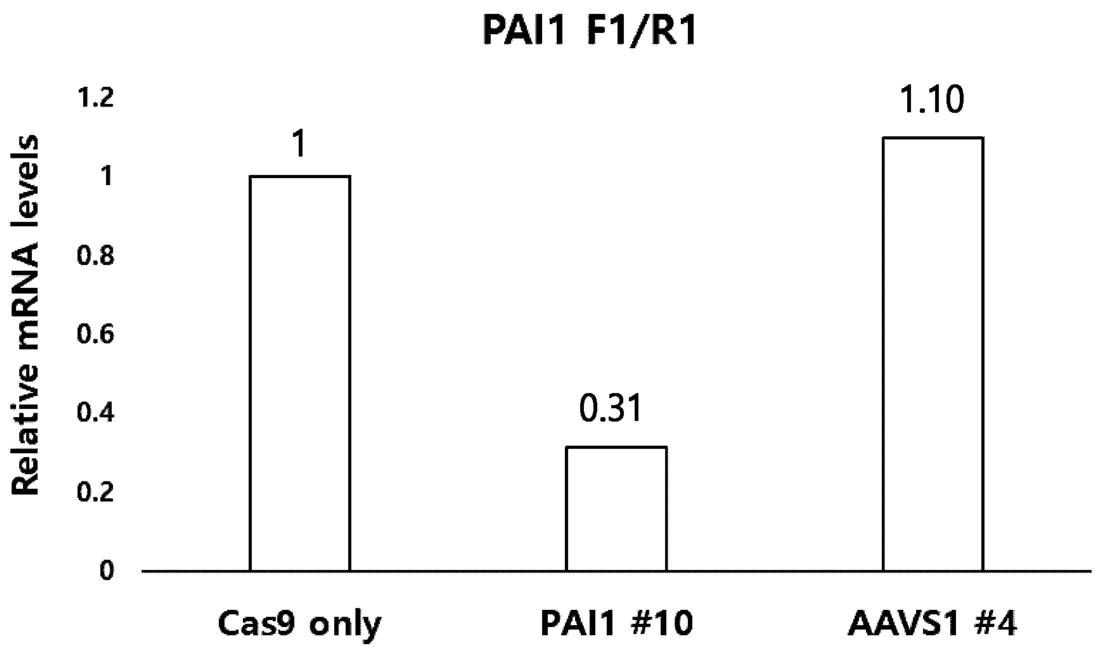
PAI1 F1/R1
Relative mRNA levels
1.2
1
0.8
0.6
0.4
0.2
0
1
0.31
1.10
Cas9 only
PAI1 #10
AAVS1 #4

FIG. 10

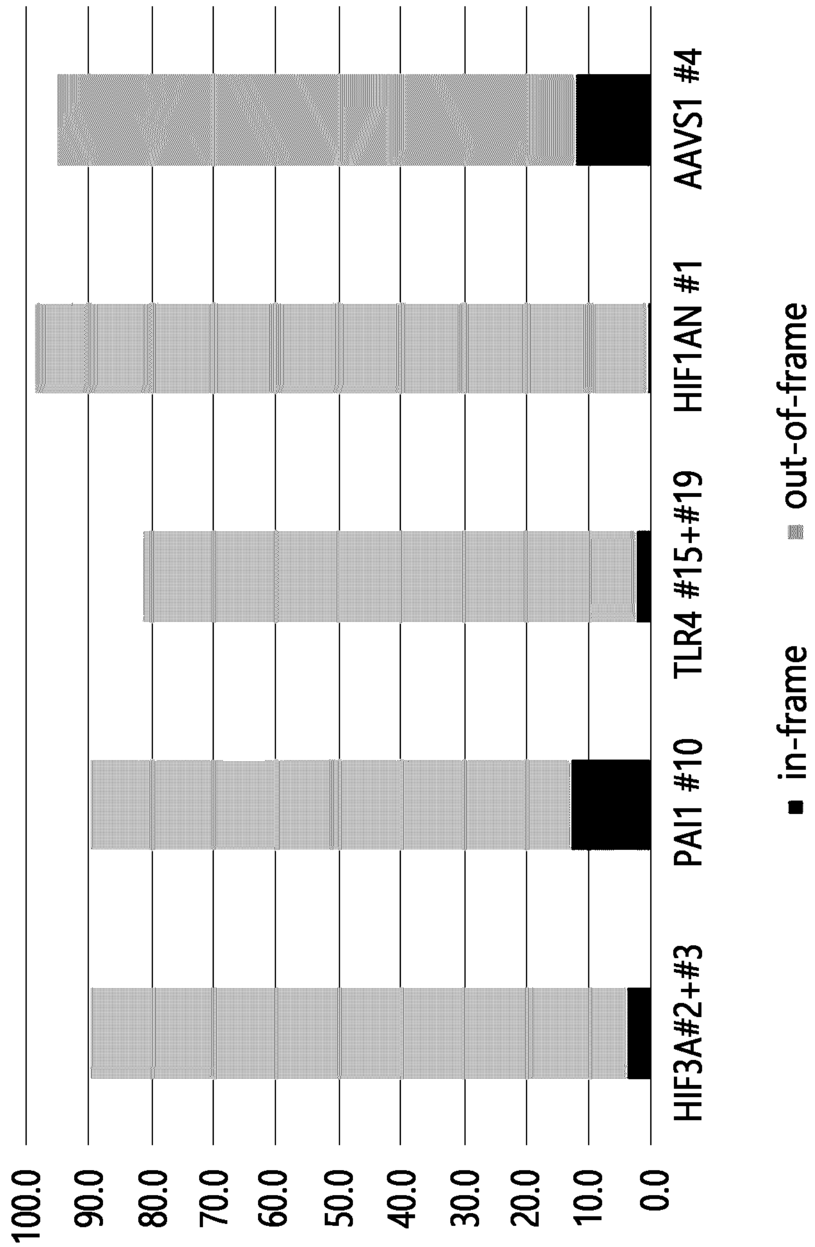
100.0
90.0
80.0
70.0
60.0
50.0
40.0
30.0
20.0
10.0
0.0
HIF3A#2+#3
PAI1 #10
TLR4 #15+#19
HIF1AN #1
AAVS1 #4
in-frame
out-of-frame

FIG. 11

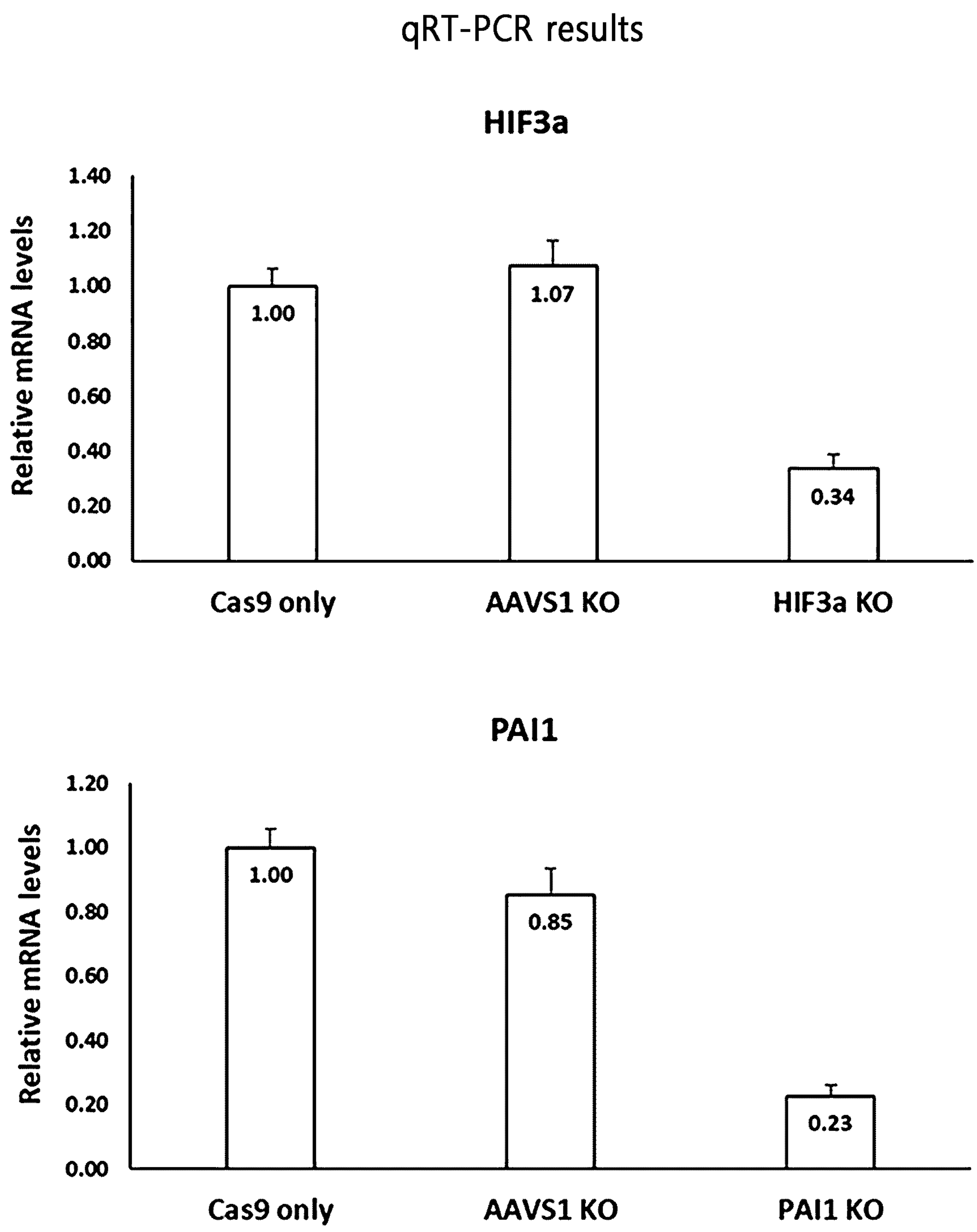
qRT-PCR results
HIF3a
Relative mRNA levels
1.40
1.20
1.00
0.80
0.60
0.40
0.20
0.00
1.00
1.07
0.34
Cas9 only
AAVS1 KO
HIF3a KO
PAI1
Relative mRNA levels
1.20
1.00
0.80
0.60
0.40
0.20
0.00
1.00
0.85
0.23
Cas9 only
AAVS1 KO
PAI1 KO

FIG. 12

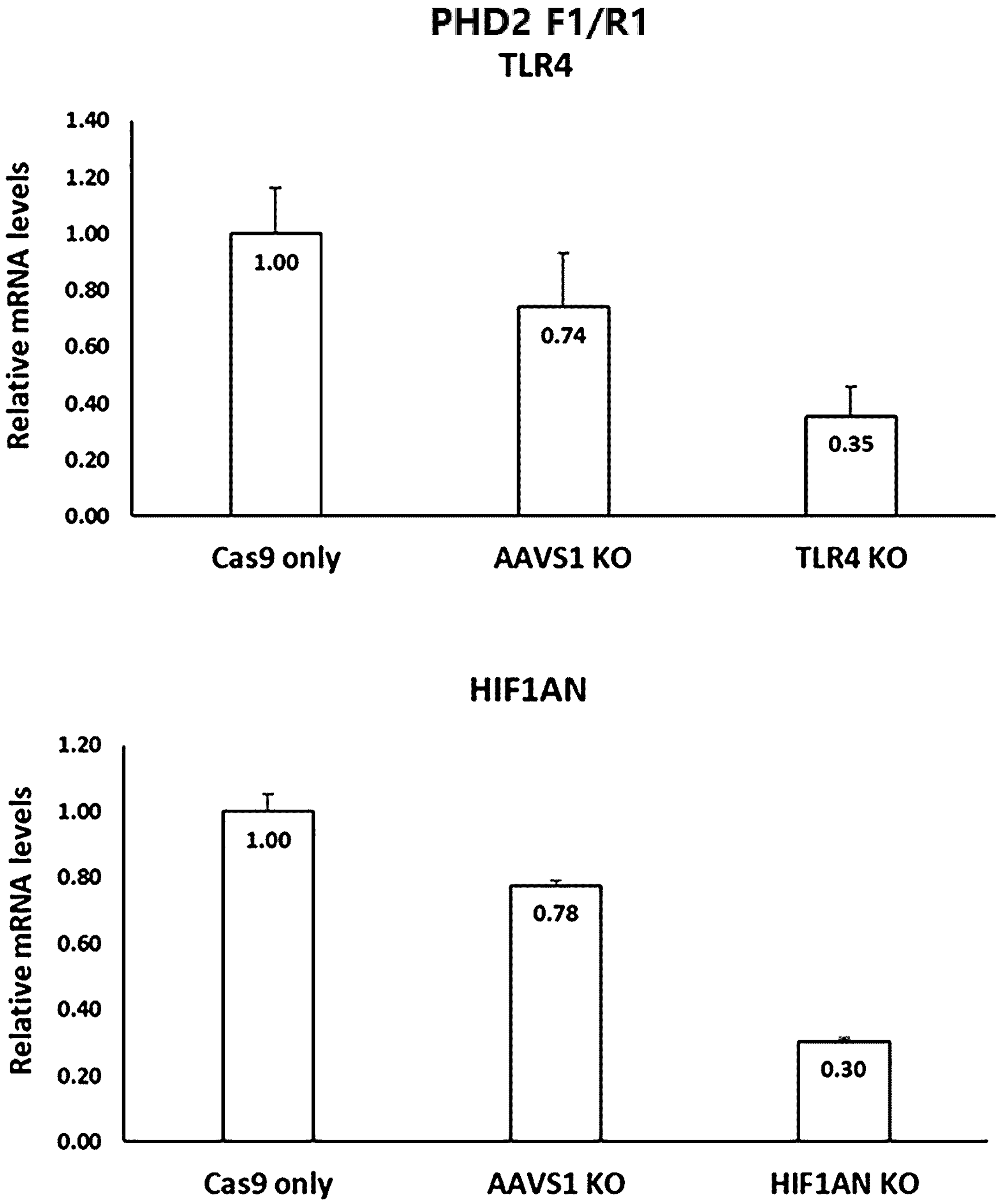
PHD2 F1/R1
TLR4
Relative mRNA levels
1.40
1.20
1.00
0.80
0.60
0.40
0.20
0.00
1.00
0.74
0.35
Cas9 only
AAVS1 KO
TLR4 KO
HIF1AN
Relative mRNA levels
1.20
1.00
0.80
0.60
0.40
0.20
0.00
1.00
0.78
0.30
Cas9 only
AAVS1 KO
HIF1AN KO

FIG. 13

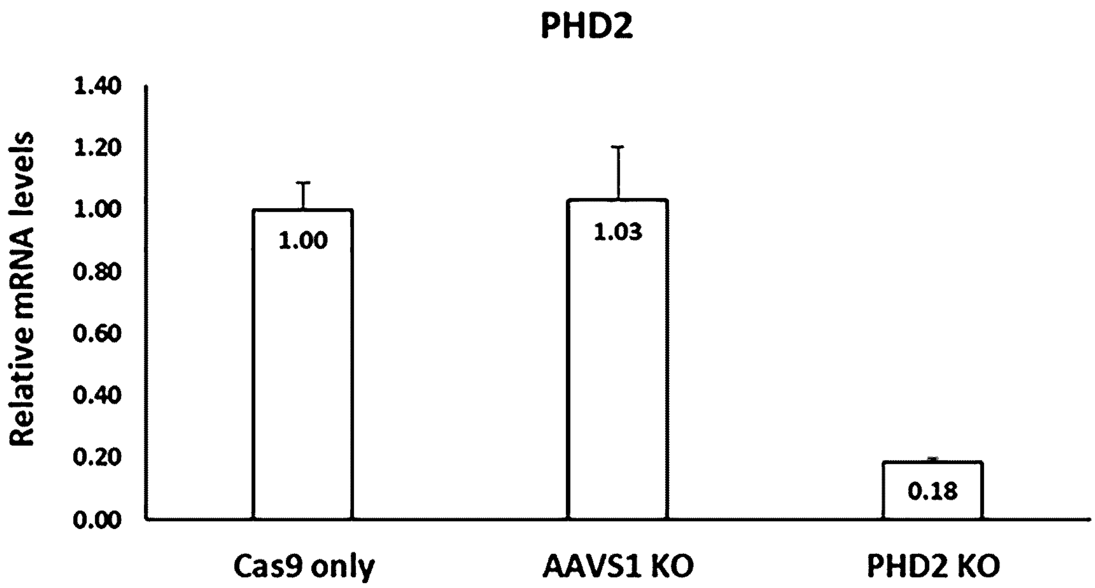
PHD2
Relative mRNA levels
1.40
1.20
1.00
0.80
0.60
0.40
0.20
0.00
1.00
1.03
0.18
Cas9 only
AAVS1 KO
PHD2 KO

FIG. 14

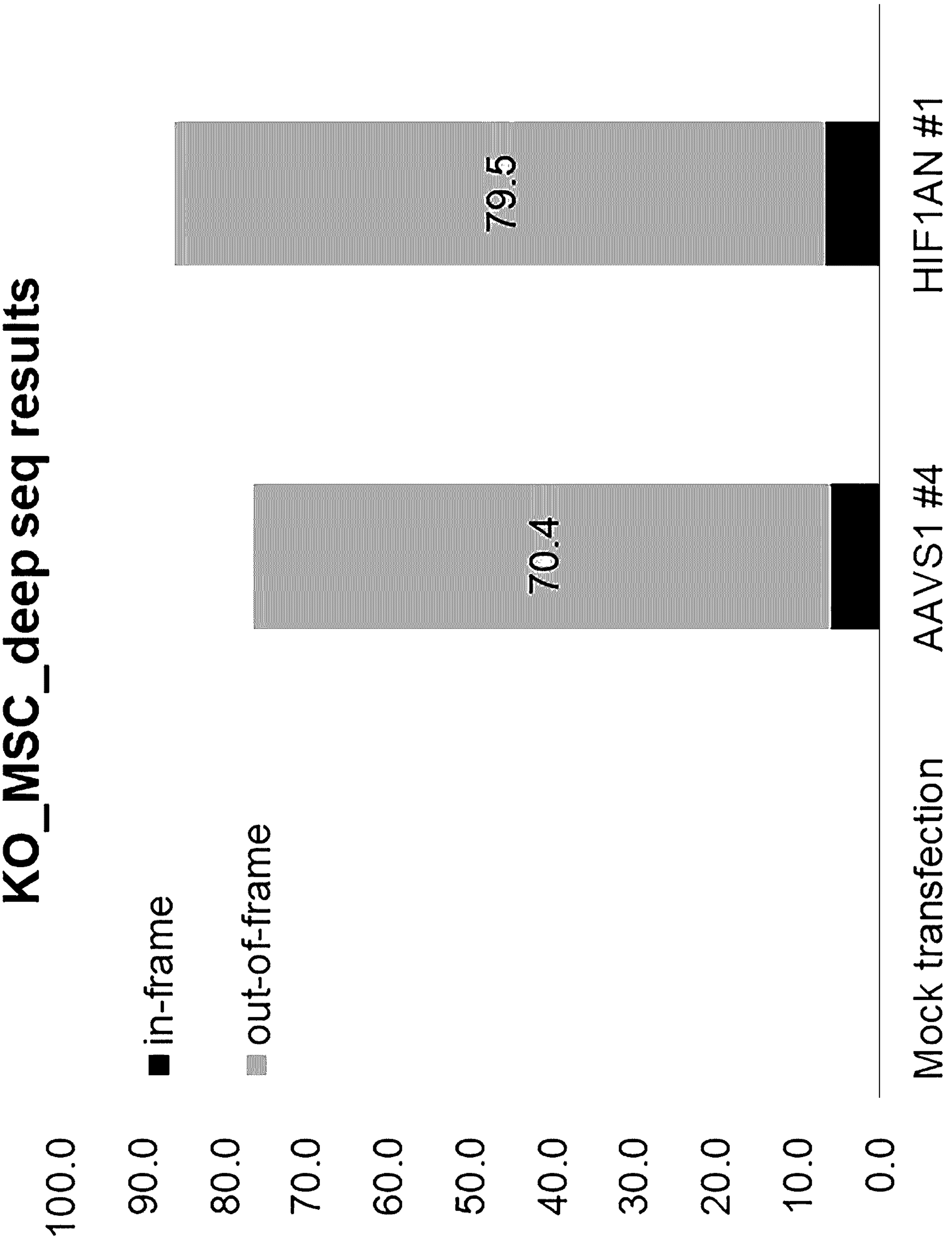
KO_MSC_deep seq results
in-frame
out-of-frame
100.0
90.0
80.0
70.0
60.0
50.0
40.0
30.0
20.0
10.0
0.0
70.4
79.5
Mock transfection
AAVS1 #4
HIF1AN #1

FIG. 15

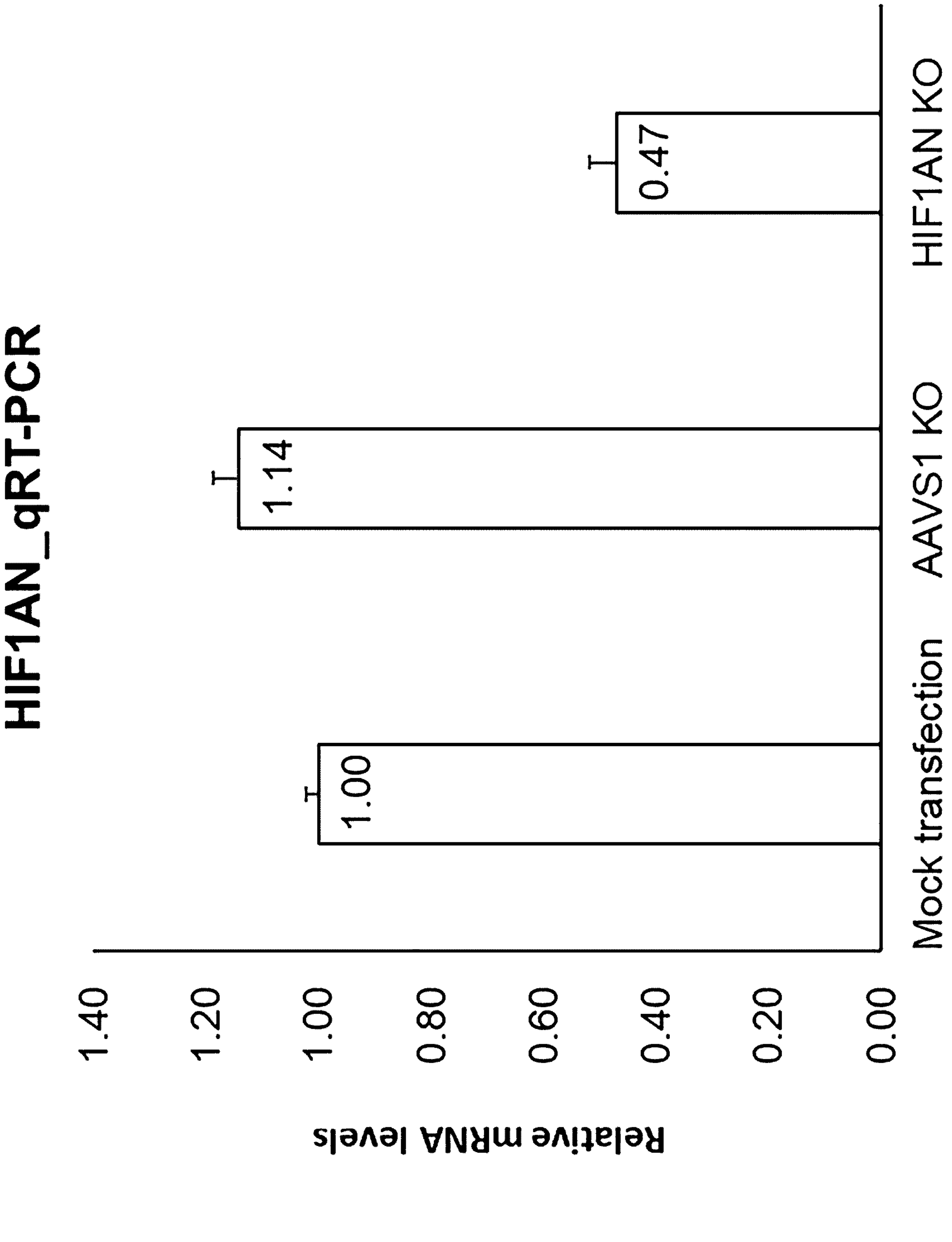
HIF1AN_qRT-PCR
Relative mRNA levels
1.40
1.20
1.00
0.80
0.60
0.40
0.20
0.00
1.00
1.14
0.47
Mock transfection
AAVS1 KO
HIF1AN KO

FIG. 16

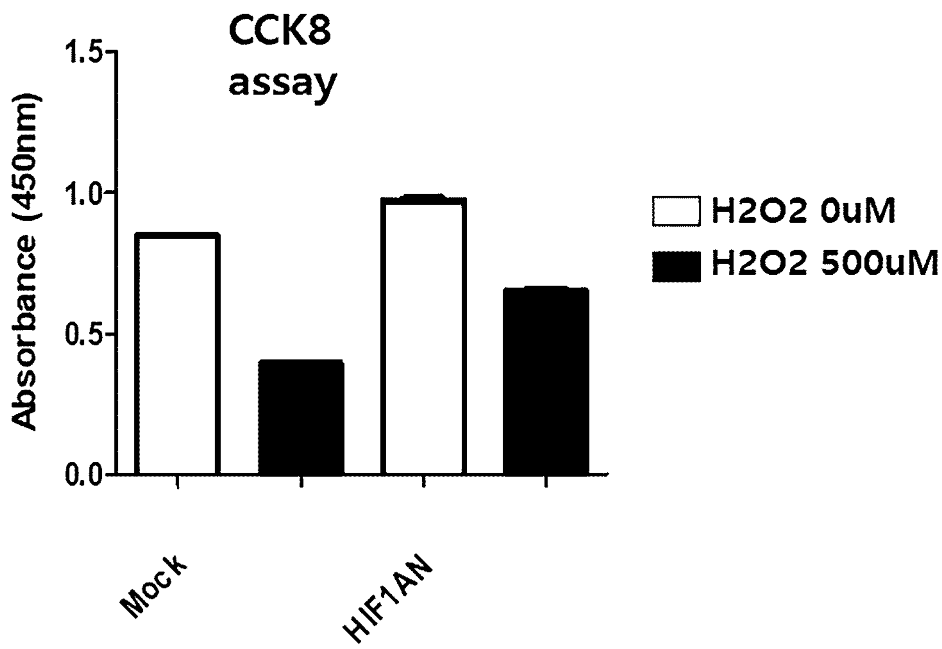
CCK8
assay
Absorbance (450nm)
1.5
1.0
0.5
0.0
Mock
HIF1AN
H2O2 0uM
H2O2 500uM

FIG. 17

| | Mock | | HIF1AN | |
|---|---|---|---|---|
| H2O2 | 0uM | 500uM | 0uM | 500uM |
| | 0.8466 | 0.3853 | 1.0007 | 0.6214 |
| | 0.8333 | 0.3887 | 0.9577 | 0.6598 |
| | 0.8522 | 0.4026 | 0.9779 | 0.6535 |
| | 0.8588 | 0.4007 | 0.9603 | 0.6516 |
| | 0.8549 | 0.3916 | 0.9751 | 0.6649 |
| average | 0.8492 | 0.3938 | 0.9743 | 0.6502 |
| | 1.30592 | 0.60559 | 1.49843 | 1 |

FIG. 18

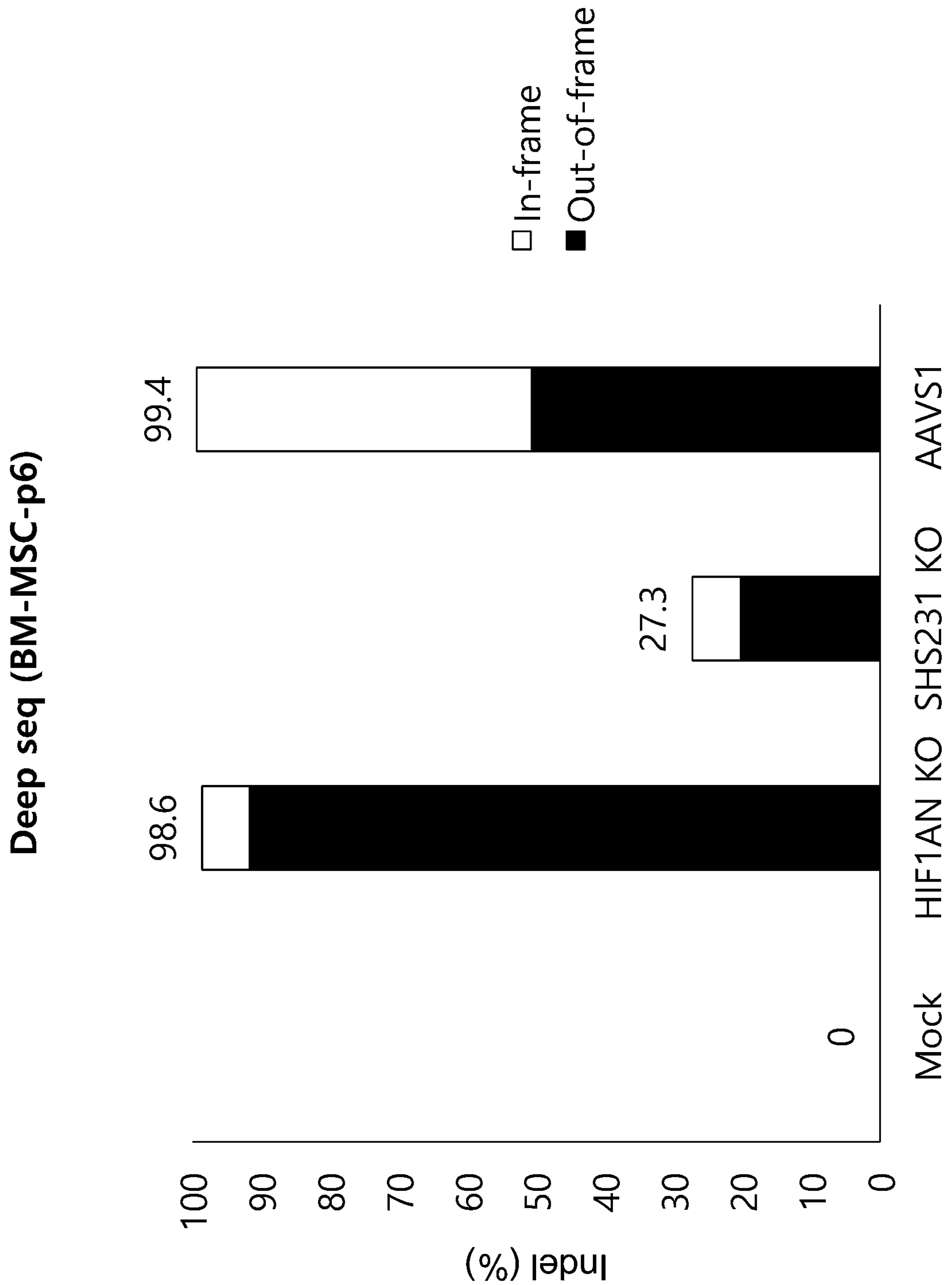
Deep seq (BM-MSC-p6)
□ In-frame
■ Out-of-frame
Indel (%)
100
90
80
70
60
50
40
30
20
10
0
0
98.6
27.3
99.4
Mock
HIF1AN KO
SHS231 KO
AAVS1

FIG. 19

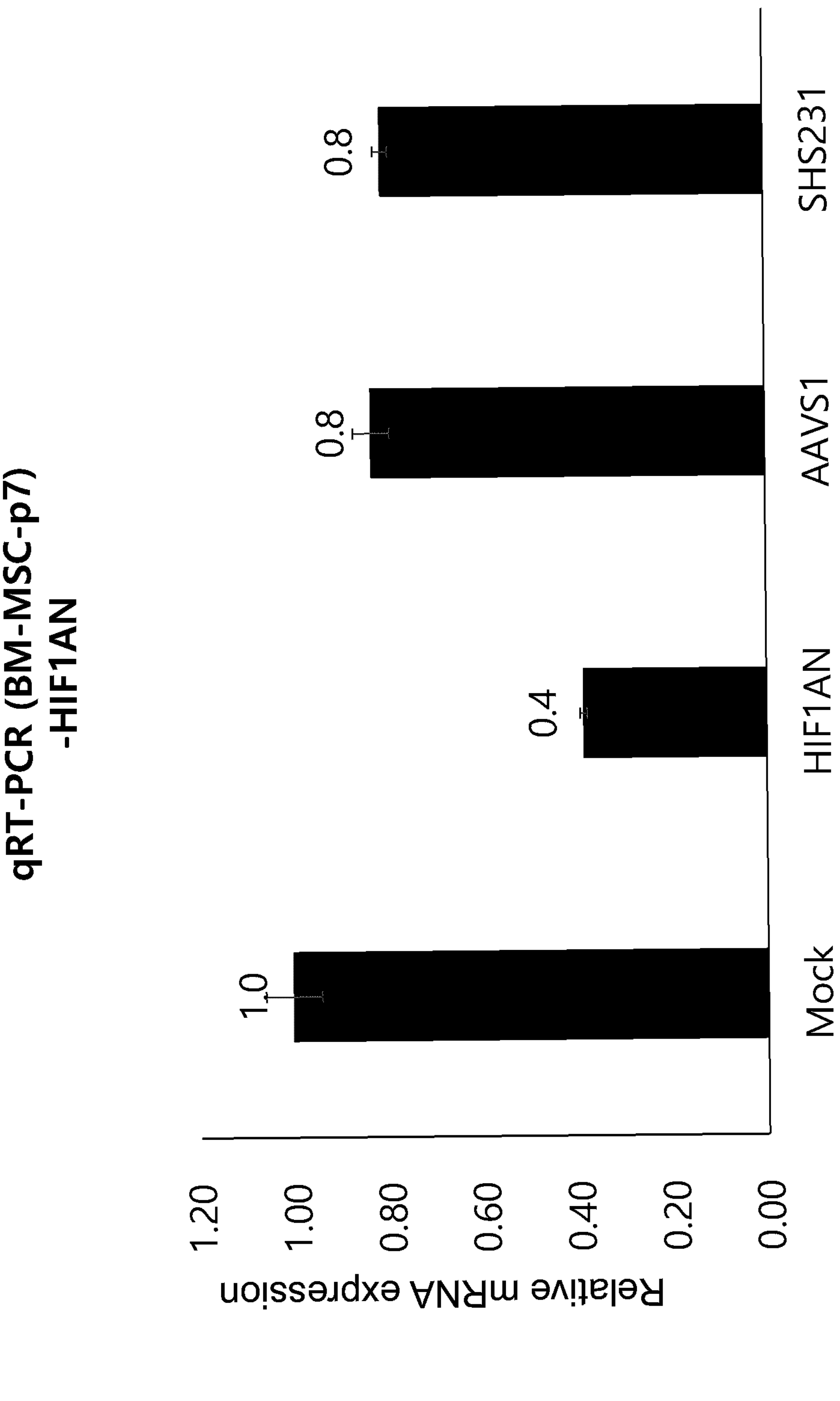
qRT-PCR (BM-MSC-p7)
-HIF1AN
Relative mRNA expression
0.00
0.20
0.40
0.60
0.80
1.00
1.20
1.0
0.4
0.8
0.8
Mock
HIF1AN
AAVS1
SHS231

FIG. 20

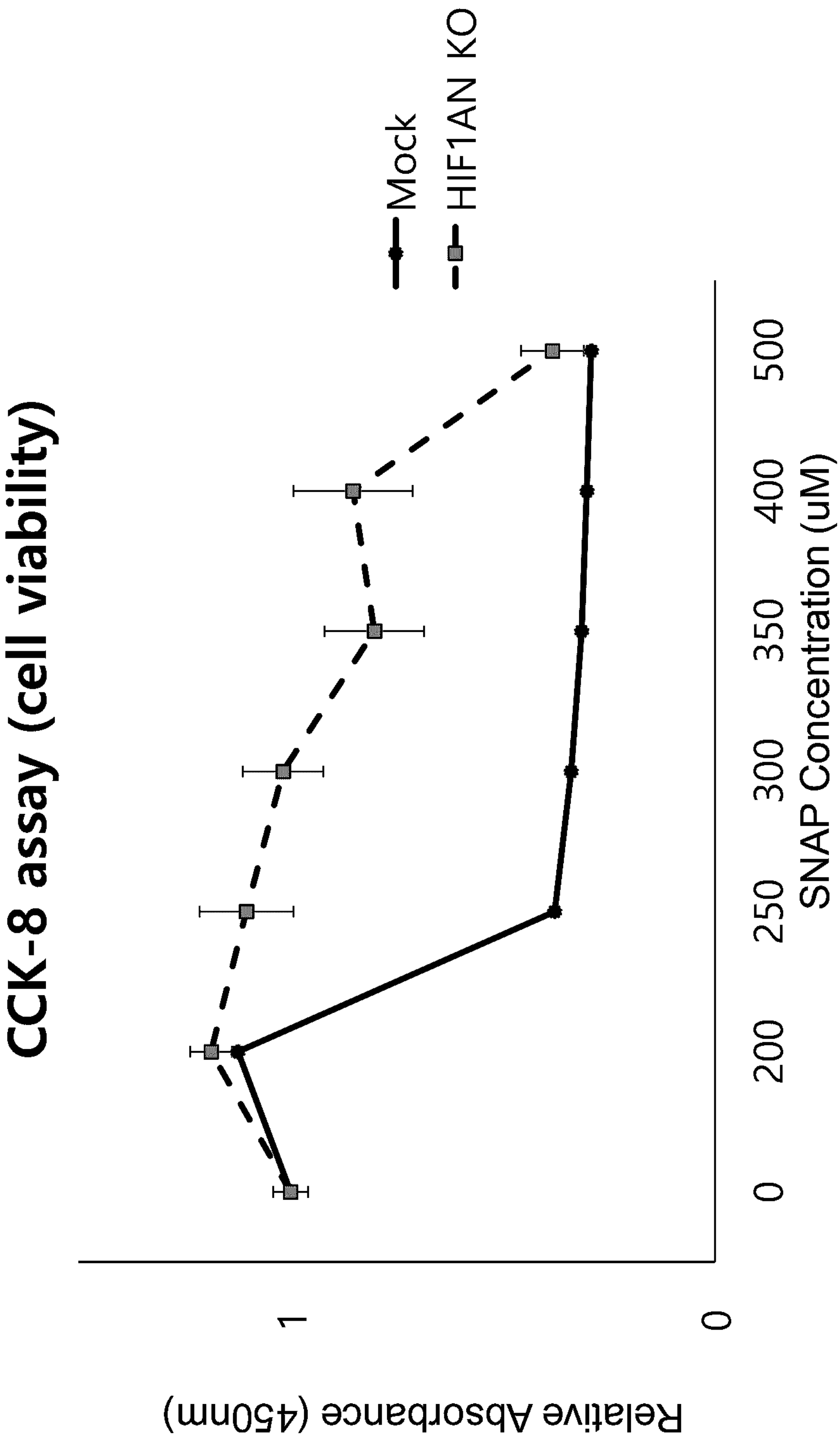
CCK-8 assay (cell viability)
Mock
HIF1AN KO
Relative Absorbance (450nm)
0
1
SNAP Concentration (uM)
0
200
250
300
350
400
500

FIG. 21

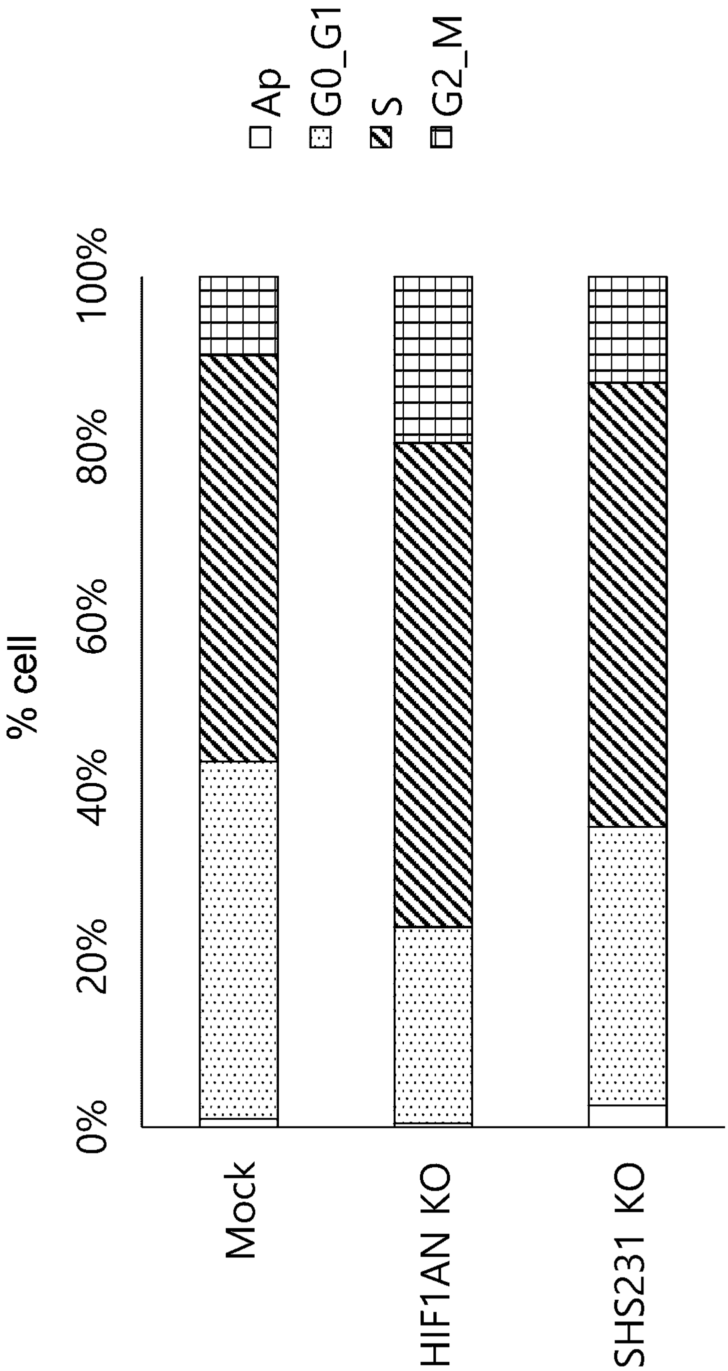

FIG. 22

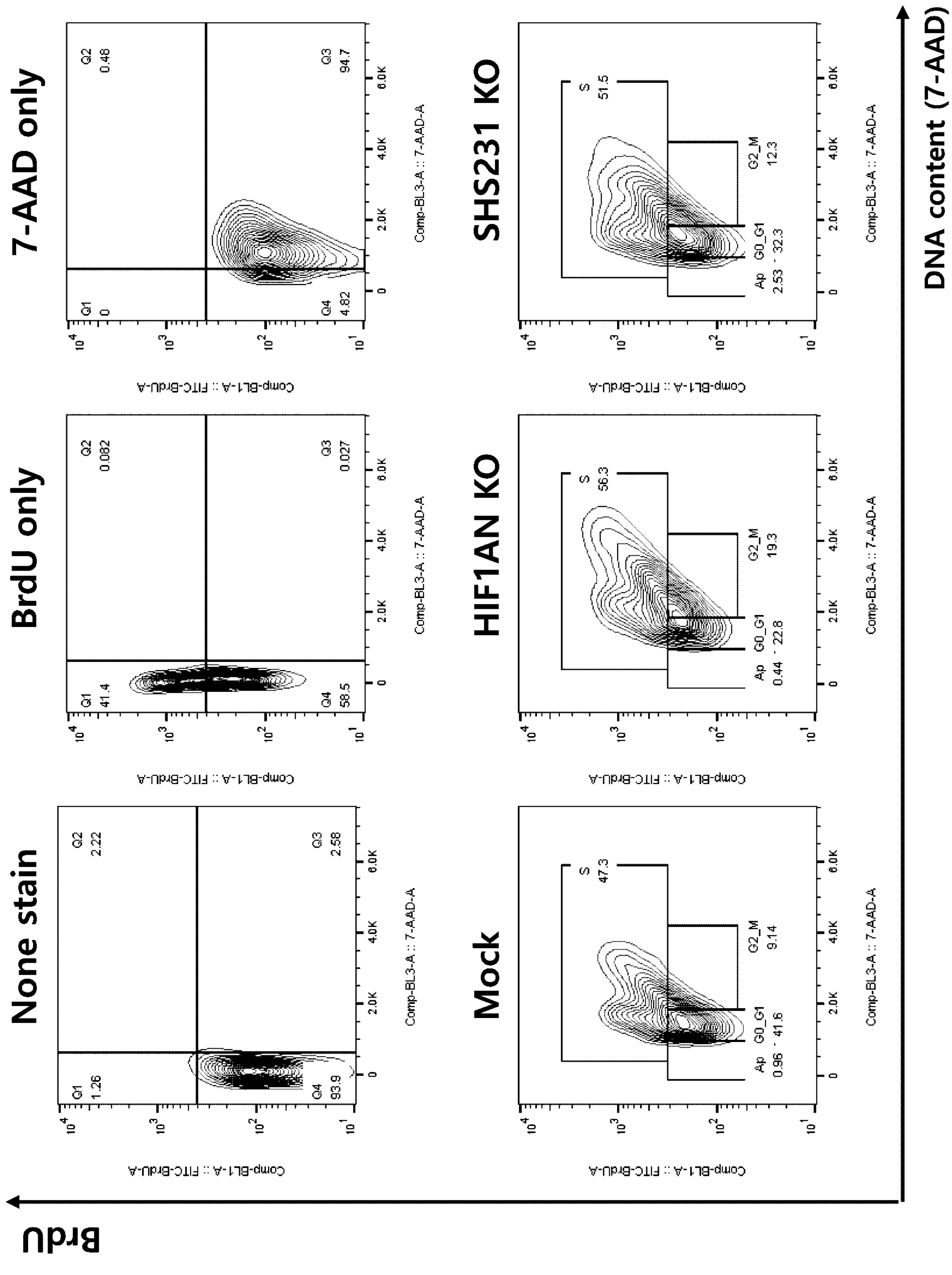
None stain
BrdU only
7-AAD only
Mock
HIF1AN KO
SHS231 KO
BrdU
DNA content (7-AAD)
Comp-BL1-A :: FITC-BrdU-A
Comp-BL3-A :: 7-AAD-A
Q1 1.26
Q2 2.22
Q3 2.58
Q4 93.9
Q1 41.4
Q2 0.082
Q3 0.027
Q4 58.5
Q1 0
Q2 0.48
Q3 94.7
Q4 4.82
S 47.3
G2_M 9.14
Ap 0.96
G0_G1 41.6
S 56.3
G2_M 19.3
Ap 0.44
G0_G1 22.8
S 51.5
G2_M 12.3
Ap 2.53
G0_G1 32.3

FIG. 23

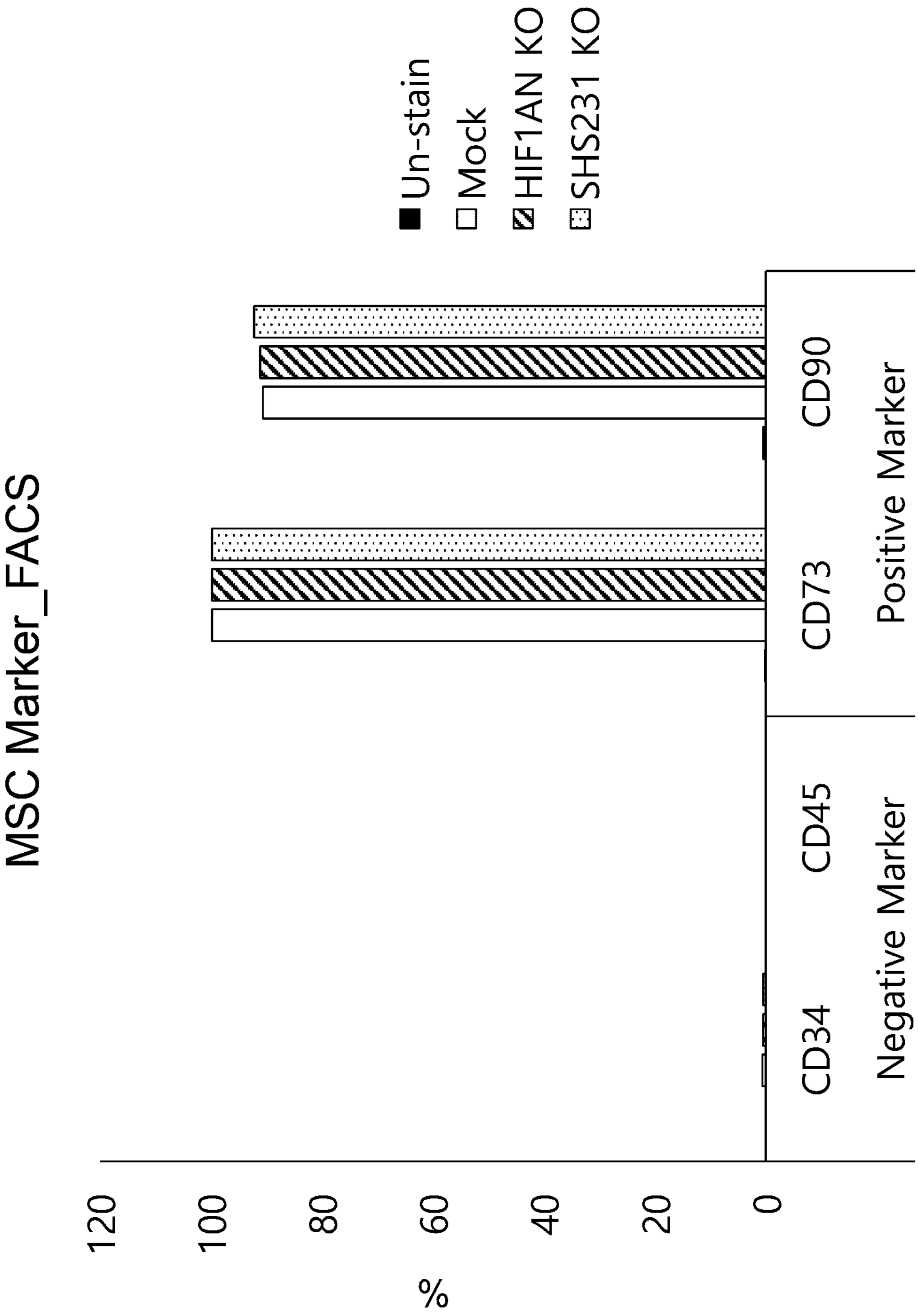
MSC Marker_FACS
Un-stain
Mock
HIF1AN KO
SHS231 KO
%
120
100
80
60
40
20
0
CD34
CD45
Negative Marker
CD73
CD90
Positive Marker

FIG. 24

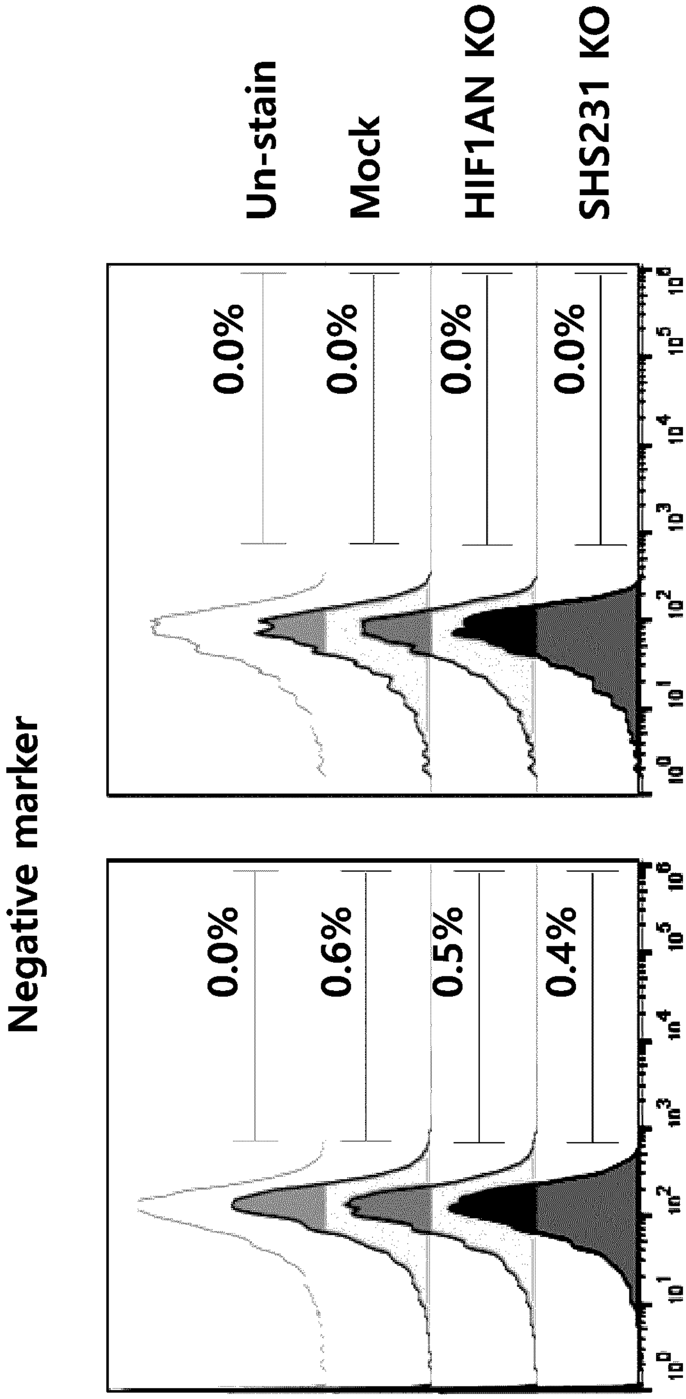
Negative marker
Un-stain
Mock
HIF1AN KO
SHS231 KO
0.0%
0.6%
0.5%
0.4%
0.0%
0.0%
0.0%
0.0%
PE-CD34
APC-CD45

FIG. 25

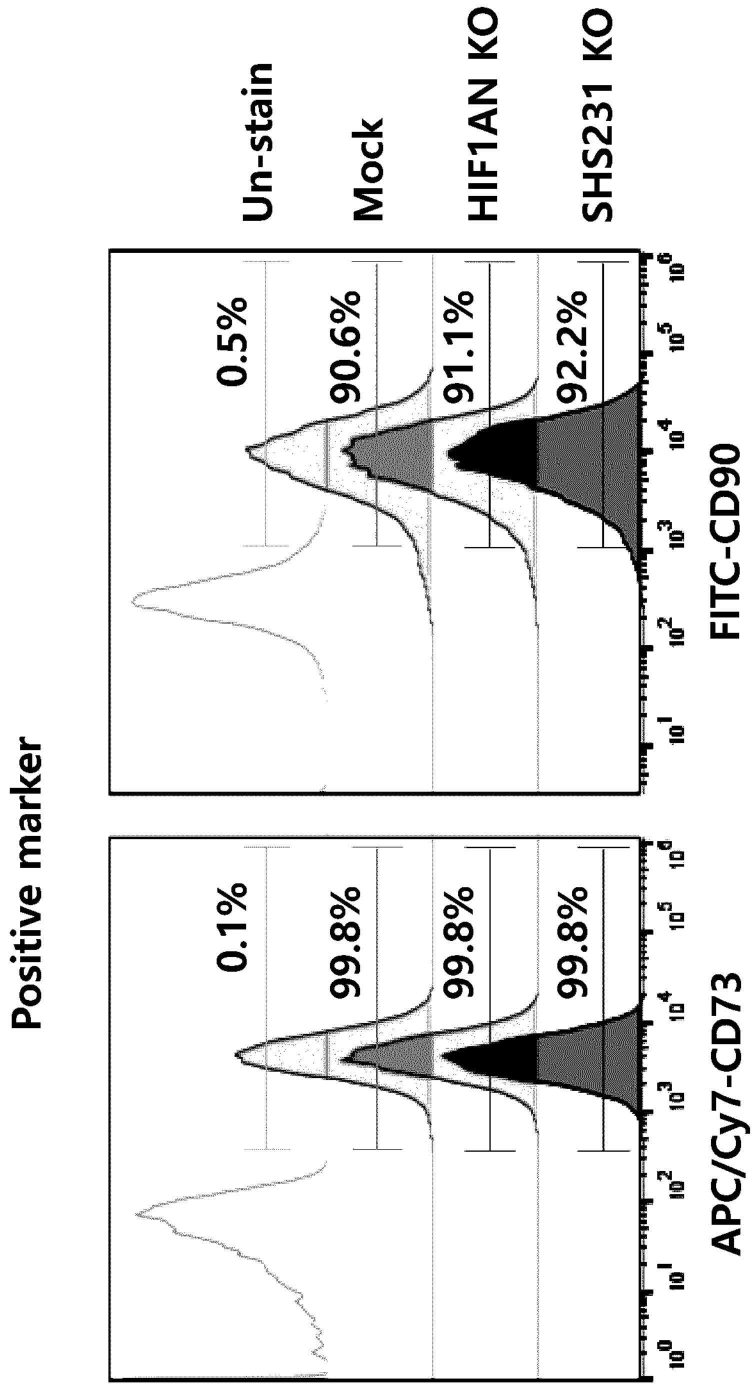
Positive marker
Un-stain
Mock
HIF1AN KO
SHS231 KO
0.5%
90.6%
91.1%
92.2%
FITC-CD90
0.1%
99.8%
99.8%
99.8%
APC/Cy7-CD73

FIG. 26

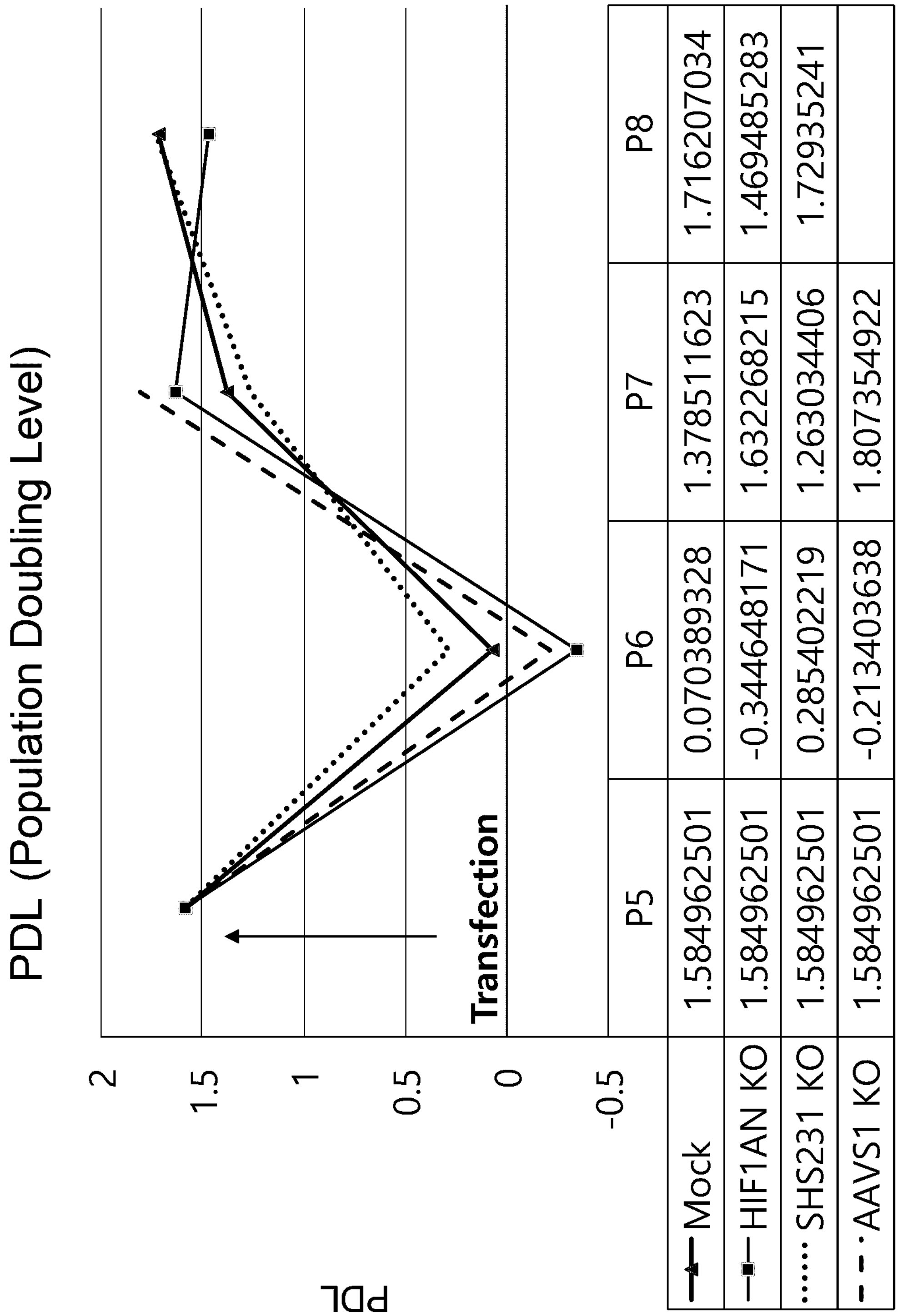


| PDL | P5 | P6 | P7 | P8 |
|---|---|---|---|---|
| Mock | 1.584962501 | 0.070389328 | 1.378511623 | 1.716207034 |
| HIF1AN KO | 1.584962501 | -0.344648171 | 1.632268215 | 1.469485283 |
| SHS231 KO | 1.584962501 | 0.285402219 | 1.263034406 | 1.72935241 |
| AAVS1 KO | 1.584962501 | -0.213403638 | 1.807354922 | |

FIG. 27

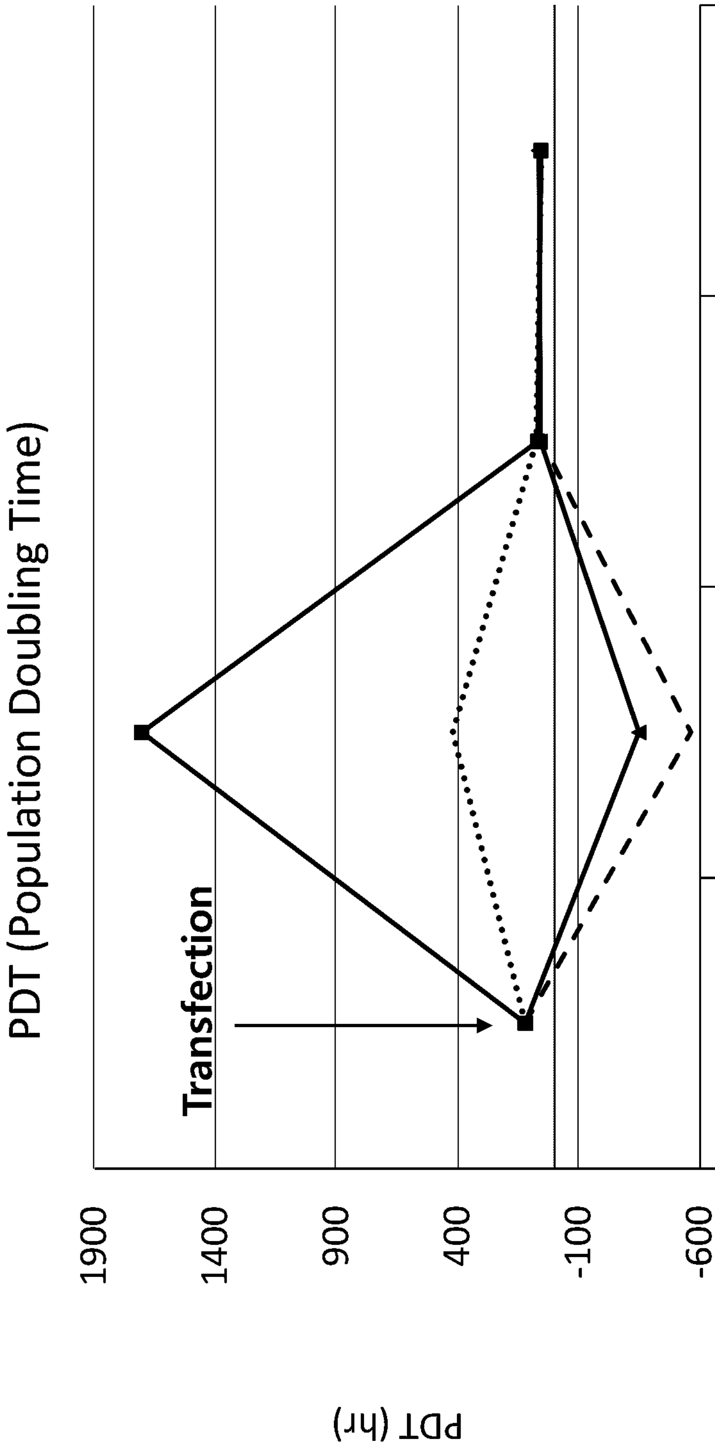


| | P5 | P6 | P7 | P8 |
|---|---|---|---|---|
| Mock | 121.1385127 | 1704.80389 | 69.64032684 | 55.93730715 |
| HIF1AN KO | 121.1385127 | -348.1811597 | 58.81386349 | 65.32899723 |
| SHS231 KO | 121.1385127 | 420.4592399 | 76.00743064 | 55.5121093 |
| AAVS1 KO | 121.1385127 | -562.3146871 | 53.11629654 | |

FIG. 28

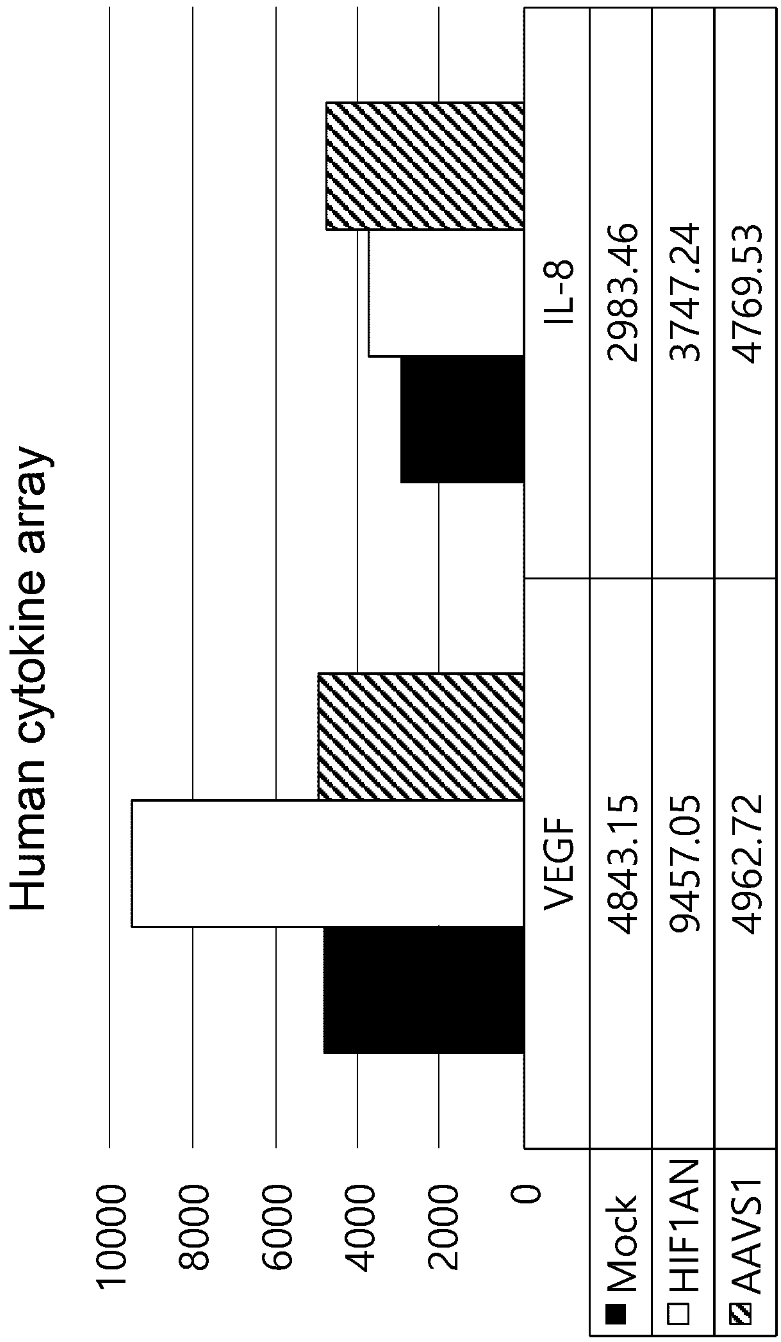
Human cytokine array
10000
8000
6000
4000
2000
0
VEGF
IL-8
Mock 4843.15 2983.46
HIF1AN 9457.05 3747.24
AAVS1 4962.72 4769.53

FIG. 29

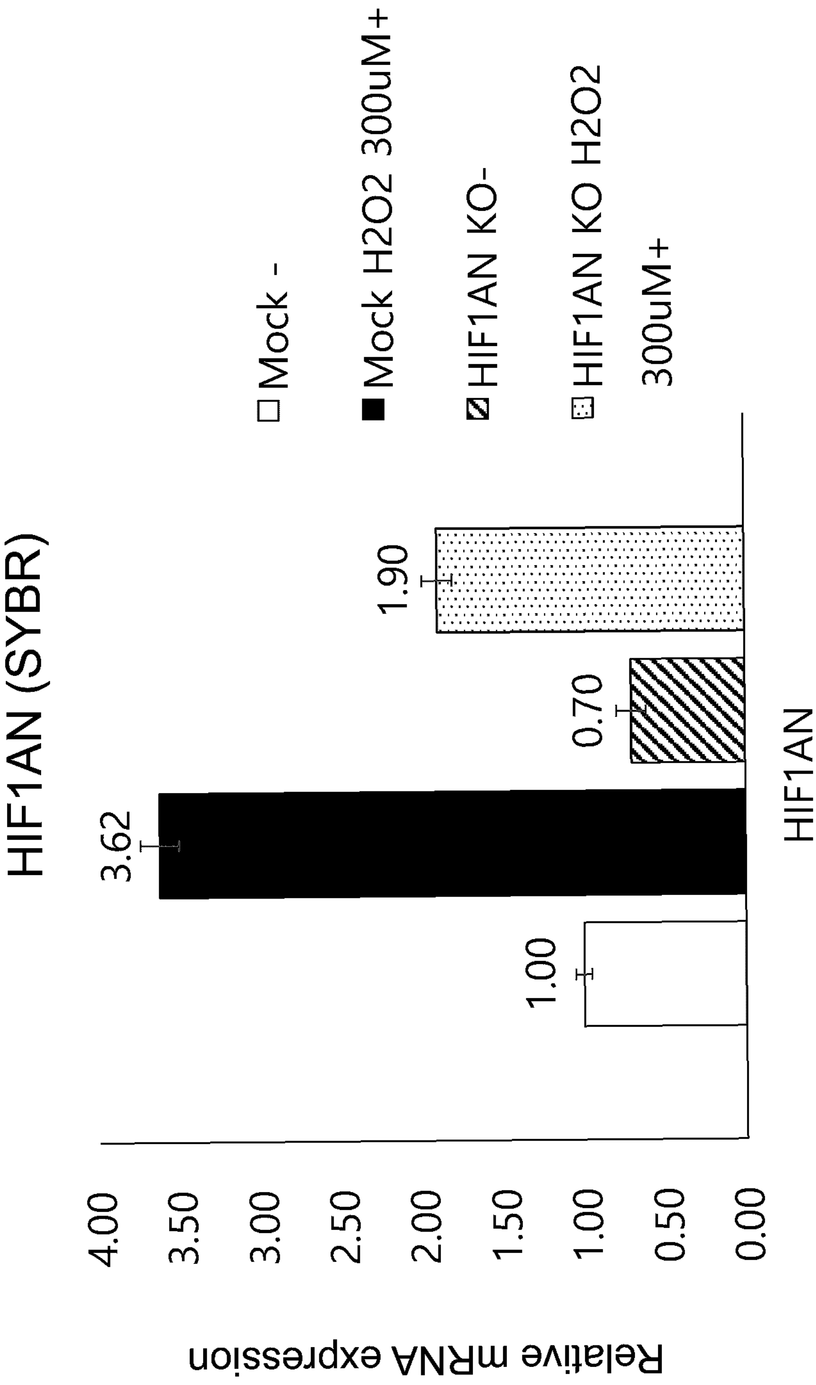
HIF1AN (SYBR)
□ Mock -
■ Mock H2O2 300uM+
HIF1AN KO-
HIF1AN KO H2O2 300uM+
1.00
3.62
0.70
1.90
HIF1AN
Relative mRNA expression
4.00
3.50
3.00
2.50
2.00
1.50
1.00
0.50
0.00

FIG. 30

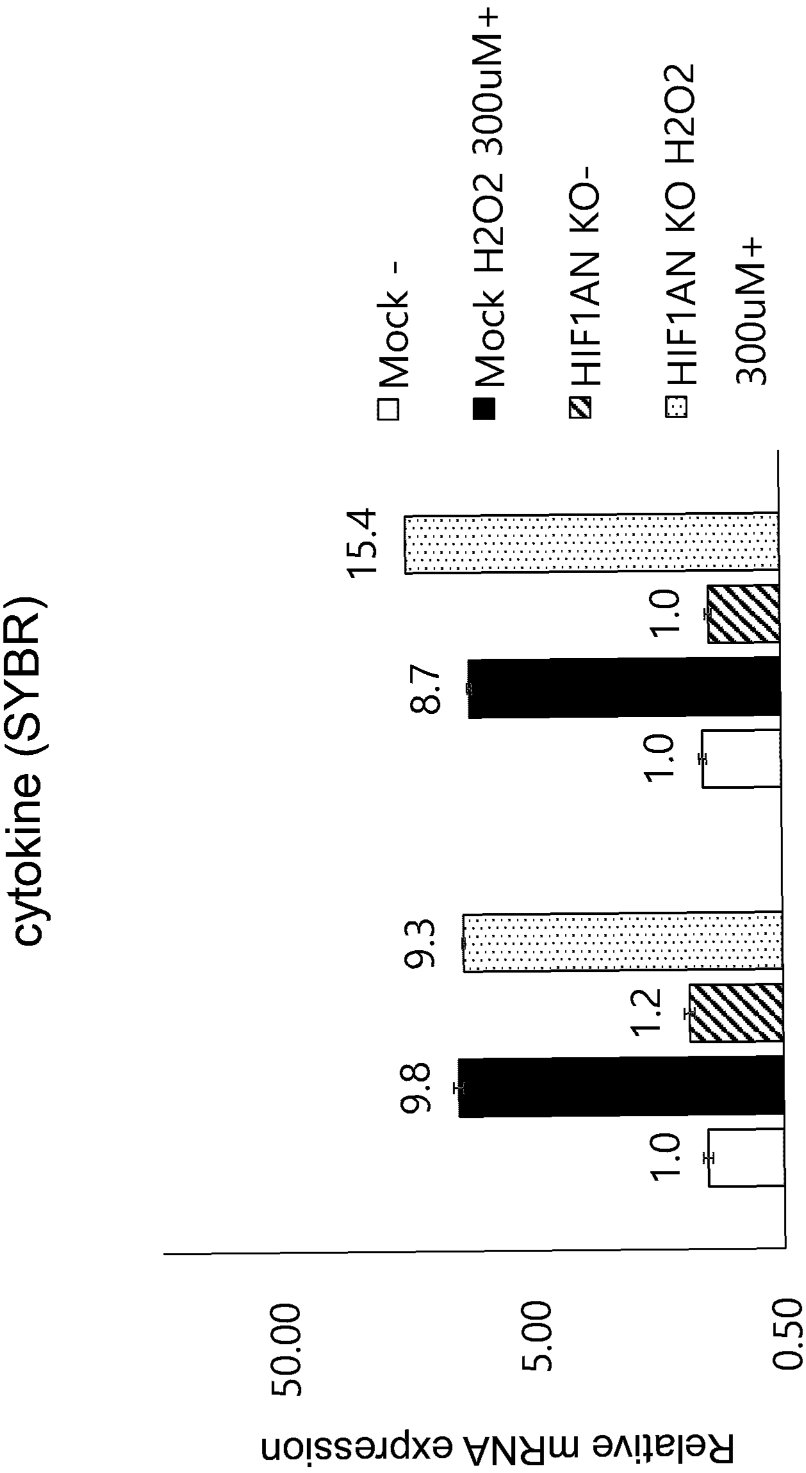
cytokine (SYBR)
Relative mRNA expression
50.00
5.00
0.50
1.0
9.8
1.2
9.3
1.0
8.7
1.0
15.4
VEGF
IL-8
Mock -
Mock H2O2 300uM+
HIF1AN KO-
HIF1AN KO H2O2 300uM+

FIG. 31

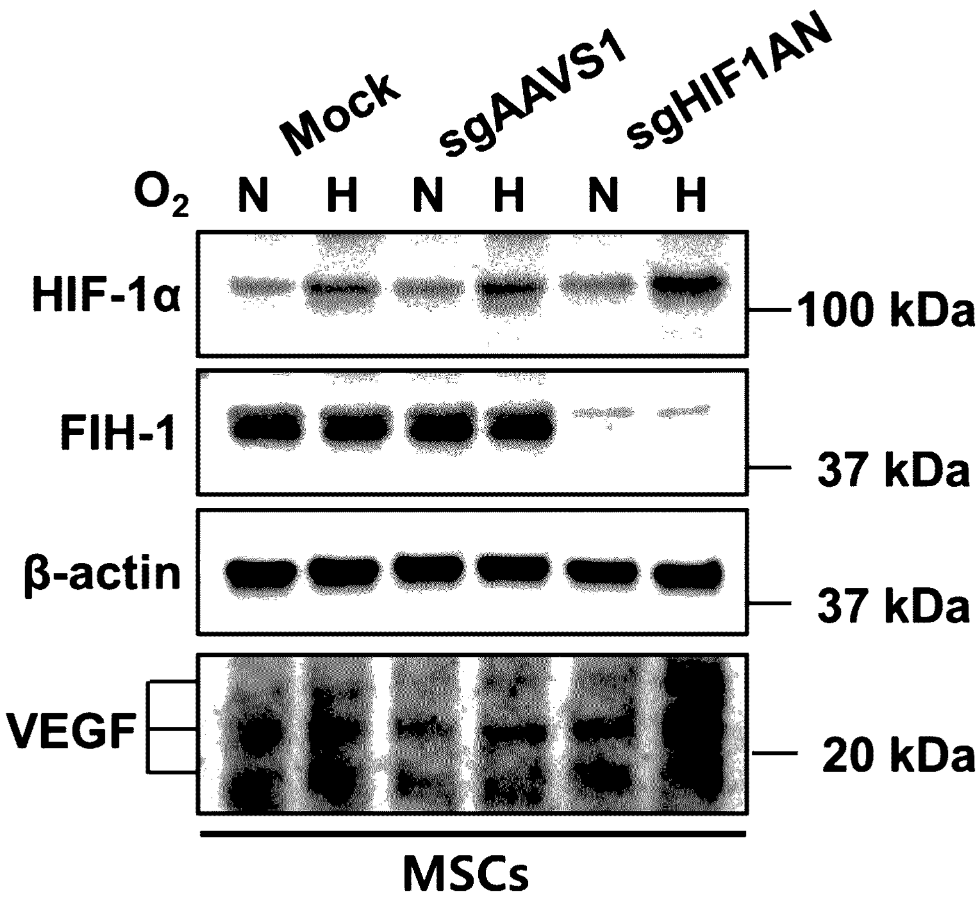
Mock
sgAAVS1
sgHIF1AN
O2
N
H
N
H
N
H
HIF-1α
100 kDa
FIH-1
37 kDa
β-actin
37 kDa
VEGF
20 kDa
MSCs

**INTERNATIONAL SEARCH REPORT**

International application No. **PCT/KR2021/000546**

**A. CLASSIFICATION OF SUBJECT MATTER**

**C12N 5/0775**(2010.01)i; **C12N 9/22**(2006.01)i; **C12N 15/10**(2006.01)i; **C12N 15/11**(2006.01)i; **C07K 14/47**(2006.01)i

According to International Patent Classification (IPC) or to both national classification and IPC

**B. FIELDS SEARCHED**

Minimum documentation searched (classification system followed by classification symbols)

C12N 5/0775(2010.01); C12Q 1/68(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 저산소(Hypoxia), Cas9, 가이드 RNA(guide RNA), 인델(InDel), HIF1AN, HIF3A, PHD2, TLR4, PAI1, 삽입(insertion), 결실(deletion)

**C. DOCUMENTS CONSIDERED TO BE RELEVANT**

| Category* | Citation of document, with indication, where appropriate, of the relevant passages | Relevant to claim No. |
|---|---|---|
| Y | FILHO, D. M. et al. Enhancing the Therapeutic Potential of Mesenchymal Stem Cells with the CRISPR-Cas System. Stem Cell Reviews and Reports. 2019, vol. 15, pp. 463-473. See abstract; pages 463 and 468; figures 1-3; and tables 1-2. | 1-2,4-5,7-10,12-13, 15-16,18-19,21-22 |
| A | | 3,6,11,14,17,20,23-27 |
| Y | MAHON, P. C. et al. FIH-1: a novel protein that interacts with HIF-1 and VHL to mediate repression of HIF-1 transcriptional activity. GENES & DEVELOPMENT. 2001, vol. 15, pp. 2675-2686. See abstract; pages 2675, 2677 and 2683; and figures 1-4 and 8. | 1-2,4-5,7-10,12-13, 15-16,18-19,21-22 |
| Y | YANG, S.-L. et al. Progress on hypoxia-inducible factor-3: Its structure, gene regulation and biological function (Review). MOLECULAR MEDICINE REPORTS. 2015, vol. 12, pp. 2411-2416. See abstract; and page 2414. | 9-10 |
| Y | NATH, B. et al. Hypoxia and Hypoxia Inducible Factors: Diverse Roles in Liver Diseases. HEPATOLOGY. 2012, vol. 55, pp. 622-633. See abstract; and pages 622-623. | 12-13 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 May 2021** | **17 May 2021** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

INTERNATIONAL SEARCH REPORT

International application No. **PCT/KR2021/000546**

**C. DOCUMENTS CONSIDERED TO BE RELEVANT**

| Category* | Citation of document, with indication, where appropriate, of the relevant passages | Relevant to claim No. |
|---|---|---|
| Y | LEE, S. et al. Cell Adhesion and Long-Term Survival of Transplanted Mesenchymal Stem Cells: A Prerequisite for Cell Therapy. Oxidative Medicine and Cellular Longevity. 2015, vol. 2015, thesis no. 632 902, pp. 1-9. See abstract; page 4, right column, page 5, left column; and table 1. | 15-16,18-19 |
| A | KR 10-2015-0101446 A (TOOLGEN INCORPORATED) 03 September 2015 (2015-09-03) | 1-27 |

Form PCT/ISA/210 (second sheet) (July 2019)

**INTERNATIONAL SEARCH REPORT**

International application No.
**PCT/KR2021/000546**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**

International application No.
**PCT/KR2021/000546**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **28-30**
because they relate to subject matter not required to be searched by this Authority, namely:

Claims 28-30 pertain to a method for treatment of the human body, including a step for direct treatment of the human body, and thus pertain to a subject matter on which the International Searching Authority is not required to carry out an international search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2019)

**INTERNATIONAL SEARCH REPORT**
**Information on patent family members**

International application No.
**PCT/KR2021/000546**

| Patent document cited in search report | Publication date (day/month/year) | Patent family member(s) | Publication date (day/month/year) |
|---|---|---|---|
| KR 10-2015-0101446 A | 03 September 2015 | AU 2013-335451 A1 | 07 May 2015 |
| | | AU 2013-335451 B2 | 15 September 2016 |
| | | AU 2015-218519 A1 | 17 September 2015 |
| | | BR 112015008708 A2 | 26 September 2017 |
| | | CA 2888190 A1 | 01 May 2014 |
| | | CN 104968784 A | 07 October 2015 |
| | | CN 104968784 B | 05 November 2019 |
| | | CN 105441440 A | 30 March 2016 |
| | | DK 2912175 T3 | 22 October 2018 |
| | | EP 2912175 A1 | 02 September 2015 |
| | | EP 2912175 B1 | 22 August 2018 |
| | | EP 3346003 A1 | 11 July 2018 |
| | | EP 3372679 A1 | 12 September 2018 |
| | | EP 3733847 A1 | 04 November 2020 |
| | | ES 2690386 T3 | 20 November 2018 |
| | | HK 1212732 A1 | 17 June 2016 |
| | | HK 1223125 A1 | 21 July 2017 |
| | | HK 1257301 A1 | 18 October 2019 |
| | | HK 1257302 A1 | 18 October 2019 |
| | | JP 2016-027807 A | 25 February 2016 |
| | | JP 2016-500003 A | 07 January 2016 |
| | | JP 2018-019698 A | 08 February 2018 |
| | | JP 2020-078303 A | 28 May 2020 |
| | | JP 6517143 B2 | 22 May 2019 |
| | | KR 10-1656236 B1 | 12 September 2016 |
| | | KR 10-1656237 B1 | 12 September 2016 |
| | | KR 10-1706085 B1 | 14 February 2017 |
| | | KR 10-2015-0101476 A | 03 September 2015 |
| | | KR 10-2015-0101477 A | 03 September 2015 |
| | | KR 10-2015-0101478 A | 03 September 2015 |
| | | KR 10-2018-0029092 A | 19 March 2018 |
| | | KR 10-2019-0137932 A | 11 December 2019 |
| | | KR 10-2021-0013288 A | 03 February 2021 |
| | | KR 10-2052286 B1 | 06 December 2019 |
| | | SG 10201809564 A | 29 November 2018 |
| | | US 10851380 B2 | 01 December 2020 |
| | | US 2015-0284727 A1 | 08 October 2015 |
| | | US 2015-0322457 A1 | 12 November 2015 |
| | | US 2015-0344912 A1 | 03 December 2015 |
| | | US 2021-0047647 A1 | 18 February 2021 |
| | | US 2021-0047648 A1 | 18 February 2021 |
| | | WO 2014-065596 A1 | 01 May 2014 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018231018 A


### Non-patent literature cited in the description


- **MAHON PC ; HIROTA K ; SEMENZA GL.** FIH-1: a novel protein that interacts with HIF-lalpha and VHL to mediate repression of HIF-1 transcriptional activity. *Genes Dev.,* 2001, vol. 15 (20), 2675